# EUROPEAN PATENT SPECIFICATION

(11) **EP 2 519 515 B1**
(45) Date of publication and mention of the grant of the patent: **06.11.2013**
(21) Application number: 10796064.3
(22) Date of filing: 22.12.2010
(51) Int. Cl.: C07D 401/12, C07D 401/14, C07D 403/12, A61K 31/497, A61P 25/28

(54) **PYRAZINE DERIVATIVES AND THEIR USE IN THE TREATMENT OF NEUROLOGICAL DISORDERS**
PYRAZINDERIVATE UND IHRE VERWENDUNG BEI DER BEHANDLUNG VON NERVENERKRANKUNGEN
DERIVES DE PYRAZINE ET LEUR UTILISATION DANS LE TRAITEMENT DE TROUBLES NEUROLOGIQUES

(30) Priority: 31.12.2009 US 291724 P
(43) Date of publication of application: 07.11.2012
(73) Proprietor: Novartis AG, 4056 Basel (CH)
(72) Inventor: TINTELNOT-BLOMLEY, Marina, CH-4002 Basel (CH); VEENSTRA, Siem Jacob, CH-4002 Basel (CH)
(74) Representative: Campbell, Lachlan Clive
(86) International application number: PCT/EP2010/070502
(87) International publication number: WO 2011/080176

(56) References cited:
- EP-A1- 1 942 105
- WO-A1-01/25229
- WO-A1-02/060492
- WO-A1-2007/056341
- WO-A1-2011/009943
- WO-A2-2008/079933
- I. NICULESCU-DUVAZ ET AL.: "Novel Inhibitors of B-RAF Based on a Disubstituted Pyrazine Scaffold. Generation of a Nanomolar Lead", J. MED. CHEM., vol. 49, no. 1, 24 November 2005 (2005-11-24), pages 407-416, XP002625448,

## Description

Alzheimer's Disease is a devastating neurodegenerative disorder. Its sporadic forms affect an elderly population (sharp increase in incidence at >75 years of age), in addition, there are various familial forms with an onset of the disease in the fourth or fifth decade of life. Pathologically, it is characterized by the presence of extracellular senile plaques, and intracellular neurofibrillar tangles in patient's brains. The core constituent of the senile plaques are small, 4 kDa amyloid peptides. They are generated by the proteolytic processing of a large transmembrane protein, amyloid precursor protein (APP). Cleavage of APP by beta-secretase (BACE-1) releases the soluble APP-beta fragment, while the 99-amino acid long C-terminus remains tethered to the membrane. This C-terminal fragment is subsequently proteolytically processed by gamma-secretase (an membrane multi-enzyme complex) to generate amyloid peptides of various length, predominantly 40 and 42 amino acids long (Hardy J, Selkoe DJ (2002) Science; 297 (5580):353-356).

If, under pathologic conditions, the generation of these peptides occurs at an increased rate, or if their removal from the brain is disturbed, increased brain amyloid peptide concentrations leads to the formation of oligomers, fibrils and eventually plaques (Farris W, et al (2007) Am.J. Pathol.; 171 (1):241-251). It has been shown, that deposition of amyloid peptides and plaques in the brain is the first measurable event in the pathogenesis of Alzheimers Disease, and that it is the trigger for loss of synapses, synaptic contacts, and neurons (Grimmer T, et al (2009) Neurobiology of Aging; 30 (12):1902-1909). Brain atrophy caused by massive neuron loss is followed by impairments in cognition, memory, orientation and the ability to perform the tasks of daily living, i.e. clinically manifest dementia (Okello A, et al (2009) Neurology; 73 (10):754-760).

BACE-1, also known as Asp2 or Memapsin 2, is a transmembrane aspartic protease highly expressed in neurons. It co-localizes with its substrate APP in Golgi and endocytic compartments (Willem M, Lammich S, Haass C (2009) Semin.Cell Dev.Biol; 20 (2):175-182). Knock-out studies in mice have demonstrated the absence of amyloid peptide formation, while the animals are healthy and fertile (Ohno M, et al (2007) Neurobiol.Dis.; 26 (1):134-145). Genetic ablation of BACE-1 in APP-overexpressing mice has demonstrated absence of plaque formation, and the reverse of cognitive deficits (Ohno M, et al (2004) Neuron; 41 (1):27-33). BACE-1 levels are elevated in the brains of sporadic Alzheimer's Disease patients (Hampel H, Shen Y (2009) Scand. J. Clin. Lab. Invest.; 69 (1):8-12).

Taken together, these findings suggest that the inhibition of BACE-1 may be a favourable therapeutic strategy for Alzheimer's Disease.

The present invention relates to novel pyrazine derivatives having BACE inhibitory activity, to their preparation, to their medical use and to medicaments comprising them.

More particularly, in a first aspect, the invention relates to a compound of the formula 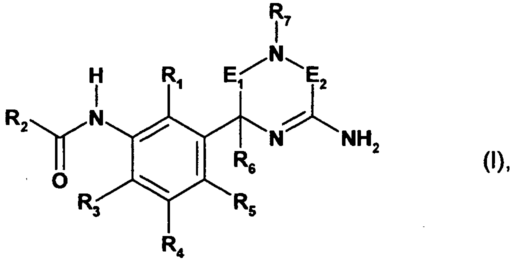 in which
R₁ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl;
R₂ is an aryl, heteroaryl or non-aromatic heterocyclyl group G₁, which group G₁ is optionally substituted by 1 to 4 substituents independently selected from the group, consisting of cyano, amino, aminocarbonyl, halogen, (C₁₋₈)alkyl, halogen-(C₁₋₈)alkyl, hydroxy, oxo, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl, (C₂₋₈)alkenoxy, (C₂₋₈)alkynoxy and a (C₃₋₈)cycloalkyl, aryl, heteroaryl or non-aromatic heterocyclyl group G₂, which group G₂ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl and (C₂₋₈)alkynyl;
R₃ is hydrogen, cyano, halogen, (C₁₋₈)alkyl, halogen-(C₁₋₈)alkyl, (C₁₋₈)alkoxy; halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C_{1- 8})alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl;
either
R₄ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl; and
R₅ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₆)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkyfthio, (C₂₋₈)alkenyl, or (C₂₋₈)alkynyl;
or
R₄ and R₅, taken together, are -C(H)=C(H)-C(H)=C(H)- or a (C₁₋₈)alkylene group, in which (C₁₋₈)alkylene group 1 or 2 -CH₂- ring members are optionally replaced with hetero ring members independently selected from the group, consisting of -N(H)-, -N[(C₁₋₈)alkyl]-, -O-, -S-, -S(=O)- or -S(=O)₂-;
R₆ is hydrogen, (C₁₋₈)alkyl, halogen-(C₁₋₈)alkyl, hydroxy-(C₁₋₈)alkyl, (C₁₋₈)alkoxy-(C₁₋₈)alkyl, mercapto-(C₁₋₈)alkyl, (C₁₋₈)alkylthio-(C₁₋₈)alkyl, amino-(C₁₋₈)alkyl, N-(C₁₋₈)alkylamino-(C₁₋₈)alkyl, N,N-di-[(C₁₋₆)alkyl]amino-(C₁₋₈)alkyl with two identical or different (C₁₋₈)alkyl moieties in the N,N-di-[(C₁₋₈)alkyl]amino moiety, (C₂₋₈)alkenyl, or (C₂₋₈)alkynyl;
R₇ is hydrogen, (C₁₋₈)alkyl, (C₁₋₈)alkyl substituted by halogen, (C₃₋₈)cycloalkyl-(C₁₋₈)alkyl, (C₃₋₈)cycloalkoxy-(C₁₋₈)alkyl, aryloxy-(C₁₋₈)alkyl, (C₁₋₈)alkoxy-(C₁₋₈)alkyl, (C₁₋₈)alkylthio-(C₁₋₈)alkyl, (C₁₋₈)alkylsulfinyl, (C₁₋₈)alkylsulfinyl-(C₁₋₈)alkyl, (C₁₋₈)alkylsulfonyl, (C₁₋₈)alkylsulfonyl-(C₁₋₈)alkyl, amino-(C₁₋₈)alkyl, (C₁₋₈)alkylamino-(C₁₋₈)alkyl, di(C₁₋₈)alkylamino-(C₁₋₈)alkyl with two identical or different (C₁₋₈)alkyl moieties in the di(C₁₋₈)alkylamino moiety, aminosulfonyl, (C₁₋₈)alkylaminosulfonyl, di(C₁₋₈)alkylaminosulfonyl with two identical or different (C₁₋₈)alkyl moieties, formyl, (C₁₋₈)alkylcarbonyl, formyl-(C₁₋₈)alkyl, (C₁₋₈)alkylcarbonyl-(C₁₋₈)alkyl, (C₁₋₈)alkoxycarbonyl, halogen-(C₁₋₈)alkoxycarbonyl, (C₁₋₈)alkoxycarbonyl-(C₁₋₈)alkyl, (C₁₋₈)alkoxy-(C₁₋₈)alkylcarbonyl, or a (C₃₋₈)cycloalkylcarbonyl, arylcarbonyl, aryl-(C₁₋₈)alkylcarbonyl, heteroarylcarbonyl, heteroaryl-(C₁₋₈)alkylcarbonyl, non-aromatic heterocyclyl-carbonyl, (C₃₋₈)cycloalkylsulfonyl, arylsulfonyl, aryl-(C₁₋₈)alkylsulfonyl, heteroarylsulfonyl, heteroaryl-(C₁₋₈)alkylsulfonyl, non-aromatic heterocyclylsulfonyl, (C₃₋₈)cycloalkyl, aryl, aryl-(C₁₋₈)alkyl, heteroaryl, heteroaryl-(C₁₋₈)alkyl or non-aromatic heterocyclyl group G₃, which group G₃ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl and a (C₃₋₈)cycloalkyl, aryl, heteroaryl or non-aromatic heterocyclyl group G₄, which group G₄ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl and (C₂₋₈)alkynyl;
E₁ is -C(R₈)(R₉)-, or -C(R₈)(R₉)-C(R₁₀)(R₁₁)-;
E₂ is -C(R₁₂)(R₁₃)-, or -C(R₁₂)(R₁₃)-C(R₁₄)(R₁₅)-;
either
each of R₈ and R₉ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
or
R₈ and R₉, taken together, are oxo or -CH₂-CH₂-;
either
each of R₁₀ and R₁₁ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
or
R₁₀ and R₁₁, taken together, are oxo or -CH₂-CH₂-;
either
each of R₁₂ and R₁₃ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
or
R₁₂ and R₁₃, taken together, are oxo or -CR₁₆R₁₇-CR₁₈R₁₉-
wherein R₁₆, R₁₇, R₁₈ and R₁₉ are independently selected from hydrogen and fluoro;
and
either
each of R₁₄ and R₁₅ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
or
R₁₄ and R₁₅, taken together, are oxo or -CH₂-CH₂-,
in free form or in salt form.

In a second aspect, the invention relates to a compound of the formula 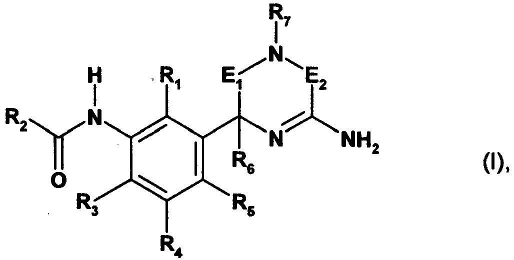 in which
R₁ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl;
R₂ is a (C₃₋₈)cycloalkyl, aryl, heteroaryl or non-aromatic heterocyclyl group G₁, which group G₁ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl and a (C₃₋₈)cycloalkyl, aryl, heteroaryl or non-aromatic heterocyclyl group G₂, which group G₂ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl and (C₂₋₈)alkynyl;
R₃ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl;
either
R₄ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl; and
R₅ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl;
or
R₄ and R₅, taken together, are -C(H)=C(H)-C(H)=C(H)- or a (C₁₋₈)alkylene group, in which (C₁₋₈)alkylene group 1 or 2 -CH₂- ring members are optionally replaced with hetero ring members independently selected from the group, consisting of -N(H)-, -N[(C₁₋₈)alkyl]-, -O-, -S-, -S(=O)- or -S(=O)₂-;
R₆ is hydrogen, (C₁₋₈)alkyl, halogen-(C₁₋₈)alkyl, hydroxy-(C₁₋₈)alkyl, (C₁₋₈)alkoxy-(C₁₋₈)alkyl, mercapto-(C₁₋₈)alkyl, (C₁₋₈)alkylthio-(C₁₋₈)alkyl, amino-(C₁₋₈)alkyl, N-(C₁₋₈)alkylamino-(C₁₋₈)alkyl, N,N-di-[(C₁₋₈)alkyl]amino-(C₁₋₈)alkyl with two identical or different (C₁₋₈)alkyl moieties in the N,N-di-[(C₁₋₈)alkyl]amino moiety, (C₂₋₈)alkenyl, or (C₂₋₈)alkynyl;
R₇ is hydrogen, (C₁₋₈)alkyl, (C₁₋₈)alkyl substituted by halogen, (C₃₋₈)cycloalkyl-(C₁₋₈)alkyl, (C₃₋₈)cycloalkoxy-(C₁₋₈)alkyl, aryloxy-(C₁₋₈)alkyl, (C₁₋₈)alkoxy-(C₁₋₈)alkyl, (C₁₋₈)alkylthio-(C₁₋₈)alkyl, (C₁₋₈)alkylsulfinyl, (C₁₋₈)alkylsulfinyl-(C₁₋₈)alkyl, (C₁₋₈)alkylsulfonyl, (C₁₋₈)alkylsulfonyl-(C₁₋₈)alkyl, amino-(C₁₋₈)alkyl, (C₁₋₈)alkylamino-(C₁₋₈)alkyl, di(C₁₋₈)alkylamino-(C₁₋₈)alkyl with two identical or different (C₁₋₈)alkyl moieties in the di(C₁₋₈)alkylamino moiety, aminosulfonyl, (C₁₋₈)alkylaminosulfonyl, di(C₁₋₈)alkylaminosulfonyl with two identical or different (C₁₋₈)alkyl moieties, formyl, (C₁₋₈)alkylcarbonyl, formyl-(C₁₋₈)alkyl, (C₁₋₈)alkylcarbonyl-(C₁₋₈)alkyl, (C₁₋₈)alkoxycarbonyl, (C₁₋₈)alkoxycarbonyl-(C₁₋₈)alkyl, or a (C₃₋₈)cycloalkylcarbonyl, arylcarbonyl, aryl-(C₁₋₈)alkylcarbonyl, heteroarylcarbonyl, heteroaryl-(C₁₋₈)alkylcarbonyl, non-aromatic heterocyclylcarbonyl, (C₃₋₈)cycloalkylsulfonyl, arylsulfonyl, aryl-(C₁₋₈)alkylsulfonyl, heteroarylsulfonyl, heteroaryl-(C₁₋₈)alkylsulfonyl, non-aromatic heterocyclylsulfonyl, (C₃₋₈)cycloalkyl, aryl, aryl-(C₁₋₈)alkyl, heteroaryl, heteroaryl-(C₁₋₈)alkyl or non-aromatic heterocyclyl group G₃, which group G₃ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl and a (C₃₋₈)cycloalkyl, aryl, heteroaryl or non-aromatic heterocyclyl group G₄, which group G₄ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl and (C₂₋₈)alkynyl;
E₁ is -C(R₈)(R₉)-, or -C(R₈)(R₉)-C(R₁₀)(R₁₁)-;
E₂ is -C(R₁₂)(R₁₃)-; or -C(R₁₂)(R₁₃)-C(R₁₄)(R₁₅)-;
either
each of R₈ and R₉ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
or
R₈ and R₉, taken together, are oxo or -CH₂-CH₂-;
either
each of R₁₀ and R₁₁ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
or
R₁₀ and R₁₁, taken together, are oxo or -CH₂-CH₂-;
either
each of R₁₂ and R₁₃ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
or
R₁₂ and R₁₃, taken together, are oxo or -CH₂-CH₂-; and
either
each of R₁₄ and R₁₅ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
or
R₁₄ and R₁₅, taken together, are oxo or -CH₂-CH₂-,
in free form or in salt form.

Halogen denotes fluorine, chlorine, bromine or iodine.

A halogenated group or moiety, such as halogenalkyl, can be mono-, poly- or per-halogenated.

An aryl group, ring or moiety is a naphthyl or, preferably, phenyl group, ring or moiety.

A heteroaryl group, ring or moiety is an aromatic 5- or 6-membered structure, in which structure 1, 2, 3 or 4 ring members are hetero ring members independently selected from the group, consisting of a nitrogen ring member, an oxygen ring member and a sulfur ring member, such as furyl, pyrrolyl, thienyl, pyrazolyl, imidazolyl, thiazolyl, isothiazolyl, oxazolyl, isoxazolyl, triazolyl, tetrazolyl, pyrazinyl, pyridazinyl, pyrimidyl or pyridyl.

A non-aromatic heterocyclyl group, ring or moiety is a non-aromatic 4-, 5-, 6- or 7-membered cyclic structure, in which cyclic structure 1, 2 or 3 ring members are hetero ring members independently selected from the group, consisting of a nitrogen ring member, an oxygen ring member and a sulfur ring member, such as azetidinyl, oxetanyl, pyrrolinyl, pyrrolidyl, tetrahydrofuryl, tetrahydrothienyl, piperidyl, piperazinyl, tetrahydropyranyl, morpholinyl or perhydroazepinyl.

Any non-cyclic carbon containing group or moiety with more than 1 carbon atom is straight-chain or branched.

Unless defined otherwise, carbon containing groups, moieties or molecules contain 1 to 8, preferably 1 to 6, preferably 1 to 4, preferably 1 or 2, carbon atoms.

The terms "alkoxy", "alkenoxy" and "alkynoxy" respectively denote alkyl, alkenyl and alkynyl groups when linked by oxygen.

On account of one or more than one asymmetrical carbon atom, which may be present in a compound of the formula I, a corresponding compound of the formula I may exist in pure optically active form or in the form of a mixture of optical isomers, e. g. in the form of a racemic mixture. All of such pure optical isomers and all of their mixtures, including the racemic mixtures, are part of the present invention.

In one embodiment, the invention therefore relates to a compound of the formula 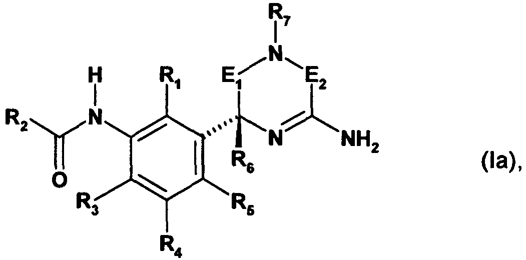 in which
E₁, E₂, R₁, R₂, R₃, R₄, R₅, R₆ and R₇ are as defined hereinbefore in relation to the formula I,
in free form or in salt form.

In one embodiment, the invention therefore relates to a compound of the formula 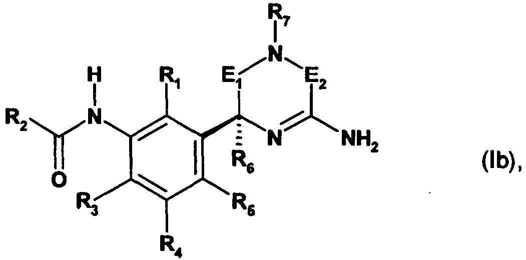 in which
E₁, E₂, R₁, R₂, R₃, R₄, R₅, R₆ and R₇ are as defined hereinbefore in relation to the formula I,
in free form or in salt form.

In one embodiment, there is provided a compound of the Examples as an isolated stereoisomer wherein the stereoisomer is in the R configuration. In another embodiment, there is provided a compound of the Examples as an isolated stereoisomer wherein the stereoisomer is in the S configuration.

As used herein, the term "isomers" refers to different compounds that have the same molecular formula but differ in arrangement and configuration of the atoms. Also as used herein, the term "an optical isomer" or "a stereoisomer" refers to any of the various stereo isomeric configurations which may exist for a given compound of the present invention and includes geometric isomers. It is understood that a substituent may be attached at a chiral center of a carbon atom. Therefore, the invention includes enantiomers, diastereomers or racemates of the compound. "Enantiomers" are a pair of stereoisomers that are non-superimposable mirror images of each other. A 1:1 mixture of a pair of enantiomers is a "racemic mixture. The term is used to designate a racemic mixture where appropriate. "Diastereoisomers" are stereoisomers that have at least two asymmetric atoms, but which are not mirror-images of each other. The absolute stereochemistry is specified according to the Cahn- Ingold- Prelog R-S system. When a compound is a pure enantiomer the stereochemistry at each chiral carbon may be specified by either R or S. Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers or axes and may thus give rise to enantiomers, diastereomers, and other stereoisomeric forms that may be defined, in terms of absolute stereochemistry, as (R)- or (S)-. The present invention is meant to include all such possible isomers, including racemic mixtures, optically pure forms and intermediate mixtures. Optically active (R)- and (S)- isomers may be prepared using chiral synthons or chiral reagents, or resolved using conventional techniques. If the compound contains a double bond, the substituent may be E or Z configuration. If the compound contains a disubstituted cycloalkyl, the cycloalkyl substituent may have a cis- or trans-configuration.

A compound of the formula I may exist in tautomeric form. All such tautomers are part of the present invention.

A compound of the formula I may exist in free form or in salt form, for example a basic compound in acid addition salt form or an acidic compound in the form of a salt with a base. All of such free compounds and salts are part of the present invention.

In one embodiment, the invention relates to a compound of the formula I, Ia, Ib, Ic, Id, Ie or If as defined herein, in free form. In another embodiment, the invention relates to a compound of the formula I, Ia, Ib, Ic, Id, Ie or If as defined herein, in salt form. In another embodiment, the invention relates to a compound of the formula I, la, Ib, Ic, Id, Ie or If as defined herein, in acid addition salt form. In a further embodiment, the invention relates to a compound of the formula I, Ia, Ib, Ic, Id, Ie or If as defined herein, in pharmaceutically acceptable salt form. In yet a further embodiment, the invention relates to a compound of the formula I, la, Ib, Ic, Id, Ie or If as defined herein, in hydrochloride salt form. In yet a further embodiment, the invention relates to any one of the compounds of the Examples in free form. In yet a further embodiment, the invention relates to any one of the compounds of the Examples in salt form. In yet a further embodiment, the invention relates to any one of the compounds of the Examples in acid addition salt form. In yet a further embodiment, the invention relates to any one of the compounds of the Examples in pharmaceutically acceptable salt form. In yet a further embodiment, the invention relates to any one of the compounds of the Examples in hydrochloride salt form.

As used herein, the terms "salt" or "salts" refers to an acid addition or base addition salt of a compound of the invention. "Salts" include in particular "pharmaceutical acceptable salts". The term "pharmaceutically acceptable salts" refers to salts that retain the biological effectiveness and properties of the compounds of this invention and, which typically are not biologically or otherwise undesirable. In many cases, the compounds of the present invention are capable of forming acid and/or base salts by virtue of the presence of amino and/or carboxyl groups or groups similar thereto.

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfornate, chloride/hydrochloride, chlortheophyllonate, citrate, ethandisulfonate, fumarate, gluceptate, gluconate, glucuronate, hippurate, , hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts. Inorganic acids from which salts can be derived include, for example, hydrochloric acid, hydrobromic acid, sulfuric acid, nitric acid and phosphoric acid. Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid and sulfosalicylic acid. Pharmaceutically acceptable base addition salts can be formed with inorganic and organic bases. Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines and basic ion exchange resins. Certain organic amines include isopropylamine, benzathine, cholinate, diethanolamine, diethylamine, lysine, meglumine, piperazine and tromethamine.

The pharmaceutically acceptable salts of the present invention can be synthesized from a parent compound, a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, e.g., in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

When both a basic group and an acid group are present in the same molecule, the compounds of the present invention may also form internal salts, e.g., zwitterionic molecules.

Furthermore, the compounds of the present invention, including their salts, can also be obtained in the form of their hydrates, or include other solvents used for their crystallization. The compounds of the present invention may inherently or by design form solvates with pharmaceutically acceptable solvents (including water); therefore, it is intended that the invention embrace both solvated and unsolvated forms. The term "solvate" refers to a molecular complex of a compound of the present invention (including pharmaceutically acceptable salts thereof) with one or more solvent molecules. Such solvent molecules are those commonly used in the pharmaceutical art, which are known to be innocuous to the recipient, e.g., water, ethanol, and the like. The term "hydrate" refers to the complex where the solvent molecule is water.

The compounds of the present invention, including salts, hydrates and solvates thereof, may inherently or by design form polymorphs. All such polymorphs are part of the present invention.

The present invention includes all pharmaceutically acceptable isotope-labeled compounds of the formula I, wherein one or more than one atom is / are replaced by one or more than one atom having the same atomic number as, but an atomic mass different from, the one(s) usually found in nature. Examples of such isotopes are those of carbon, such as ¹¹C, ¹³C or ¹⁴C, chlorine, such as ³⁶Cl, fluorine, such as ¹⁸F, bromine, such as ⁷⁶Br, hydrogen, such as ²H or ³H, iodine, such as ¹²³I,¹²⁴I, ¹²⁵I or ¹³¹I, nitrogen, such as ¹³N or ¹⁵N, oxygen, such as ¹⁵O, ¹⁷O or ¹⁸O, phosphorus, such as ³²P, or sulphur, such as ³⁵S. An isotope-labeled compound of the formula I can be prepared by a process analogous to those described in the Examples or by a conventional technique known to those skilled in the art using an appropriate isotopically-labeled reagent or starting material. The incorporation of a heavier isotope, such as ²H, may provide greater metabolic stability to a compound of the formula I, which may result in, for example, an increased *in vivo*-half-life of the compound or in reduced dosage requirements. Certain isotope-labeled compounds of the formula I, for example those incorporating a radioactive isotope, such as ³H or ¹⁴C, may be used in drug or substrate-tissue distribution studies. Compounds of the formula I with a positron emitting isotope, such as ¹¹C, ¹⁸F, ¹³N or ¹⁵O, may be useful in positron emission tomography (PET) or single photon emission computed tomography (SPECT) studies, e. g. to examine substrate-receptor occupancies.

Pharmaceutically acceptable solvates in accordance with the invention include those wherein the solvent of crystallization may be isotopically substituted, e.g. D2O, d6-acetone, d6-DMSO.

Compounds of the invention, i.e. compounds of formula I, Ia, Ib, Ic, Id, Ie or If that contain groups capable of acting as donors and/or acceptors for hydrogen bonds may be capable of forming co-crystals with suitable co-crystal formers. These co-crystals may be prepared from compounds of formula I, la, Ib, Ic, Id, Ie or If by known co-crystal forming procedures. Such procedures include grinding, heating, co-subliming, co-melting, or contacting in solution compounds of formula I, Ia, Ib, Ic, Id, Ie or If with the co-crystal former under crystallization conditions and isolating co-crystals thereby formed. Suitable co-crystal formers include those described in WO 2004/078163. Hence the invention further provides co-crystals comprising a compound of formula I, Ia, Ib, Ic, Id, Ie or If.

In certain embodiments, the invention relates to a compound of the formula I, Ia, Ib, Ic, Id, Ie or If in free form or in salt form, in which:
(1) R₁ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl;
(2) R₁ is hydrogen, cyano, halogen, (C₁₋₄)alkyl, halogen-(C₁₋₄)alkyl, (C₁₋₄)alkoxy, or halogen-(C₁₋₄)alkoxy;
(3) R₁ is hydrogen;
(4) R₂ is a (C₃₋₈)cycloalkyl, aryl, heteroaryl or non-aromatic heterocyclyl group G₁, which group G₁ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl and a (C₃₋₈)cycloalkyl, aryl, heteroaryl or non-aromatic heterocyclyl group G₂, which group G₂ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl and (C₂₋₈)alkynyl;
(5) R₂ is a (C₃₋₈)cycloalkyl, aryl, heteroaryl or non-aromatic heterocyclyl group G₁, which group G₁ is optionally substituted by 1 to 4 substituents independently selected from the group, consisting of cyano, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl and a (C₃₋₈)cycloalkyl, aryl, heteroaryl or non-aromatic heterocyclyl group G₂, which group G₂ is optionally substituted by 1 to 4 substituents independently selected from the group, consisting of cyano, halogen, (C_{1- 8})alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl and (C₂₋₈)alkynyl;
(6) R₂ is a (C₃₋₈)cycloalkyl, aryl or heteroaryl group G₁, which group G₁ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl and a (C₃₋₈)cycloalkyl, aryl or heteroaryl group G₂, which group G₂ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl and (C₂₋₈)alkynyl;
(7) R₂ is a heteroaryl group G₁, which group G₁ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl and a (C₃₋₈)cycloalkyl, aryl or heteroaryl group G₂, which group G₂ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl and (C₂₋₈)alkynyl;
(8) R₂ is a heteroaryl group G₁, which group G₁ is optionally substituted by 1 or 2 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl and a (C₃₋₈)cycloalkyl, aryl or heteroaryl group G₂, which group G₂ is unsubstituted;
**(9)** R₂ is an aryl or heteroaryl group G₁, which group G₁ is optionally substituted by 1, 2, 3 or 4 substituents independently selected from the group, consisting of cyano, amino, aminocarbonyl, halogen, (C₁₋₈)alkyl, halogen-(C₁₋₈)alkyl, hydroxy, oxo, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl, (C₂₋₈)alkenoxy, and (C₂₋₈)alkynoxy;
**(10)** R₂ is phenyl or a 5- or 6-membered heteroaryl group G₁ in which structure 1, 2, 3, or 4 ring members are hetero ring members independently selected from the group consisiting of a nitrogen ring member, an oxygen ring member and a sulfur ring member, which group G₁ is optionally substituted by 1, 2, 3 or 4 substituents independently selected from the group, consisting of cyano, amino, aminocarbonyl, halogen, (C₁₋₄)alkyl, halogen-(C₁₋₄)alkyl, hydroxy, oxo, (C₁₋₄)alkoxy, halogen-(C₁₋₄)alkoxy, (C₁₋₄)alkylthio, halogen-(C₁₋₄)alkylthio, (C₁₋₄)alkoxy-(C₁₋₄)alkyl, (C₁₋₄)alkoxy-(C₁₋₄)alkoxy, (C₁₋₄)alkoxy-(C₁₋₄)alkylthio, (C₁₋₄)alkylthio-(C₁₋₄)alkyl, (C₁₋₄)alkylthio-(C₁₋₄)alkoxy, (C₁₋₄)alkylthio-(C₁₋₄)alkylthio, (C₂₋₄)alkenyl, (C₂₋₄)alkynyl, (C₂₋₄)alkenoxy, and (C₂₋₄)alkynoxy;
**(11)** R₂ is a 6-membered heteroaryl group G₁ in which structure 1, 2, 3, or 4 ring members are hetero ring members independently selected from the group consisiting of a nitrogen ring member, an oxygen ring member and a sulfur ring member, which group G₁ is optionally substituted by 1, 2, 3 or 4 substituents independently selected from the group, consisting of cyano, amino, aminocarbonyl, halogen, (C₁₋₄)alkyl, halogen-(C₁₋₄)alkyl, hydroxy, oxo, (C₁₋₄)alkoxy, halogen-(C₁₋₄)alkoxy, (C₁₋₄)alkylthio, halogen-(C₁₋₄)alkylthio, (C₁₋₄)alkoxy-(C₁₋₄)alkyl, (C₁₋₄)alkoxy-(C₁₋₄)alkoxy, (C₁₋₄)alkoxy-(C₁₋₄)alkylthio, (C₁₋₄)alkylthio-(C₁₋₄)alkyl, (C₁₋₄)alkylthio-(C₁₋₄)alkoxy, (C₁₋₄)alkyfthio-(C₁₋₄)alkylthio, (C₂₋₄)alkenyl, (C₂₋₄)alkynyl, (C₂₋₄)alkenoxy, and (C₂₋₄)alkynoxy;
**(12)** R₂ is a 6-membered heteroaryl group G₁ in which structure 1, 2, 3, or 4 ring members are hetero ring members independently selected from the group consisiting of a nitrogen ring member, an oxygen ring member and a sulfur ring member, which group G₁ is optionally substituted by 1, 2, 3 or 4 substituents independently selected from the group, consisting of cyano, halogen, (C₁₋₄)alkyl, halogen-(C₁₋₄)alkyl, hydroxy, oxo, (C₁₋₄)alkoxy and halogen-(C₁₋₄)alkoxy;
**(13)** R₂ is a pyridyl or pyrazinyl group which is optionally substituted by 1, 2 or 3 substituents independently selected from the group, consisting of cyano, amino, aminocarbonyl, halogen, (C₁₋₄)alkyl, halogen-(C₁₋₄)alkyl, hydroxy, oxo, (C₁₋₄)alkoxy, halogen-(C₁₋₄)alkoxy, (C₁₋₄)alkylthio, halogen-(C₁₋₄)alkylthio, (C₁₋₄)alkoxy-(C₁₋₄)alkyl, (C₁₋₄)alkoxy-(C₁₋₄)alkoxy, (C_{1- 4})alkoxy-(C₁₋₄)alkylthio, (C₁₋₄)alkylthio-(C₁₋₄)alkyl, (C₁₋₄)alkylthio-(C₁₋₄)alkoxy, (C₁₋₄)alkylthio-(C₁₋₄)alkylthio, (C₁₋₄)alkenyl, (C₁₋₄)alkynyl, (C₂₋₄)alkenoxy, and (C₁₋₄)alkynoxy;
**(14)** R₂ is a pyridyl or pyrazinyl group which is optionally substituted by 1, 2 or 3 substituents independently selected from the group, consisting of cyano, halogen, (C₁₋₄)alkyl, halogen-(C₁₋₄)alkyl, hydroxy, oxo, (C₁₋₄)alkoxy and halogen-(C₁₋₄)alkoxy;
**(15)** R₂ is a pyridin-2-yl or pyrazin-2-yl group which is optionally substituted by 1, 2 or 3 substituents independently selected from the group, consisting of cyano, amino, aminocarbonyl, halogen, (C₁₋₄)alkyl, halogen-(C₁₋₄)alkyl, hydroxy, oxo, (C₁₋₄)alkoxy, halogen-(C₁₋₄)alkoxy, (C₁₋₄)alkylthio, halogen-(C₁₋₄)alkylthio, (C₁₋₄)alkoxy-(C₁₋₄)alkyl, (C₁₋₄)alkoxy-(C₁₋₄)alkoxy, (C₁₋₄)alkoxy-(C₁₋₄)alkylthio, (C₁₋₄)alkylthio-(C₁₋₄)alkyl, (C₁₋₄)alkylthio-(C₁₋₄)alkoxy, (C₁₋₄)alkylthio-(C₁₋₄)alkylthio, (C₂₋₄)alkenyl, (C₁₋₄)alkynyl, (C₂₋₄)alkenoxy, and (C₁₋₄)alkynoxy;
**(16)** R₂ is a pyridin-2-yl or pyrazin-2-yl group which is optionally substituted by 1, 2 or 3 substituents independently selected from the group, consisting of cyano, halogen, (C₁₋₄)alkyl, halogen-(C₁₋₄)alkyl, hydroxy, oxo, (C₁₋₄)alkoxy and halogen-(C₁₋₄)alkoxy;
**(17)** R₂ is a pyridin-2-yl or pyrazin-2-yl group which is optionally substituted by 1 or 2 substituents independently selected from the group, consisting of cyano, amino, fluoro, bromo, chloro, hydroxyl, oxo, methyl and difluoromethoxy;
**(18)** R₂ is a pyridyl or pyrazinyl group which is substituted by 1, 2 or 3 substituents and wherein one of the substituents is located at the *para* position of the pyridyl or pyrazinyl group relative to the amide linker and wherein the substituents are independently selected from the group, consisting of cyano, amino, aminocarbonyl, halogen, (C₁₋₄)alkyl, halogen-(C₁₋₄)alkyl, hydroxy, oxo, (C₁₋₄)alkoxy, halogen-(C₁₋₄)alkoxy, (C₁₋₄)alkylthio, halogen-(C₁₋₄)alkylthio, (C₁₋₄)alkoxy-(C₁₋₄)alkyl, (C₁₋₄)alkoxy-(C₁₋₄)alkoxy, (C₁₋₄)alkoxy-(C₁₋₄)alkylthio, (C₁₋₄)alkylthio-(C₁₋₄)alkyl, (C₁₋₄)alkylthio-(C₁₋₄)alkoxy, (C₁₋₄)alkylthio-(C₁₋₄)alkylthio, (C₂₋₄)alkenyl, (C₁₋₄)alkynyl, (C₂₋₄)alkenoxy, and (C₂₋₄)alkynoxy;
**(19)** R₂ is a pyridyl or pyrazinyl group which is substituted by 1, 2 or 3 substituents and wherein one of the substituents is located at the *para* position of the pyridyl or pyrazinyl group relative to the amide linker and wherein the substituents are independently selected from the group, consisting of cyano, halogen, (C₁₋₄)alkyl, halogen-(C₁₋₄)alkyl, hydroxy, oxo, (C₁₋₄)alkoxy and halogen-(C₁₋₄)alkoxy;
**(20)** R₂ is a pyridin-2-yl or pyrazin-2-yl group which is substituted by 1, 2 or 3 substituents and wherein one of the substituents is located at the *para* position of the pyridin-2-yl or pyrazin-2-yl group relative to the amide linker and wherein the substituents are independently selected from the group, consisting of cyano, halogen, (C₁₋₄)alkyl, halogen-(C₁₋₄)alkyl, hydroxy, oxo, (C₁₋₄)alkoxy and halogen-(C₁₋₄)alkoxy;
(**21**) R₂ is a pyridyl or pyrazinyl group which is substituted by 2 or 3 substituents and wherein one of the substituents is located at the para position and one of the substituents is located at the *ortho* position of the pyridyl or pyrazinyl group relative to the amide linker and wherein the substituents are independently selected from the group, consisting of cyano, amino, aminocarbonyl, halogen, (C₁₋₄)alkyl, halogen-(C₁₋₄)alkyl, hydroxy, oxo, (C₁₋₄)alkoxy, halogen-(C₁₋₄)alkoxy, (C₁₋₄)alkylthio, halogen-(C₁₋₄)alkylthio, (C₁₋₄)alkoxy-(C₁₋₄)alkyl, (C₁₋₄)alkoxy-(C₁₋₄)alkoxy, (C₁₋₄)alkoxy-(C₁₋₄)alkylthio, (C₁₋₄)alkylthio-(C₁₋₄)alkyl, (C₁₋₄)alkylthio-(C₁₋₄)alkoxy, (C₁₋₄)alkylthio-(C₁₋₄)alkylthio, (C₂₋₄)alkenyl, (C₂₋₄)alkynyl, (C₂₋₄)alkenoxy, and (C₂₋₄)alkynoxy;
**(22)** R₂ is a pyridyl or pyrazinyl group which is substituted by 2 or 3 substituents and wherein one of the substituents is located at the para position and one of the substituents is located at the *ortho* position of the pyridyl or pyrazinyl group relative to the amide linker and wherein the substituents are independently selected from the group, consisting of cyano, halogen, (C₁₋₄)alkyl, halogen-(C₁₋₄)alkyl, hydroxy, oxo, (C₁₋₄)alkoxy and halogen-(C₁₋₄)alkoxy;
**(23)** R₂ is a pyridin-2-yl or pyrazin-2-yl group which is substituted by 2 substituents and wherein one of the substituents is located at the para position and one of the substituents is located at the *ortho* position of the pyridin-2-yl or pyrazin-2-yl group relative to the amide linker and wherein the substituents are independently selected from the group, consisting of cyano, halogen, (C₁₋₄)alkyl, halogen-(C₁₋₄)alkyl, hydroxy, oxo, (C₁₋₄)alkoxy and halogen-(C₁₋₄)alkoxy;
**(24)** R₂ is a pyridin-2-yl or pyrazin-2-yl group which is substituted by 2 substituents and wherein one of the substituents is located at the para position and one of the substituents is located at the *ortho* position of the pyridin-2-yl or pyrazin-2-yl group relative to the amide linker and wherein the substituents are independently selected from the group, consisting of cyano, amino, fluoro, bromo, chloro, hydroxyl, oxo, methyl and difluoromethoxy;
**(25)** R₃ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁-₈)alkylthio-(C₁₋₈)alkylthio, (C₂₋₈)alkenyl, or (C₂₋₈)alkynyl;
**(26)** R₃ is hydrogen, cyano, halogen, (C₁₋₄)alkyl, halogen-(C₁₋₄)alkyl, (C₁₋₄)alkoxy, or halogen-(C₁₋₄)alkoxy;
**(27)** R₃ is hydrogen;
**(28)** either
   R₄ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl; and
   R₅ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-( C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl;
   or
   R₄ and R₅, taken together, are -C(H)=C(H)-C(H)=C(H)- or a (C₁₋₈)alkylene group, in which (C₁₋₈)alkylene group 1 or 2 -CH₂- ring members are optionally replaced with hetero ring members independently selected from the group, consisting of -N(H)-, -N[(C₁₋₈)alkyl]-, -O-, -S-, -S(=O)- or -S(=O)₂-;
**(29)** R₄ is hydrogen, cyano, halogen, (C₁₋₄)alkyl, halogen-(C₁₋₄)alkyl, (C₁₋₄)alkoxy, or halogen-(C₁₋₄)alkoxy;
**(30)** R₅ is hydrogen, cyano, halogen, (C₁₋₄)alkyl, halogen-(C₁₋₄)alkyl, (C₁₋₄)alkoxy, or halogen-(C₁₋₄)alkoxy;
**(31)** R₄ is hydrogen, or halogen; and
   R₅ is hydrogen, or halogen;
**(32)** R₄ is hydrogen; and
   R₅ is halogen;
**(33)** R₄ is hydrogen; and
   R₅ is fluoro;
**(34)** R₄ is hydrogen; and
   R₅ is hydrogen or fluoro;
**(35)** R₄ is halogen; and
   R₅ is hydrogen;
**(36)** each of R₄ and R₅ is hydrogen;
**(37)** R₆ is hydrogen, (C₁₋₈)alkyl, halogen-(C₁₋₈)alkyl, hydroxy-(C₁₋₈)alkyl, (C₁₋₈)alkoxy-(C₁₋₈)alkyl, mercapto-(C₁₋₈)alkyl, (C₁₋₈)alkylthio-(C₁₋₈)alkyl, amino-(C₁₋₈)alkyl, N-(C₁₋₈)alkylamino-(C₁₋₈)alkyl, N,N-di-[(C₁₋₈)alkyl]amino-(C₁₋₈)alkyl with two identical or different (C₁₋₈)alkyl moieties in the N,N-di-[(C₁₋₈)alkyl]amino moiety, (C₂₋₈)alkenyl, or (C₂₋₈)alkynyl;
**(38)** R₆ is (C₁₋₈)alkyl, or halogen-(C₁₋₈)alkyl;
**(39)** R₆ is (C₁₋₃)alkyl, or halogen-(C₁₋₃)alkyl;
**(40)** R₆ is (C₁₋₈)alkyl, or fluorine-substituted (C₁₋₈)alkyl;
**(41)** R₆ is (C₁₋₃)alkyl, or fluorine-substituted (C₁₋₃)alkyl;
**(42)** R₆ is methyl, fluoromethyl, or difluoromethyl;
**(43)** R₇ is hydrogen, (C₁₋₈)alkyl, (C₁₋₈)alkyl substituted by halogen, (C₃₋₈)cycloalkyl-(C₁₋₈)alkyl, (C₃₋₈)cycloalkoxy-(C₁₋₈)alkyl, aryloxy-(C₁₋₈)alkyl, (C₁₋₈)alkoxy-(C₁₋₈)alkyl, (C₁₋₈)alkylthio-(C₁₋₈)alkyl, (C₁₋₈)alkylsulfinyl, (C₁₋₈)alkylsulfinyl-(C₁₋₈)alkyl, (C₁₋₈)alkylsulfonyl, (C₁₋₈)alkylsulfonyl-(C₁₋₈)alkyl, amino-(C₁₋₈)alkyl, (C₁₋₈)alkylamino-(C₁₋₈)alkyl, di(C₁₋₈)alkylamino-(C₁₋₈)alkyl with two identical or different (C₁₋₈)alkyl moieties in the di(C₁₋₈)alkylamino moiety, aminosulfonyl, (C₁₋₈)alkylaminosulfonyl, di(C₁₋₈)alkylaminosulfonyl with two identical or different (C₁₋₈)alkyl moieties, formyl, (C₁₋₈)alkylcarbonyl, formyl-(C₁₋₈)alkyl, (C₁₋₈)alkylcarbonyl-(C₁₋₈)alkyl, (C₁₋₈)alkoxycarbonyl, (C₁₋₈)alkoxycarbonyl-(C₁₋₈)alkyl, or a (C₃₋₈)cycloalkylcarbonyl, arylcarbonyl, aryl-(C₁₋₈)alkylcarbonyl, heteroarylcarbonyl, heteroaryl-(C₁₋₈)alkylcarbonyl, non-aromatic heterocyclylcarbonyl, (C₃₋₈)cycloalkylsulfonyl, arylsulfonyl, aryl-(C₁₋₈)alkylsulfonyl, heteroarylsulfonyl, heteroaryl-(C₁₋₈)alkylsulfonyl, non-aromatic heterocyclylsulfonyl, (C₃₋₈)cycloalkyl, aryl, aryl-(C₁₋₈)alkyl, heteroaryl, heteroaryl-(C₁₋₈)alkyl or non-aromatic heterocyclyl group G₃, which group G₃ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl and a (C₃₋₈)cycloalkyl, aryl, heteroaryl or non-aromatic heterocyclyl group G₄, which group G₄ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl and (C₂₋₈)alkynyl;
(44) R₇ is (C₁₋₈)alkyl, (C₁₋₈)alkoxy-(C₁₋₈)alkyl, or a heteroaryl group G₃, which group G₃ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl and a (C₃₋₈)cycloalkyl, aryl, heteroaryl or non-aromatic heterocyclyl group G₄, which group G₄ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl and (C₂₋₈)alkynyl;
**(45)** R₇ is hydrogen, (C₁₋₆)alkyl, halogen-(C₁₋₈)alkyl, (C₁₋₄)alkoxy-(C₁₋₄)alkyl, (C₁₋₆)alkylcarbonyl, (C₁₋₆)alkoxycarbonyl, halogen-(C₁₋₆)alkoxycarbonyl, (C₁₋₆)alkoxy-(C₁₋₆)alkylcarbonyl, (C₃₋₆)cycloalkyl, (C₃₋₈)cycloalkyl-carbonyl, or a heteroaryl group optionally substituted by 1, 2, or 3 substituents independently selected from the group consisting of cyano, halogen, hydroxyl, (C₁₋₄)alkyl, halogen-(C₁₋₄)alkyl, (C₁₋₄)alkoxy, halogen-(C₁₋₄)alkoxy, (C₁₋₃)alkoxy-(C₁₋₃)alkyl and (C₁₋₃)alkoxy-(C₁₋₃)alkoxy;
**(46)** R₇ is hydrogen, methyl, ethyl, isopropyl, acetyl, methoxyethyl, methoxycarbonyl, dichloroethoxycarbonyl, methoxymethylcarbonyl, cyclopropylcarbonyl, pyridinyl, or methyl substituted pyrazolyl;
**(47)** E₁ is -C(R₈)(R₉)-, or -C(R₈)(R₉)-C(R₁₀)(R₁₁)-;
**(48)** E₁ is -C(R₈)(R₉)-;
**(49)** E₂ is -C(R₁₂)(R₁₃)-, or -C(R₁₂)(R₁₃)-C(R₁₄)(R₁₅)-;
**(50)** E₂ is -C(R₁₂)(R₁₃)-;
**(51)** either
   each of R₈ and R₉ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
   or
   R₈ and R₉, taken together, are oxo or -CH₂-CH₂-;
**(52)** either
   each of R₈ and R₉ is independently selected from the group, consisting of hydrogen, cyano, halogen, (C₁₋₃)alkyl and halogen-(C₁₋₃)alkyl;
   or
   R₈ and R₉, taken together, are oxo or -CH₂-CH₂-;
**(53)** either
   each of R₈ and R₉ is hydrogen;
   or
   R₈ and R₉, taken together, are oxo;
**(54)** each of R₈ and R₉ is hydrogen;
**(55)** either each of R₁₀ and R₁₁ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
   or
   R₁₀ and R₁₁, taken together, are oxo or -CH₂-CH₂-;
**(56)** each of R₁₀ and R₁₁ is hydrogen;
**(57)** either
   each of R₁₂ and R₁₃ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
   or
   R₁₂ and R₁₃, taken together, are oxo or -CH₂-CH₂-;
**(58)** each of R₁₂ and R₁₃ is independently selected from the group, consisting of hydrogen, halogen, (C₁₋₈)alkyl and halogen-(C₁₋₈)alkyl;
**(59)** each of R₁₂ and R₁₃ is independently selected from the group, consisting of hydrogen, (C₁₋₈)alkyl and halogen-(C₁₋₈)alkyl;
**(60)** either
   each of R₁₂ and R₁₃ is independently selected from the group, consisting of hydrogen, cyano, halogen, (C₁₋₃)alkyl and halogen-(C₁₋₃)alkyl;
   or
   R₁₂ and R₁₃, taken together, are oxo or -CR₁₆R₁₇-CR₁₈R₁₉-
   wherein R₁₆, R₁₇, R₁₈ and R₁₉ are independently selected from hydrogen and fluoro;
**(61**) either
   each of R₁₂ and R₁₃ is independently selected from the group, consisting of hydrogen, (C₁₋₃)alkyl and halogen-(C₁₋₃)alkyl;
   or
   R₁₂ and R₁₃, taken together, are oxo;
**(62)** either
   each of R₁₂ and R₁₃ is independently selected from the group, consisting of hydrogen, methyl and ethyl;
   or
   R₁₂ and R₁₃, taken together, are oxo;
**(63)** R₁₂ is (C₁₋₈)alkyl, and R₁₃ is halogen-(C₁₋₈)alkyl;
**(64)** R₂ is (C₁₋₃)alkyl, and R₁₃ is halogen-(C₁₋₃)alkyl;
**(65)** each of R₁₂ and R₁₃ is hydrogen;
**(66)** R₁₂ and R₁₃, taken together, are oxo;
**(67)** either
   each of R₁₄ and R₁₅ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
   or
   R₁₄ and R₁₅, taken together, are oxo or -CH₂-CH₂-;
**(68)** each of R₁₄ and R₁₅ is hydrogen.

The skilled person would understand that the embodiments (1) to (68) may be used independently, collectively or in any combination or sub-combination to the limit the scope of the invention as described hereinbefore in relation to compounds of the formula I, Ia, Ib, Ic, Id, Ie or If.

In one embodiment, the invention relates to a compound of the formula 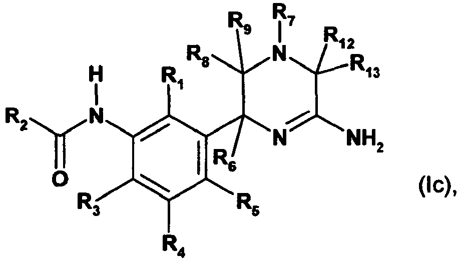 in which
R₁ is hydrogen, cyano, halogen, (C₁₋₄)alkyl, halogen-(C₁₋₄)alkyl, (C₁₋₄)alkoxy, or halogen-(C₁₋₄)alkoxy;
R₂ is phenyl or a 5- or 6-membered heteroaryl group G₁ in which structure 1, 2, 3, or 4 ring members are hetero ring members independently selected from the group consisiting of a nitrogen, ring member, an oxygen ring member and a sulfur ring member, which group G₁ is optionally substituted by 1, 2, 3 or 4 substituents independently selected from the group, consisting of cyano, amino, aminocarbonyl, halogen, (C₁₋₄)alkyl, halogen-(C₁₋₄)alkyl, hydroxy, oxo, (C₁₋₄)alkoxy, halogen-(C₁₋₄)alkoxy, (C₁₋₄)alkylthio, halogen-(C₁₋₄)alkylthio, (C₁₋₄)alkoxy-(C₁₋₄)alkyl, (C₁₋₄)alkoxy-(C₁₋₄)alkoxy, (C₁₋₄)alkoxy-(C₁₋₄)alkylthio, (C₁₋₄)alkylthio-(C₁₋₄)alkyl, (C₁₋₄)alkylthio-(C₁₋₄)alkoxy, (C₁₋₄)alkylthio-(C₁₋₄)alkylthio, (C₂₋₄)alkenyl, (C₂₋₄)alkynyl, (C₂₋₄)alkenoxy, and (C₂₋₄)alkynoxy;
R₃, R₄ and R₅ are independently selected from the group consisting of hydrogen, cyano, halogen, (C₁₋₄)alkyl, halogen-(C₁₋₄)alkyl, (C₁₋₄)alkoxy, or halogen-(C₁₋₄)alkoxy;
R₆ is (C₁₋₃)alkyl, or fluorine-substituted (C₁₋₃)alkyl;
R₇ is hydrogen, (C₁₋₆)alkyl, halogen-(C₁₋₆)alkyl, (C₁₋₄)alkoxy-(C₁₋₄)alkyl, (C₁₋₆)alkylcarbonyl, (C₁₋₆)alkoxycarbonyl, halogen-(C₁₋₆)alkoxycarbonyl, (C₁₋₆)alkoxy-(C₁-₆)alkylcarbonyl, (C₃₋₆)cycloalkyl, (C₃₋₆)cycloalkyl-carbonyl, or a heteroaryl group optionally substituted by 1, 2, or 3 substituents independently selected from the group consisting of cyano, halogen, hydroxyl, (C₁₋₄)alkyl, halogen-(C₁₋₄)alkyl, (C₁₋₄)alkoxy, halogen-(C₁₋₄)alkoxy, (C₁₋₃)alkoxy-(C₁₋₃)alkyl and (C₁₋₃)alkoxy-(C₁₋₃)alkoxy;
either
each of R₈ and R₉ is independently selected from the group, consisting of hydrogen, cyano, halogen, (C₁₋₃)alkyl and halogen-(C₁₋₃)alkyl;
or
R₈ and R₉, taken together, are oxo or -CH₂-CH₂-; and
either
each of R₁₂ and R₁₃ is independently selected from the group, consisting of hydrogen, cyano, halogen, (C₁₋₃)alkyl and halogen-(C₁₋₃)alkyl;
or
R₁₂ and R₁₃, taken together, are oxo or -CR₁₆R₁₇-CR₁₈R₁₉-
wherein R₁₆, R₁₇, R₁₈ and R₁₉ are independently selected from hydrogen and fluoro; in free form or in pharmaceutically acceptable salt form.

In another embodiment, the invention relates to a compound of the formula 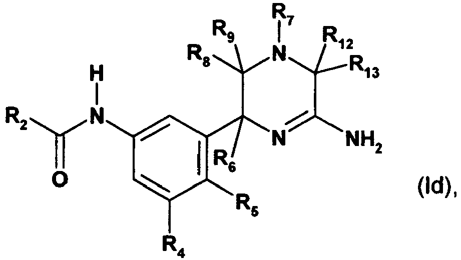 in which
R₂ is a 6-membered heteroaryl group G₁ in which structure 1, 2, 3, or 4 ring members are hetero ring members independently selected from the group consisiting of a nitrogen ring member, an oxygen ring member and a sulfur ring member, which group G₁ is optionally substituted by 1, 2, 3 or 4 substituents independently selected from the group, consisting of cyano, amino, aminocarbonyl, halogen, (C₁₋₄)alkyl, halogen-(C₁₋₄)alkyl, hydroxy, oxo, (C₁₋₄)alkoxy, halogen-(C₁₋₄)alkoxy, (C₁₋₄)alkylthio, halogen-(C₁₋₄)alkylthio, (C₁₋₄)alkoxy-(C₁₋₄)alkyl, (C₁₋₄)alkoxy-(C₁₋₄)alkoxy, (C₁₋₄)alkoxy-(C₁₋₄)alkylthio, (C₁₋₄)alkylthio-(C₁₋₄)alkyl, (C₁₋₄)alkylthio-(C₁₋₄)alkoxy, (C₁₋₄)alkylthio-(C₁₋₄)alkylthio, (C₂₋₄)alkenyl, (C₂₋₄)alkynyl, (C₂₋₄)alkenoxy, and (C₂₋₄)alkynoxy;
R₄ and R₅ are independently hydrogen, or halogen;
R₆ is (C₁₋₃)alkyl, or fluorine-substituted (C₁₋₃)alkyl;
R₇ is hydrogen, (C₁₋₆)alkyl, halogen-(C₁₋₆)alkyl, (C₁₋₄)alkoxy-(C₁₋₄)alkyl, (C₁₋₆)alkylcarbonyl, (C₁₋₆)alkoxycarbonyl, halogen-(C₁₋₆)alkoxycarbonyl, (C₁₋₆)alkoxy-(C₁₋₆)alkylcarbonyl, (C₃₋₆)cycloalkyl, (C₃₋₆)cycloalkyl-carbonyl, or a heteroaryl group optionally substituted by 1, 2, or 3 substituents independently selected from the group consisting of cyano, halogen, hydroxyl, (C₁₋₄)alkyl, halogen-(C₁₋₄)alkyl, (C₁₋₄)alkoxy, halogen-(C₁₋₄)alkoxy, (C₁₋₃)alkoxy-(C₁₋₃)alkyl and (C₁₋₃)alkoxy-(C₁₋₃)alkoxy;
either
each of R₈ and R₉ is hydrogen;
or
R₈ and R₉, taken together, are oxo; and
either
each of R₁₂ and R₁₃ is independently selected from the group, consisting of hydrogen, (C₁₋₃)alkyl and halogen-(C₁₋₃)alkyl;
or
R₁₂ and R₁₃, taken together, are oxo;
in free form or in pharmaceutically acceptable salt form.

In a further embodiment, the invention relates to a compound of the formula 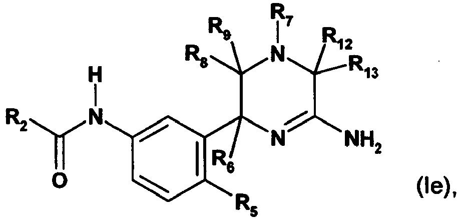 in which
R₂ is a pyridyl or pyrazinyl group which is substituted by 1, 2 or 3 substituents and wherein one of the substituents is located at the *para* position of the pyridyl or pyrazinyl group relative to the amide linker and wherein the substituents are independently selected from the group, consisting of cyano, amino, aminocarbonyl, halogen, (C₁₋₄)alkyl, halogen-(C₁₋₄)alkyl, hydroxy, oxo, (C₁₋₄)alkoxy, halogen-(C₁₋₄)alkoxy, (C₁₋₄)alkylthio, halogen-(C₁₋₄)alkylthio, (C₁₋₄)alkoxy-(C₁₋₄)alkyl, (C₁₋₄)alkoxy-(C₁₋₄)alkoxy, (C₁₋₄)alkoxy-(C₁₋₄)alkylthio, (C_{1- 4})alkylthio-(C₁₋₄)alkyl, (C₁₋₄)alkylthio-(C₁₋₄)alkoxy, (C₁₋₄)alkylthio-(C₁₋₄)alkylthio, (C₂₋₄)alkenyl, (C₁₋₄)alkynyl, (C₂₋₄)alkenoxy, and (C₂₋₄)alkynoxy;
R₅ is hydrogen or fluoro;
R₆ is methyl, fluoromethyl, or difluoromethyl;
R₇ is hydrogen, (C₁₋₆)alkyl, halogen-(C₁₋₆)alkyl, (C₁₋₄)alkoxy-(C₁₋₄)alkyl, (C₁₋₆)alkylcarbonyl, (C₁₋₆)alkoxycarbonyl, halogen-(C₁₋₆)alkoxycarbonyl, (C₁₋₆)alkoxy-(C₁₋₆)alkylcarbonyl, (C₃₋₆)cycloalkyl, (C₃₋₆)cycloalkyl-carbonyl, or a heteroaryl group optionally substituted by 1, 2, or 3 substituents independently selected from the group consisting of cyano, halogen, hydroxyl, (C₁₋₄)alkyl, halogen-(C₁₋₄)alkyl, (C₁₋₄)alkoxy, halogen-(C₁₋₄)alkoxy, (C₁₋₃)alkoxy-(C₁₋₃)alkyl and (C₁₋₃)alkoxy-(C₁₋₃)alkoxy;
either
each of R₈ and R₉ is hydrogen;
or
R₈ and R₉, taken together, are oxo; and
either
each of R₁₂ and R₁₃ is independently selected from the group, consisting of hydrogen, (C₁₋₃)alkyl and halogen-(C₁₋₃)alkyl;
or
R₁₂ and R₁₃, taken together, are oxo;
in free form or in pharmaceutically acceptable salt form.

In yet a further embodiment, the invention relates to a compound of the formula 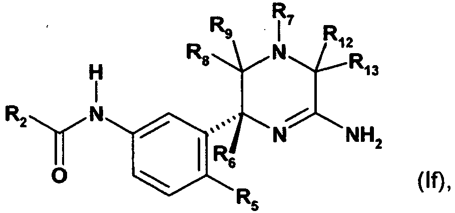 in which
R₂ is a pyridyl or pyrazinyl group which is substituted by 1, 2 or 3 substituents and wherein one of the substituents is located at the *para* position of the pyridyl or pyrazinyl group relative to the amide linker and wherein the substituents are independently selected from the group, consisting of cyano, halogen, (C₁₋₄)alkyl, halogen-(C₁₋₄)alkyl, hydroxy, oxo, (C₁₋₄)alkoxy and halogen-(C₁₋₄)alkoxy;
R₅ is hydrogen or fluoro;
R₆ is methyl, fluoromethyl, or difluoromethyl;
R₇ is hydrogen, (C₁₋₆)alkyl, halogen-(C₁₋₆)alkyl, (C₁₋₄)alkoxy-(C₁₋₄)alkyl, (C₁₋₆)alkylcarbonyl, (C₁₋₆)alkoxycarbonyl, halogen-(C₁₋₆)alkoxycarbonyl, (C₁₋₆)alkoxy-(C₁₋₆)alkylcarbonyl, (C₃₋₆)cycloalkyl, (C₃₋₆)cycloalkyl-carbonyl, or a heteroaryl group optionally substituted by 1, 2, or 3 substituents independently selected from the group consisting of cyano, halogen, hydroxyl, (C₁₋₄)alkyl, halogen-(C₁₋₄)alkyl, (C₁₋₄)alkoxy, halogen-(C₁₋₄)alkoxy, (C₁₋₃)alkoxy-(C₁₋₃)alkyl and (C₁₋₃)alkoxy-(C₁₋₃)alkoxy;
either
each of R₈ and R₉ is hydrogen;
or
R₈ and R₉, taken together, are oxo; and
either
each of R₁₂ and R₁₃ is independently selected from the group, consisting of hydrogen, (C₁₋₃)alkyl and halogen-(C₁₋₃)alkyl;
or
R₁₂ and R₁₃, taken together, are oxo;
in free form or in pharmaceutically acceptable salt form.

In another embodiment, the invention relates to a compound of the invention, or a pharmaceutically acceptable salt thereof, which is selected from:
5-Bromo-pyridine-2-carboxylic acid [3-(6-amino-2,4-dimethyl-3-oxo-2,3,4,5-tetrahydro-pyrazin-2-yl)-4-fluoro-phenyl]-amide;
5-Bromo-pyridine-2-carboxylic acid [3-(6-amino-2,4-dimethyl-2,3,4,5-tetrahydro-pyrazin-2-yl)-4-fluoro-phenyl]-amide;
5-Bromo-pyridine-2-carboxylic acid [3-(6-amino-2,4-dimethyl-3-oxo-2,3,4,5-tetrahydro-pyrazin-2-yl)-phenyl]-amide;
5-Bromo-pyridine-2-carboxylic acid [3-(6-amino-2,4-dimethyl-5-oxo-2,3,4,5-tetrahydro-pyrazin-2-yl)-4-fluoro-phenyl]-amide;
5-Chloro-pyridine-2-carboxylic acid [3-(6-amino-2,4-dimethyl-5-oxo-2,3,4,5-tetrahydro-pyrazin-2-yl)-4-fluoro-phenyl]-amide;
5-Bromo-pyridine-2-carboxylic acid [3-(6-amino-4-ethyl-2-methyl-5-oxo-2,3,4,5-tetrahydro-pyrazin-2-yl)-4-fluoro-phenyl]-amide;
3,5-Dichloro-pyridine-2-carboxylic acid [3-(6-amino-2,4-dimethyl-5-oxo-2,3,4,5-tetrahydro-pyrazin-2-yl)-4-fluoro-phenyl]-amide;
3,5-Dichloro-pyridine-2-carboxylic acid [3-(6-amino-4-ethyl-2-methyl-5-oxo-2,3,4,5-tetrahydro-pyrazin-2-yl)-4-fluoro-phenyl]-amide;
5-Bromo-pyridine-2-carboxylic acid [3-(6-amino-4-isopropyl-2-methyl-5-oxo-2,3,4,5-tetrahydro-pyrazin-2-yl)-4-fluoro-phenyl]-amide;
3,5-Dichloro-pyridine-2-carboxylic acid {3-[6-amino-4-(2-methoxy-ethyl)-2-methyl-5-oxo-2,3,4,5-tetrahydro-pyrazin-2-yl]-4-fluoro-phenyl}-amide;
5-Bromo-pyridine-2-carboxylic acid {3-[6-amino-4-(2-methoxy-ethyl)-2-methyl-5-oxo-2,3,4,5-tetrahydro-pyrazin-2-yl]-4-fluoro-phenyl}-amide;
5-Bromo-pyridine-2-carboxylic acid {3-[6-amino-2-methyl-4-(1-methyl-1H-pyrazol-4-yl)-5-oxo-2,3,4,5-tetrahydro-pyrazin-2-yl]-4-fluoro-phenyl}-amide;
5-Bromo-pyridine-2-carboxylic acid [3-(6-amino-2-methyl-5-oxo-4-pyridin-3-yl-2,3,4,5-tetrahydro-pyrazin-2-yl)-4-fluoro-phenyl]-amide;
5-Cyano-pyridine-2-carboxylic acid [3-(6-amino-4-ethyl-2-methyl-5-oxo-2,3,4,5-tetrahydro-pyrazin-2-yl)-4-fluoro-phenyl]-amide;
5-Bromo-pyridine-2-carboxylic acid [3-(6-amino-5-ethyl-2,4-dimethyl-3-oxo-2,3,4,5-tetrahydro-pyrazin-2-yl)-phenyl]-amide;
5-Bromo-pyridine-2-carboxylic acid [3-(6-amino-2-difluoromethyl-2,3,4,5-tetrahydro-pyrazin-2-yl)-4-fluoro-phenyl]-amide;
5-Cyano-pyridine-2-carboxylic acid [3-(6-amino-2-difluoromethyl-2,3,4,5-tetrahydro-pyrazin-2-yl)-4-fluoro-phenyl]-amide;
5-Cyano-pyridine-2-carboxylic acid [3-(6-amino-2-difluoromethyl-5-oxo-2,3,4,5-tetrahydro-pyrazin-2-yl)-4-fluoro-phenyl]-amide;
5-Amino-3-{5-[(5-bromo-pyridine-2-carbonyl)-amino]-2-fluoro-phenyl}-3-difluoromethyl-3,6-dihydro-2H-pyrazine-1-carboxylic acid methyl ester;
5-Amino-3-{5-[(5-cyano-pyridine-2-carbonyl)-amino]-2-fluoro-phenyl}-3-difluoromethyl-3,6-dihydro-2H-pyrazine-1-carboxylic acid methyl ester;
5-Bromo-3-methyl-pyridine-2-carboxylic acid [3-(4-acetyl-6-amino-2-difluoromethyl-2,3,4,5-tetrahydro-pyrazin-2-yl)-4-fluoro-phenyl]-amide;
5-Amino-3-{5-[(5-bromo-3-methyl-pyridine-2-carbonyl)-amino]-2-fluoro-phenyl}-3-difluoromethyl-3,6-dihydro-2H-pyrazine-1-carboxylic acid 2,2-dichloro-ethyl ester;
5-Bromo-3-methyl-pyridine-2-carboxylic acid {3-[6-amino-2-difluoromethyl-4-(2-methoxy-acetyl)-2,3,4,5-tetrahydro-pyrazin-2-yl]-4-fluoro-phenyl}-amide;
5-Bromo-3-methyl-pyridine-2-carboxylic acid [3-(6-amino-4-cyclopropanecarbonyl-2-difluoromethyl-2,3,4,5-tetrahydro-pyrazin-2-yl)-4-fluoro-phenyl]-amide;
5-Bromo-3-methyl-pyridine-2-carboxylic acid [3-(6-amino-2-difluoromethyl-2,3,4,5-tetrahydro-pyrazin-2-yl)-4-fluoro-phenyl]-amide;
5-Difluoromethoxy-3-methyl-pyridine-2-carboxylic acid [3-(4-acetyl-6-amino-2-difluoromethyl-2,3,4,5-tetrahydro-pyrazin-2-yl)-4-fluoro-phenyl]-amide;
5-Difluoromethoxy-3-methyl-pyridine-2-carboxylic acid [3-(6-amino-2-difluoromethyl-2,3,4,5-tetrahydro-pyrazin-2-yl)-4-fluoro-phenyl]-amide;
3-Amino-5-methoxy-pyrazine-2-carboxylic acid [3-(6-amino-2-difluoromethyl-2,3,4,5-tetrahydro-pyrazin-2-yl)-4-fluoro-phenyl]-amide; and
3-Amino-5-oxo-4,5-dihydro-pyrazine-2-carboxylic acid [3-(6-amino-2-difluoromethyl-2,3,4,5-tetrahydro-pyrazin-2-yl)-4-fluoro-phenyl]-amide.

In a further aspect, the invention relates to a process for the preparation of a compound of the formula I, in free form or in salt form, comprising
a) the reaction of a compound of the formula 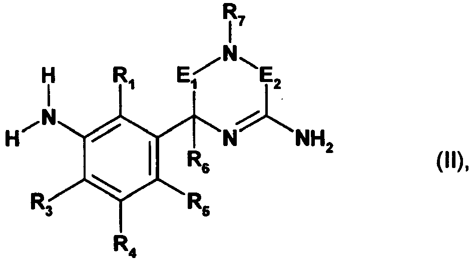 in which R₁, R₃, R₄, R₅, R₆, R₇, E₁ and E₂ are as defined for the formula I, in free form or in salt form, with a compound of the formula 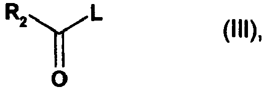 in which R₂ is as defined for the formula I and L is a leaving group, in free form or in salt form,
b) the optional reduction, oxidation or other functionalisation of the resulting compound,
c) the cleavage of any protecting group(s) optionally present and
d) the recovery of the so obtainable compound of the formula I in free form or in salt form.

The reactions can be effected according to conventional methods, for example as described in the Examples.

The working-up of the reaction mixtures and the purification of the compounds thus obtainable may be carried out in accordance with known procedures.

Salts may be prepared from free compounds in known manner, and vice-versa.

Compounds of the formula I can also be prepared by further conventional processes, which processes are further aspects of the invention, for example as described in the Examples.

The starting materials of the formulae II and III are known or may be prepared according to conventional procedures starting from known compounds, may be prepared from known compounds as described in the Examples or may be prepared using procedures analogous to those described in the Examples.

Compounds of the formula I, in free form, salt form, or in pharmaceutically acceptable salt form, hereinafter often referred to as "agents of the invention", exhibit valuable pharmacological properties, when tested *in vitro* or *in vivo,* and are, therefore, useful in medicaments, in therapy or for use as research chemicals, for example as tool compounds.

E. g., agents of the invention are inhibitors of aspartic proteases and can be used for the treatment or prevention of a condition, disease or disorder involving processing by such enzymes. Particularly, agents of the invention inhibit beta-secretase and, thus, the generation of beta-amyloid and the subsequent aggregation into oligomers and fibrils.

The inhibiting properties of an agent of the invention towards proteases can be evaluated in tests as described hereinafter.

### Test 1: Inhibition of human BACE-1

Recombinant BACE-1 (extracellular domain, expressed in baculovirus and purified using standard methods) at 0.1 to 10 nM concentrations is incubated with the test compound at various concentrations for 1 hour at room temperature in 10 to 100 mM acetate buffer, pH 4.5, containing 0.1 % CHAPS. Synthetic fluorescence-quenched peptide substrate, derived from the sequence of APP and containing a suitable fluorophore-quencher pair, is added to a final concentration of 1 to 5 µM, and the increase in fluorescence is recorded at a suitable excitation / emission wavelength in a microplate spectro-fluorimeter for 5 to 30 minutes in 1-minute intervals. IC₅₀ values are calculated from percentage of inhibition of BACE-1 activity as a function of the test compound concentration.

### Test 2: Inhibition of human BACE-2

Recombinant BACE-2 (extracellular domain, expressed in baculovirus and purified using standard methods) at 0.1 to 10 nM concentrations is incubated with the test compound at various concentrations for 1 hour at room temperature in 10 to 100 mM acetate buffer, pH 4.5, containing 0.1 % CHAPS. Synthetic peptide substrate, derived from the sequence of APP and containing a suitable fluorophore-quencher pair, is added to a final concentration of 1 to 5 µM, and the increase in fluorescence is recorded at a suitable excitation / emission wavelength in a microplate spectro-fluorimeter for 5 to 30 minutes in 1-minute intervals. IC₅₀ values are calculated from percentage of inhibition of BACE-2 activity as a function of the test compound concentration.

### Test 3: Inhibition of human cathepsin D

Recombinant cathepsin D (expressed as procathepsin D in baculovirus, purified using standard methods and activated by incubation in sodium formate buffer pH 3.7) is incubated with the test compound at various concentrations for 1 hour at room temperature in sodium formate or sodium acetate buffer at a suitable pH within the range of pH 3.0 to 5.0. Synthetic peptide substrate Mca-Gly-Lys-Pro-Ile-Leu-Phe-Phe-Arg-Leu-Lys(DNP)-D-Arg-NH₂ is added to a final concentration of 1 to 5 µM, and the increase in fluorescence is recorded at excitation of 325 nm and emission at 400 nm in a microplate spectro-fluorimeter for 5 to 30 minutes in 1-minute intervals. IC₅₀ values are calculated from the percentage of inhibition of cathepsin D-activity as a function of the test compound concentration.

### Test 4: Inhibition of cellular release of amyloid peptide 1-40

Chinese hamster ovary cells are transfected with the gene for amyloid precursor protein. The cells are plated at a density of 8000 cells/well into 96-well microtiter plates and cultivated for 24 hours in DMEM cell culture medium containing 10 % FCS. The test compound is added to the cells at various concentrations, and the cells are cultivated for 24 hours in the presence of the test compound. The supernatants are collected, and the concentration of amyloid peptide 1-40 is determined using state of the art immunoassay techniques, for example sandwich ELISA, homogenous time-resolved fluorescence (HTRF) immunoassay, or electrochemiluminescence immunoassay. The potency of the compound is calculated from the percentage of inhibition of amyloid peptide release as a function of the test compound concentration.

Agents of the invention were tested in at least one of the above-described tests. Specific activities of agents of the invention are described in Example 30.

As used herein, the term "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated.

The term "a therapeutically effective amount" of a compound of the present invention refers to an amount of the compound of the present invention that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of the compound of the present invention that, when administered to a subject, is effective to (1) at least partially alleviating, inhibiting, preventing and/or ameliorating a condition, or a disorder or a disease (i) mediated by BACE-1 or (ii) associated with BACE-1 activity, or (iii) characterized by activity (normal or abnormal) of BACE-1; or (2) reducing or inhibiting the activity of BACE-1. In another non-limiting embodiment, the term "a therapeutically effective amount" refers to the amount of the compound of the present invention that, when administered to a cell, or a tissue, or a non-cellular biological material, or a medium, is effective to at least partially reduce or inhibit the activity of BACE-1. The meaning of the term "a therapeutically effective amount" as illustrated in the above embodiments for BACE-1 also applies by the same means to any other relevant proteins/peptides/enzymes, such as BACE-2, or cathepsin D.

As used herein, the term "subject" refers to an animal. Typically the animal is a mammal. A subject also refers to for example, primates (e.g., humans, male or female), cows, sheep, goats, horses, dogs, cats, rabbits, rats, mice, fish, birds and the like. In certain embodiments, the subject is a primate. In yet other embodiments, the subject is a human.

As used herein, the term "inhibit", "inhibition" or "inhibiting" refers to the reduction or suppression of a given condition, symptom, or disorder, or disease, or a significant decrease in the baseline activity of a biological activity or process.

As used herein, the term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (e.g., stabilization of a discernible symptom), physiologically, (e.g., stabilization of a physical parameter), or both.

As used herein, the term "prevention" of any particular disease or disorder refers to the administration of a compound of the invention to a subject before any symptoms of that disease or disorder are apparent.

As used herein, a subject is "in need of a treatment if such subject would benefit biologically, medically or in quality of life from such treatment.

As used herein, the term an "agent" of the invention is used interchangeably with the term a "compound" of the invention and has no difference in meaning therefrom.

As used herein, the term "a," "an," "the" and similar terms used in the context of the present invention (especially in the context of the claims) are to be construed to cover both the singular and plural unless otherwise indicated herein or clearly contradicted by the context. The use of any and all examples, or exemplary language (e.g. "such as') provided herein is intended merely to better illuminate the invention and does not pose a limitation on the scope of the invention otherwise claimed.

Due to their inhibiting properties towards proteases, agents of the invention are useful, e. g., in the treatment or prevention of a variety of disabilitating psychiatric, psychotic, neurological or vascular states, e. g. of a condition, disease or disorder of the vascular system or of the nervous system, in which beta-amyloid generation or aggregation plays a role, or, based on the inhibition of BACE-2 (beta-site APP-cleaving enzyme 2) or cathepsin D, which are dose homologues of the pepsin-type aspartyl proteases and beta-secretase, and the correlation of the BACE-2 or cathepsin D expression with a more tumorigenic or metastatic potential of tumor cells, as anti-cancer medicaments, e. g. in the suppression of the metastasis process associated with tumor cells. The said condition, disease or disorder of the vascular system or of the nervous system is exemplified by, and includes, without limitation, an anxiety disorder, such as panic disorder with or without agoraphobia, agoraphobia without history of panic disorder, an animal or other specific phobia, including a social phobia, social anxiety disorder, anxiety, obsessive-compulsive disorder, a stress disorder, including post-traumatic or acute stress disorder, or a generalized or substance-induced anxiety disorder; a neurosis; seizures; epilepsy, especially partial seizures, simple, complex or partial seizures evolving to secondarily generalized seizures or generalized seizures [absence (typical or atypical), myoclonic, clonic, tonic, tonic-clonic or atonic seizures]; convulsions; migraine; an affective disorder, including a depressive or bipolar disorder, e. g. single-episode or recurrent major depressive disorder, major depression, a dysthymic disorder, dysthymia, depressive disorder NOS, bipolar I or bipolar II manic disorder or cyclothymic disorder; a psychotic disorder, including schizophrenia or depression; neurodegeneration, e. g. neurodegeneration arising from cerebral ischemia; an acute, traumatic or chronic degenerative process of the nervous system, such as Parkinson's disease, Down's syndrome, dementia, e. g. senile dementia, dementia with Lewy bodies or a fronto-temporal dementia, a cognitive disorder, cognitive impairment, e. g. mild cognitive impairment, memory impairment, an amyloid neuropathy, a peripheral neuropathy, Alzheimer's disease, Gerstmann-Straeussler-Scheinker syndrome, Niemann-Pick disease, e. g. Niemann-Pick type C disease, brain inflammation, a brain, spinal cord or nerve injury, e. g. traumatic brain injury (TBI), a nerve trauma or a brain trauma, vascular amyloidosis, cerebral haemorrhage with amyloidosis, Huntington's chorea, amyotrophic lateral sclerosis, multiple sclerosis or fragile X syndrome; scrapie; cerebral amyloid angiopathy; an encephalopathy, e. g. transmissible spongiform encephalopathy; stroke; an attention disorder, e. g. attention deficit hyperactivity disorder; Tourette's syndrome; a speech disorder, including stuttering; a disorder of the circadian rhythm, e. g. in subjects suffering from the effects of jet lag or shift work; pain; nociception; itch; emesis, including acute, delayed or anticipatory emesis, such as emesis induced by chemotherapy or radiation, motion sickness, or post-operative nausea or vomiting; an eating disorder, including anorexia nervosa or bulimia nervosa; premenstrual syndrome; a muscle spasm or spasticity, e. g. in paraplegic patients; a hearing disorder, e. g. tinnitus or age-related hearing impairment; urinary incontinence; glaucoma; inclusion-body myositis; or a substance-related disorder, including substance abuse or dependency, including a substance, such as alcohol, withdrawal disorder. Agents of the invention may also be useful in enhancing cognition, e. g. in a subject suffering from a dementing condition, such as Alzheimer's disease; as pre-medication prior to anaesthesia or a minor medical intervention, such as endoscopy, including gastric endoscopy; or as ligands, e. g. radioligands or positron emission tomography (PET) ligands.

For the above-mentioned indications, the appropriate dosage will vary depending on, e. g., the compound employed as active pharmaceutical ingredient, the host, the mode of administration, the nature and severity of the condition, disease or disorder or the effect desired. However, in general, satisfactory results in animals are indicated to be obtained at a daily dosage of from about 0.1 to about 100, preferably from about 1 to about 50, mg/kg of animal body weight. In larger mammals, for example humans, an indicated daily dosage is in the range of from about 0.5 to about 2000, preferably from about 2 to about 200, mg of an agent of the invention conveniently administered, for example, in divided doses up to four times a day or in sustained release form.

An agent of the invention may be administered by any conventional route, in particular en-terally, preferably orally, e. g. in the form of a tablet or capsule, or parenterally, e. g. in the form of an injectable solution or suspension.

In a further aspect, the invention relates to a pharmaceutical composition comprising an agent of the invention as active pharmaceutical ingredient in association with at least one pharmaceutically acceptable carrier or diluent and optionally in association with other auxiliary substances, such as inhibitors of cytochrome P450 enzymes, agents preventing the degradation of active pharmaceutical ingredients by cytochrome P450, agents improving or enhancing the pharmacokinetics of active pharmaceutical ingredients, agents improving or enhancing the bioavailability of active pharmaceutical ingredients, and so on, e. g. grapefruit juice, ketoconazole or, preferably, ritonavir. Such a composition may be manufactured in conventional manner, e. g. by mixing its components. Unit dosage forms contain, e. g., from about 0.1 to about 1000, preferably from about 1 to about 500, mg of an agent of the invention.

In addition, the pharmaceutical compositions of the present invention can be made up in a solid form (including without limitation capsules, tablets, pills, granules, powders or suppositories), or in a liquid form (including without limitation solutions, suspensions or emulsions): The pharmaceutical compositions can be subjected to conventional pharmaceutical operations such as sterilization and/or can contain conventional inert diluents, lubricating agents, or buffering agents, as well as adjuvants, such as preservatives, stabilizers, wetting agents, emulsifers and buffers, etc.

Typically, the pharmaceutical compositions are tablets or gelatin capsules comprising the active ingredient together with
a) diluents, e.g., lactose, dextrose, sucrose, mannitol, sorbitol, cellulose and/or glycine;
b) lubricants, e.g., silica, talcum, stearic acid, its magnesium or calcium salt and/or polyethyleneglycol; for tablets also
c) binders, e.g., magnesium aluminum silicate, starch paste, gelatin, tragacanth, methylcellulose, sodium carboxymethylcellulose and/or polyvinylpyrrolidone; if desired
d) disintegrants, e.g., starches, agar, alginic acid or its sodium salt, or effervescent mixtures; and/or
e) absorbents, colorants, flavors and sweeteners.

Tablets may be either film coated or enteric coated according to methods known in the art.

Suitable compositions for oral administration include an effective amount of a compound of the invention in the form of tablets, lozenges, aqueous or oily suspensions, dispersible powders or granules, emulsion, hard or soft capsules, or syrups or elixirs. Compositions intended for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations. Tablets may contain the active ingredient in admixture with nontoxic pharmaceutically acceptable excipients which are suitable for the manufacture of tablets. These excipients are, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Certain injectable compositions are aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-75%, or contain about 1-50%, of the active ingredient.

Suitable compositions for transdermal application include an effective amount of a compound of the invention with a suitable carrier. Carriers suitable for transdermal delivery include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

Suitable compositions for topical application, e.g., to the skin and eyes, include aqueous solutions, suspensions, ointments, creams, gels or sprayable formulations, e.g., for delivery by aerosol or the like. Such topical delivery systems will in particular be appropriate for dermal application, e.g., for the treatment of skin cancer, e.g., for prophylactic use in sun creams, lotions, sprays and the like. They are thus particularly suited for use in topical, including cosmetic, formulations well-known in the art. Such may contain solubilizers, stabilizers, tonicity enhancing agents, buffers and preservatives.

As used herein a topical application may also pertain to an inhalation or to an intranasal application. They may be conveniently delivered in the form of a dry powder (either alone, as a mixture, for example a dry blend with lactose, or a mixed component particle, for example with phospholipids) from a dry powder inhaler or an aerosol spray presentation from a pressurised container, pump, spray, atomizer or nebuliser, with or without the use of a suitable propellant.

The present invention further provides anhydrous pharmaceutical compositions and dosage forms comprising the compounds of the present invention as active ingredients, since water may facilitate the degradation of certain compounds.

Anhydrous pharmaceutical compositions and dosage forms of the invention can be prepared using anhydrous or low moisture containing ingredients and low moisture or low humidity conditions. An anhydrous pharmaceutical composition may be prepared and stored such that its anhydrous nature is maintained. Accordingly, anhydrous compositions are packaged using materials known to prevent exposure to water such that they can be included in suitable formulary kits. Examples of suitable packaging include, but are not limited to, hermetically sealed foils, plastics, unit dose containers (e. g., vials), blister packs, and strip packs.

The invention further provides pharmaceutical compositions and dosage forms that comprise one or more agents that reduce the rate by which the compound of the present invention as an active ingredient will decompose. Such agents, which are referred to herein as "stabilizers," include, but are not limited to, antioxidants such as ascorbic acid, pH buffers, or salt buffers, etc.

In accordance with the foregoing, in a further aspect, the invention relates to an agent of the invention for use as a medicament, for example for the treatment or prevention of a neurological or vascular condition, disease or disorder, in which beta-amyloid generation or aggregation plays a role, or for the suppression of the metastasis process associated with tumor cells. In a further embodiment, the invention relates to an agent of the invention for use in the treatment of a disease or disorder mediated by BACE-1, BACE-2 or cathepsin D activity. In one embodiment, the invention relates to an agent of the invention for use in the treatment of Alzheimer's Disease or mild cognitive impairment.

In a further aspect, the invention relates to the use of an agent of the invention as an active pharmaceutical ingredient in a medicament, for example for the treatment or prevention of a neurological or vascular condition, disease or disorder, in which beta-amyloid generation or aggregation plays a role, or for the suppression of the metastasis process associated with tumor cells. In a further embodiment, the invention relates to the use of an agent of the invention as an active pharmaceutical ingredient in a medicament for the treatment or prevention of a disease or disorder mediated by BACE-1, BACE-2 or cathepsin D activity. In one embodiment, the invention relates to the use of an agent of the invention as an active pharmaceutical ingredient in a medicament for the treatment or prevention of Alzheimer's Disease or mild cognitive impairment.

In a further aspect, the invention relates to the use of an agent of the invention for the manufacture of a medicament for the treatment or prevention of a neurological or vascular condition, disease or disorder, in which beta-amyloid generation or aggregation plays a role, or for the suppression of the metastasis process associated with tumor cells. In a further embodiment, the invention relates to the use of an agent of the invention for the manufacture of a medicament for the treatment or prevention of a disease or disorder mediated by BACE-1, BACE-2 or cathepsin D activity. In one embodiment, the invention relates to the use of an agent of the invention for the manufacture of a medicament for the treatment or prevention of Alzheimer's Disease or mild cognitive impairment.

In a further aspect, the invention relates to a method for the treatment or prevention of a neurological or vascular condition, disease or disorder, in which beta-amyloid generation or aggregation plays a role, or for the suppression of the metastasis process associated with tumor cells, in a subject in need of such treatment, prevention or suppression, which method comprises administering to such subject an effective amount of an agent of the invention. In one embodiment, the invention relates to a method of modulating BACE-1, BACE-2 or cathepsin D activity in a subject, wherein the method comprises administering to the subject a therapeutically effective amount of an agent of the invention. In another embodiment, the invention relates to a method for the treatment or prevention of a disease mediated by BACE-1, BACE-2 or cathepsin D activity, in a subject in need of such treatment or prevention, which method comprises administering to such subject an effective amount of an agent of the invention. In yet another embodiment, the invention relates to a method for the treatment or prevention of Alzheimer's Disease or mild cognitive impairment, in a subject in need of such treatment or prevention, which method comprises administering to such subject an effective amount of an agent of the invention.

An agent of the invention can be administered as sole active pharmaceutical ingredient or as a combination with at least one other active pharmaceutical ingredient effective, e. g., in the treatment or prevention of a neurological or vascular condition, disease or disorder, in which beta-amyloid generation or aggregation plays a role, or in the suppression of the metastasis process associated with tumor cells. Such a pharmaceutical combination may be in the form of a unit dosage form, which unit dosage form comprises a predetermined quantity of each of the at least two active components in association with at least one pharmaceutically acceptable carrier or diluent. Alternatively, the pharmaceutical combination may be in the form of a package comprising the at least two active components separately, e. g. a pack or dispenser-device adapted for the concomitant or separate administration of the at least two active components, in which these active components are separately arranged. In a further aspect, the invention relates to such pharmaceutical combinations.

In a further aspect, the invention therefore relates to a pharmaceutical combination comprising a therapeutically effective amount of an agent of the invention and a second drug substance, for simultaneous or sequential administration.

In one embodiment, the invention provides a product comprising a compound of an agent of the invention and at least one other therapeutic agent as a combined preparation for simultaneous, separate or sequential use in therapy. In one embodiment, the therapy is the treatment of a disease or condition mediated by BACE-1, BACE-2 or cathepsin D activity.

In one embodiment, the invention provides a pharmaceutical composition comprising an agent of the invention and another therapeutic agent(s). Optionally, the pharmaceutical composition may comprise a pharmaceutically acceptable excipient, as described above.

In one embodiment, the invention provides a kit comprising two or more separate pharmaceutical compositions, at least one of which contains an agent of the invention. In one embodiment, the kit comprises means for separately retaining said compositions, such as a container, divided bottle, or divided foil packet. An example of such a kit is a blister pack, as typically used for the packaging of tablets, capsules and the like. The kit of the invention may be used for administering different dosage forms, for example, oral and parenteral, for administering the separate compositions at different dosage intervals, or for titrating the separate compositions against one another. To assist compliance, the kit of the invention typically comprises directions for administration.

In the combination therapies of the invention, the agent of the invention and the other therapeutic agent may be manufactured and/or formulated by the same or different manufacturers. Moreover, the compound of the invention and the other therapeutic may be brought together into a combination therapy: (i) prior to release of the combination product to physicians (e.g. in the case of a kit comprising the compound of the invention and the other therapeutic agent); (ii) by the physician themselves (or under the guidance of the physician) shortly before administration; (iii) in the patient themselves, e.g. during sequential administration of the compound of the invention and the other therapeutic agent. Accordingly, the invention provides an agent of the invention for use in the treatment of a disease or condition mediated by BACE-1, BACE-2 or cathepsin D activity, wherein the medicament is prepared for administration with another therapeutic agent. The invention also provides the use of another therapeutic agent for treating a disease or condition mediated by BACE-1, BACE-2 or cathepsin D activity, wherein the medicament is administered with an agent of the invention.

The invention also provides an agent of the invention for use in a method of treating a disease or condition mediated by BACE-1, BACE-2 or cathepsin D activity, wherein the agent of the invention is prepared for administration with another therapeutic agent. The invention also provides another therapeutic agent for use in a method of treating a disease or condition mediated by BACE-1, BACE-2 or cathepsin D activity, wherein the other therapeutic agent is prepared for administration with an agent of the invention. The invention also provides an agent of the invention for use in a method of treating a disease or condition mediated by BACE-1, BACE-2 or cathepsin D activity, wherein the agent of the invention is administered with another therapeutic agent. The invention also provides another therapeutic agent for use in a method of treating a disease or condition mediated by BACE-1, BACE-2 or cathepsin D activity, wherein the other therapeutic agent is administered with an agent of the invention.

The invention also provides the use of an agent of the invention for treating a disease or condition mediated by BACE-1, BACE-2 or cathepsin D activity, wherein the patient has previously (e.g. within 24 hours) been treated with another therapeutic agent. The invention also provides the use of another therapeutic agent for treating a disease or condition mediated by BACE-1, BACE-2 or cathepsin D activity, wherein the patient has previously (e.g. within 24 hours) been treated with an agent of the invention.

In one embodiment, the invention relates to a compound of the invention in combination with another therapeutic agent wherein the other therapeutic agent is selected from:
(a) acetylcholinesterase inhibitors, such as donepezil (Aricept™), rivastigmine (Exelon™) and galantamine (Razadyne™);
(b) glutamate antagonists, such as memantine (Namenda™);
(c) antidepressant medications for low mood and irritability, such as citalopram (Celexa™), fluoxetine (Prozac™), paroxeine (Paxil™), sertraline (Zoloft™) and trazodone (Desyrel™);
(d) anxiolytics for anxiety, restlessness, verbally disruptive behavior and resistance, such as lorazepam (Ativan™) and oxazepam (Serax™);
(e) antipsychotic medications for hallucinations, delusions, aggression, agitation, hostility and uncooperativeness, such as aripiprazole (Abilify™), clozapine (Clozaril™), haloperidol (Haldol™), olanzapine (Zyprexa™), quetiapine (Seroquel™), risperidone (Risperdal™) and ziprasidone (Geodon™);
(f) mood stabilizers, such as carbamazepine (Tegretol™) and divalproex (Depakote™);
(g) nicotinic apha-7 agonists;
(h) mGluR5 antagonists;
(i) H3 agonists; and
(j) amyloid therapy vaccines.

The following Examples illustrate the invention, but do not limit it.

### Examples

### Abbreviations

| | |
|---|---|
| Ac | acetyl |
| ACN | acetonitrile |
| aq | aqueous |
| Boc | tert-butoxycarbonyl |
| conc | concentrated |
| DCM | dichloromethane |
| DIPE | diisopropyl ether |
| DIPEA | diisopropylethylamine |
| DMSO | dimethylsulfoxide |
| EDC | 1-(3-dimethylaminopropyl)-3-ethylcarbodiimide |
| eq | equivalent(s) |
| ESIMS | electrospray ionization mass spectrometry |
| Et | ethyl |
| h | hour(s) |
| HPLC | high performance liquid chromatography |
| HOAt | 1-hydroxy-7-azabenzotriazole |
| Me | methyl |
| min | minute(s) |
| NMR | nuclear magnetic resonance spectrometry |
| rt | room temperature |
| R_{f} | retention factor (TLC) |
| Rt | retention time |
| TBME | tert-butyl-methyl-ether |
| tBu | tert-butyl |
| TFA | trifluoroacetic acid |
| THF | tetrahydrofuran |

### General chromatography information

| **HPLC method H1 (Rt_{H1}):** | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA, B) ACN + 0.05 Vol.-% TFA |
| HPLC-gradient | 0-100 % B in 3.25 min, flow = 0.7 ml/min |
| | |

| **HPLC method H2 (Rt_{H2}):** | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA, B) ACN + 0.05 Vol.-% TFA |
| HPLC-gradient: | 10-100% B in 3.25 min, flow = 0.7 ml/min |
| | |

| **HPLC method H3 (Rt_{H3}):** | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA, B) ACN + 0.05 Vol.-% TFA |
| HPLC-gradient: | 30-100% B in 3.25 min, flow = 0.7 ml/min |

| **HPLC method H4 (Rt_{H4}):** | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA, B) ACN + 0.05 Vol.-% TFA |
| HPLC-gradient: | 40-100% B in 3.25 min, flow = 0.7 ml/min |
| | |

| **HPLC method H5 (Rt_{H5}):** | |
|---|---|
| HPLC-column dimensions: | 3.0 x 30 mm |
| HPLC-column type: | Zorbax SB-C8, 1.8 µm |
| HPLC-eluent: | A) water + 0.05 Vol.-% TFA, B) ACN + 0.05 Vol.-% TFA |
| HPLC-gradient: | 10 - 95 % B in 2.00 min, 95 % B 2.00 min, flow = 0.7 ml/min |

| **HPLC method H6 (Rt_{H6}):** | |
|---|---|
| HPLC-column dimensions: | 3.0 x 30 mm |
| HPLC-column type: | Zorbax SB-C18, 1.8 µm |
| HPLC-eluent: | A) water + 0.05 Vol.-% TFA, B) ACN + 0.05 Vol.-% TFA |
| HPLC-gradient: | 50-100% B in 3.25 min, flow = 0.7 ml/min |
| | |

| **UPLC method H7 (Rt_{H7}):** | |
|---|---|
| HPLC-column dimensions: | 2.1 x 50 mm |
| HPLC-column type: | Acquity UPLC HSS T3, 1.8 µm |
| HPLC-eluent: | A) water + 0.05 Vol.-% formic acid + 3.75 mM ammonium acetate B) ACN + 0.04 Vol.-% formic acid |
| HPLC-gradient: | 2 - 98 % B in 1.7 min, 98% B 0.45 min, flow = 1.2 ml/min |
| | |

| **HPLC method H8 (Rt_{H8}):** | |
|---|---|
| HPLC-column dimensions: | 2.1 x 30 mm |
| HPLC-column type: | Ascentis Express C18, 2.8 µm |
| HPLC-eluent: | A) water + 0.05 Vol.-% formic acid + 3.75 mM ammonium acetate, B) ACN + 0.04 Vol.-% formic acid |
| HPLC-gradient: | 2 - 98 % B in 1.4 min, 0.75 min 98% B, flow = 1.2 ml/min |
| HPLC-column temperature: | 50 °C |

### Example 1: 5-Bromo-pyridine-2-carboxylic acid [3-(6-amino-2,4-dimethyl-3-oxo-2,3,4,5-tetrahydro-pyrazin-2-yl)-4-fluoro-phenyl]-amide

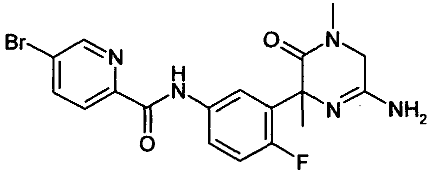

### 1a) 2-Amino-2-(5-bromo-2-fluoro-phenyl)-propionic acid

10 g (50 mmol) of 1-(5-bromo-2-fluoro-phenyl)-ethanone, 6.4 g (100 mmol) of NH₄Cl and 6.5 g (100 mmol) of KCN were dissolved in 200 ml of aq NH₃. The mixture was stirred at rt overnight and extracted with Et₂O, the organic phase was washed with water and brine, dried over Na₂SO₄ and filtered, the filtrate was concentrated *in vacuo* and taken up in 100 ml of conc hydrochloric acid, and the mixture was refluxed overnight and concentrated *in vacuo.* The residue was washed twice with DIPE to give the hydrochloride salt of the title compound in the form of a beige powder {HPLC: Rt_{H1} = 2.137 min; ESIMS: 262, 264 [(M+H)⁺, 1 Br]; ¹H-NMR (360 MHz, D₂O): 7.63 (dd, 1 H), 7.51 (ddd, 1 H), 7.01 (dd, 1 H), 3.56 (s, 3H)}. An aq solution of the salt was treated with 2.2 eq of 2N aq NaOH, washed with TBME and neutralized with 1.2 eq of 2N HCl. The title compound crystallized from the aq solution in the form of colourless crystals {¹H-NMR (400 MHz, CD₃OD): 7.72 (dd, 1 H), 7.57 (ddd, 1 H), 7.13 (dd, 1 H), 1.87 (s, 3H)}.

### 1b) 2-Amino-2-(5-bromo-2-fluoro-phenyl)-propionic acid methyl ester

MeOH (530 ml) was cooled to -10°C and treated dropwise with 134 ml (1.84 mmol) of SOCl₂. Compound 1a) (50 g, 167.5 mmol) was added in portions. The mixture was slowly heated, stirred at reflux for 18 h, concentrated, taken up in water, washed with TBME, basified with K₂CO₃ and extracted three times with DCM. The combined organic layers were dried over K₂CO₃ and evaporated to yield the title compound in the form of a resin {HPLC: Rt_{H2} = 2.266 min; ESIMS: 276, 278 [(M+H)⁺, 1Br]; ¹H-NMR (360 MHz, DMSO-d₆): 7.91 (dd, 1H), 7.54 (ddd, 1H), 7.17 (dd, 1H), 3.62 (s, 3H), 1.48 (s, 3H)}.

### 1c) 2-(5-Bromo-2-fluoro-phenyl)-2-(2-chloro-acetylamino)-propionic acid methyl ester

To a solution of compound 1b) (3.25 g, 11.77 mmol) in 30 ml of DCM were added at -5°C 2.67 ml (15.30 mmol) of DIPEA and then dropwise 1.037 ml (12.95 mmol) of chloroacetyl chloride. The mixture was stirred for 30 min at -5°C and then for 1 h without cooling and diluted with TBME and water. The organic phase was washed with water, 1 N HCl and brine, dried over MgSO₄ x H₂O and evaporated. Crystallization from EtOAc yielded the title compound in the form of greyish crystals {HPLC: Rt_{H2} = 3.415 min; ESIMS: 352, 354 [(M+H)⁺, 1Br]; ¹H-NMR (360 MHz, CDCl₃): 8.19 (br, 1 H), 7.75 (dd, 1 H), 7.45 (ddd, 1H), 6.94 (dd, 1 H), 3.99 (d, 1 H), 3.92 (d, 1 H), 3.81 (s, 3H), 2.09 (s, 3H)}.

### 1d) 3-(5-Bromo-2-fluoro-phenyl)-1,3-dimethyl-piperazine-2,5-dione

To a suspension of compound 1c) (353 mg, 1 mmol) in EtOH were added 2.5 ml of MeNH₂ (33 % in EtOH). The mixture was stirred for 1.5 h at 50°C and evaporated. Crystallization from TBME / hexane yielded the title compound in the form of colourless crystals {HPLC: Rt_{H2} = 2.337 min; ESIMS: 315, 317 [(M+H)⁺, 1Br]; ¹H-NMR (360 MHz, DMSO-d₆): 8.67 (br, 1 H), 7.66 - 7.57 (m, 2H), 7.24 (dd, 1 H), 4.13 (d, 1 H), 3.96 (d, 1 H), 2.89 (s, 3H), 1.79 (s, 3H)}.

### 1e) 3-(5-Bromo-2-fluoro-phenyl)-1,3-dimethyl-5-thioxo-piperazin-2-one

A mixture of compound 1d) (158 mg, 0.5 mmol), 142 mg (0.35 mmol) of Lawesson's reagent and 2 ml of THF was stirred for 2 h at 50°C, cooled and filtered to yield the title compound in the form of colourless crystals {HPLC: Rt_{H2}= 2.837 min; ESIMS: 331, 333 [(M+H)⁺, 1Br]; ¹H-NMR (600 MHz, DMSO-d₆): 11.00 (s, 1H), 7.65 (ddd, 1 H), 7.60 (dd, 1 H), 7.24 (dd, 1H), 4.61 (d, 1 H), 4.42 (d, 1H), 2.88 (s, 3H), 1.80 (s, 3H)}.

### 1f) 5-Amino-3-(5-bromo-2-fluoro-phenyl)-1,3-dimethyl-3,6-dihydro-1H-pyrazin-2-one

To a suspension of compound 1e) (2.03 g, 6.13 mmol) in 30 ml of MeOH and 30 ml of THF were added 11.6 ml of aq NH₃ (25 %) and 9.6 ml of tBuOOH (80 % in water). The mixture was stirred overnight at 40°C, cooled down and treated with sodium thiosulphate to destroy excess peroxide. MeOH and THF were evaporated, the residue was extracted with EtOAc, the organic phase was extracted twice with 1 N HCl, and the combined acidic layers were basified with solid K₂CO₃ and extracted with DCM. The organic extracts were dried over MgSO₄ x H₂O and evaporated. The residue was stirred with TBME, and after filtration the title compound was obtained in the form of colourless crystals {HPLC: Rt_{H2} = 2.096 min; ESIMS: 314, 316 [(M+H)⁺, 1 Br]; ¹H-NMR (360 MHz, DMSO-d₆): 7.55 (dd, 1 H), 7.49 (ddd, 1H), 7.08 (dd, 1 H), 5.85 (br s, 2H), 4.11 (d, 1H), 3.90 (d, 1H), 2.88 (s, 3H), 1.59 (s, 3H)}.

### 1g) [6-(5-Bromo-2-fluoro-phenyl)-4,6-dimethyl-5-oxo-3,4,5,6-tetrahydro-pyrazin-2-yl]-carbamic acid tert-butyl ester

To a suspension of compound 1f) (995 mg, 3.17 mmol) in 12 ml of THF and 2 ml of DCM were added 0.83 ml (4.75 mmol) of DIPEA and 760 mg (3.48 mmol) of BoC₂O. The mixture was stirred overnight, diluted with TBME, washed with water and brine, dried over MgSO₄ x H₂O and evaporated. The residue was purified by chromatography on silica gel (hexane/25 to 50 % EtOAc) to yield the title compound in the form of a colourless foam {HPLC: Rt_{H2} = 3.027 min; ESIMS: 414, 416 [(M+H)⁺, 1Br]; ¹H-NMR (360 MHz, CDCl₃; very broad signals due to rotamers): 7.60 - 6.70 (m, 3H), 4.60 and 4.05 (two br s, 2H), 3.00 (br s, 3H), 1.84 and 1.70 (two s, 3H), 1.40 (s, 9H)}.

### 1h) [6-(5-Amino-2-fluoro-phenyl)-4,6-dimethyl-5-oxo-3,4,5,6-tetrahydro-pyrazin-2-yl]-carbamic acid tert-butyl ester

Compound 1g) (100 mg, 0.241 mmol) and rac-trans-N,N-dimethylcyclohexane-1,2-diamine (5.2 mg, 0.036 mmol) were dissolved in 7 ml of EtOH. The mixture was treated with an aq solution of 31.4 mg (0.483 mmol) of NaN₃ and 2.4 mg (2.4 mmol) of L-(+)-ascorbic acid sodium salt, degassed, brought under a nitrogen atmosphere, treated with 4.6 mg (0.024 mmol) of CuI, stirred for 2 h at 45°C, diluted with TBME, washed with water, dried over MgSO₄ x H₂O and evaporated. The residue was taken up in EtOH and stirred under a hydrogen atmosphere in the presence of 5 mg of Pd on carbon (10 %), until all of the azide had been hydrogenated. The mixture was filtered over celite, the filtrate was evaporated, and the residue was purified by chromatography on silica gel (hexane /15 to 40 % EtOAc) to yield the title compound in the form of a colourless foam {HPLC: Rt_{H2}= 2.145 min; ESIMS: 351 [(M+H)⁺]; ¹H-NMR (360 MHz, CDCl₃; very broad signals due to rotamers): 7.60 - 6.40 (br m), 4.80 - 3.40 (br), 3.02 (s, 3H), 1.79 (br s, 3H), 1.40 (s, 9H)}.

### 1i) (6-{5-[(5-Bromo-pyridine-2-carbonyl)-amino]-2-fluoro-phenyl}-4,6-dimethyl-5-oxo-3,4,5,6-tetrahydro-pyrazin-2-yl)-carbamic acid tert-butyl ester

To an ice-cold solution of compound 1h) (67 mg, 0.192 mmol), 43 mg (0.211 mmol) of 5-bromo-pyridine-2-carboxylic acid, 34 mg (0.25 mmol) of HOAt and 48 mg (0.25 mmol) of EDC x HCl in DCM were added 0.66 ml (0.48 mmol) of Et₃N. The mixture was stirred overnight, diluted with EtOAc, washed with 5 % aq NaHCO₃ solution and brine, dried over Na₂SO₄ and evaporated. The residue was purified by chromatography on silica gel (hexane /25 to 65 % EtOAc) to yield the title compound in the form of a colourless foam {HPLC: Rt_{H2} = 3.230 min; ESIMS: 534, 536 [(M+H)⁺, 1Br]; ¹H-NMR (360 MHz, CDCl₃; very broad signals due to rotamers): 9.78 (s, 1 H), 8.60 (s, 1H), 8.11 (d, 1H), 7.98 (d, 1 H), 7.80 - 7.60 (m, 2H), 7.10-6.90 (m, 1 H), 4.65 (br, 1 H), 4.08 (br, 1 H), 3.02 (br s, 3H), 1.90 and 1.83 (two br s, 3H), 1.40 (s, 9H)}.

### 1j) 5-Bromo-pyridine-2-carboxylic acid [3-(6-amino-2,4-dimethyl-3-oxo-2,3,4,5-tetrahydro-pyrazin-2-yl)-4-fluoro-phenyl]-amide

A mixture of compound 1i) (67 mg, 0.126 mmol) and 3 ml of 3N HCl in MeOH was stirred for 3 h at 45°C and then evaporated. The residue was basified with 10 % aq Na₂CO₃ solution, the mixture was extracted with DCM, and the organic phase was dried over Na₂SO₄ and evaporated. The residue was purified by chromatography on silica gel (DCM /5 to 10 % MeOH) to yield the title compound in the form of beige crystals {HPLC: Rt_{H2}= 2.665 min; ESIMS: 434, 436 [(M+H)⁺, 1 Br]; ¹H-NMR (600 MHz, DMSO-d₆): 10.72 (s, 1 H), 8.81 (s, 1 H), 8.33 (d, 1 H), 8.09 (d, 1 H), 7.98 (d, 1H), 7.85 - 7.80 (m, 1 H), 7.04 (t, 1 H), 5.74 (br s), 4.08 (br, 1H), 4.06 (d, 1 H), 3.91 (d, 1 H), 2.87 (s, 3H), 1.60 (s, 3H)}.

### Example 2: 5-Bromo-pyridine-2-carboxylic acid [3-(6-amino-2,4-dimethyl-2,3,4,5-tetrahydro-pyrazin-2-yl)-4-fluoro-phenyl]-amide

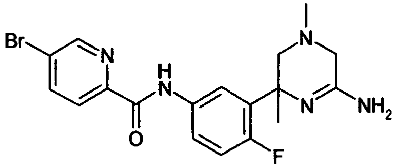

### 2a) [6-(5-Amino-2-fluoro-phenyl)-4,6-dimethyl-3,4,5,6-tetrahydro-pyrazin-2-yl]-carbamic acid tert-butyl ester

A stirred solution of compound 1h) (93 mg, 0.266 mmol) in 1.5 ml of THF was treated at 4°C with 0.6 ml of a 2M solution of LiAlH₄ in THF. The mixture was stirred for 30 min, treated with 0.32 ml (0.4 mmol) of CHCl₃, stirred for 1 h, quenched by adding 0.045 ml of water, followed by 0.045 ml of 4N aq NaOH solution and by 0.115 ml of water, dried over Na₂SO₄ and evaporated. The residue was purified by chromatography on silica gel [hexane /25 to 65 % EtOAc (containing 5 % MeOH)] to yield the title compound in the form of a colourless resin {HPLC: Rt_{H2} = 2.365 min; ESIMS: 337 [(M+H)⁺]; ¹H-NMR (360 MHz, CDCl₃; very broad signals due to rotamers): 6.77 (dd, 1 H), 6.53 - 6.42 (m, 2H), 3.65 - 3.60 (m, 2H), 3.05 (d, 1 H), 2.54 (d, 1H), 2.21 (s, 3H), 1.60 (s, 3H), 1.43 (s, 9H)}.

### 2b) (6-{5-[(5-Bromo-pyridine-2-carbonyl)-amino]-2-fluoro-phenyl}-4,6-dimethyl-3,4,5,6-tetrahydro-pyrazin-2-yl)-carbamic acid tert-butyl ester

The title compound was prepared by a procedure analogous to that used in Example 1i) {HPLC: Rt_{H2} = 3.418 min; ESIMS: 520, 522 [(M+H)⁺, 1 Br]; ¹H-NMR (360 MHz, CDCl₃; data of major rotamer): 9.75 (br s, 1 H), 8.58 (d, 1 H), 8.08 (d, 1H), 7.97 (dd, 1H), 7.80 - 7.60 (m, 2H), 7.79 - 7.72 (m, 1 H), 7.37 (dd, 1H), 7.03 (dd, 1H), 3.29 (d, 1 H), 3.20 - 3.00 (br, 2H), 2.56 (d, 1 H), 2.20 (s, 3H), 1.63 (s, 3H), 1.48 (s, 9H)}.

### 2c) 5-Bromo-pyridine-2-carboxylic acid [3-(6-amino-2,4-dimethyl-2,3,4,5-tetrahydro-pyrazin-2-yl)-4-fluoro-phenyl]-amide

The title compound was prepared by a procedure analogous to that used in Example 1j) {HPLC: Rt_{H2} = 2.801 min; ESIMS: 420, 422 [(M+H)⁺, 1Br]; ¹H-NMR (600 MHz, DMSO-d₆): 10.52 (s, 1 H), 8.89 (s, 1 H), 8.32 (dd, 1H), 8.07 (dd, 1H), 7.85 (m, 1 H), 7.72 (m, 1 H), 7.09 (dd, 1 H), 5.70 - 5.52 (br, 1 H), 2.77 (d, 1 H), 2.68 (d, 1 H), 2.55 - 2.45 (m, 2H), 2.10 (s, 3H), 1.43 (s, 3H)}.

### Example 3: 5-Bromo-pyridine-2-carboxylic acid [3-(6-amino-2,4-dimethyl-3-oxo-2,3,4,5-tetrahydro-pyrazin-2-yl)-phenyl]-amide

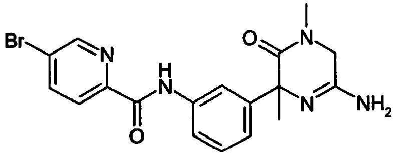

The title compound was prepared by procedures analogous to those used in Example 1 {HPLC: Rt_{H2}= 2.661 min; ESIMS: 416, 418 [(M+H)⁺, 1Br]; ¹H-NMR (600 MHz, DMSO-d₆): 10.55 (s, 1H), 8.86 (s, 1 H), 8.33 (dd, 1H), 8.08 (dd, 1H), 7.83 (s, 1H), 7.74 (d, 1 H), 7.28 (t, 1 H), 7.10 (d, 1H), 6.15 - 6.00 (br, 2H), 3.82 (d, 1H), 3.78 (d, 1H), 2.80 (s, 3H), 1.55 (s, 3H)}.

### Example 4: 5-Bromo-pyridine-2-carboxylic acid [3-(6-amino-2,4-dimethyl-5-oxo-2,3,4,5-tetrahydro-pyrazin-2-yl)-4-fluoro-phenyl]-amide hydrochloride

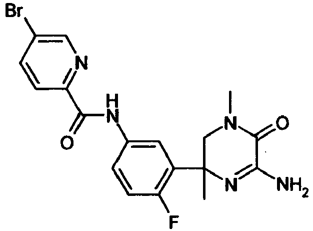

### 4a) 2-Amino-2-(5-bromo-2-fluoro-phenyl)-propan-1-ol

A stirred suspension of compound 1a) (10.0 g, 38.0 mmol) in 110 ml of THF was treated dropwise with borane dimethyl sulfide (12.08 ml, 114 mmol) and then heated to reflux. The mixture was stirred for 5 h, cooled down, carefully quenched by adding dropwise 25 ml of MeOH, followed by 12 ml of 4N HCl and by 100 ml of MeOH, concentrated in vacuo, diluted with 200 ml of MeOH, concentrated, diluted with water, basified with 10 % aq Na₂CO₃ solution and extracted three times with DCM. The organic phases were dried over K₂CO₃ and evaporated to yield the title compound in the form of a colourless solid {HPLC: Rt_{H1} = 2.540 min; ESIMS: 248, 250 [(M+H)⁺, 1Br]; ¹H-NMR (360 MHz, DMSO-d₆): 7.84 (dd, 1H), 7.49 (ddd, 1 H), 7.34 (dd, 1 H), 4.84 (br t, 1 H), 3.64 (br dd, 1 H), 3.50 (br dd, 1 H), 2.15 (br s, 2H), 1.35 (s, 3H)}.

### 4b) [1-(5-Bromo-2-fluoro-phenyl)-2-hydroxy-1-methyl-ethyl]-carbamic acid tert-butyl ester

Compound 4a) (17.98 g, 72.5 mmol) and 23 g (109 mmol) of Boc₂O were dissolved in 43 ml of dioxane. The mixture was treated with 43 ml of saturated aq NaHCO₃ solution, stirred overnight, diluted with water and extracted with TBME. The organic phase was washed with brine, dried over MgSO₄ x H₂O evaporated and diluted with hexane to yield the title compound in the form of colourless crystals {HPLC: Rt_{H3} = 2.906 min; ESIMS: 370, 372 [(M+Na)⁺, 1Br]; ¹H-NMR (360 MHz, CDCl₃): 7.47 (dd, 1 H), 7.40 (ddd, 1 H), 6.93 (dd, 1 H), 5.24 (br s, 1 H), 4.15 (br d, 1 H), 3.88 (d, 1 H), 1.59 (s, 3H), 1.48 (br s, 9H)}.

### 4c) 4-(5-Bromo-2-fluoro-phenyl)-4-methyl-2,2-dioxo-2lambda*6*-[1,2,3]oxathiazolidine-3-carboxylic acid tert-butyl ester

A solution of compound 4b) (22.46 g, 64.5 mmol) in 645 ml of DCM was added dropwise at 0°C to a solution of 9.42 ml (129 mmol) of thionyl chloride in 26.1 ml (330 mmol) of pyridine. The mixture was slowly warmed to 25°C, stirred for 16 h, treated with 1 N HCl and extracted with TBME. The organic phase was treated with charcoal, filtered over celite, evaporated, taken up in 130 ml of ACN, treated at 0 to 5°C with 7.3 mg (0.032 mmol) of Ru(III)Cl₃ hydrate, then with 13.8 g of NaIO₄ and with 130 ml of water, stirred for 1 h at 25°C, diluted with water and extracted with DCM. The extract was washed with brine, dried over MgSO₄ x H₂O treated with charcoal, filtered over celite, evaporated and diluted with hexane to yield the title compound in the form of a colourless crystalline solid {HPLC: Rt_{H4} = 3.019 min; ESIMS: 841, 843, 845 [(2M+Na)⁺, 1Br]; ¹H-NMR (360 MHz, CDCl₃): 7.52 (ddd, 1 H), 7.43 (dd, 1 H), 7.05 (dd, 1H), 4.72 (d, 1H), 4.48 (d, 1 H), 2.05 (s, 3H), 1.55 (s, 9H)}.

### 4d) [1-(5-Bromo-2-fluoro-phenyl)-1-methyl-2-methylamino-ethyl]-carbamic acid tert-butyl ester

A mixture of compound 4c) (2.0 g, 4.88 mmol) and 12.14 ml (98 mmol) of MeNH₂ (33 % in EtOH) was stirred for 18 h at 25°C, treated with 10 ml of 2N HCl, stirred for 1 h, neutralized with 10 % aq NaHCO₃ solution and extracted with DCM. The organic phase was dried over K₂CO₃ and purified by chromatography on silica gel (DCM /1 to 2 % MeOH) to yield the title compound in the form of a colourless resin {HPLC: Rt_{H2} = 2.918 min; ESIMS: 361, 363 [(M+H)⁺, 1Br]; ¹H-NMR (360 MHz, CDCl₃): 7.80 - 7.67 (m, 1H), 7.41 (ddd, 1 H), 6.96 (dd, 1 H), 3.85 - 3.55 (m, 2H), 2.75 and 2.69 (two s, 3H; two rotamers), 2.05 (s, 3H), 1.68 and 1.45 (two s, 9H; two rotamers)}.

### 4e) N-[2-(5-Bromo-2-fluoro-phenyl)-2-tert-butoxycarbonylamino-propyl]-N-methyl-oxalamic acid methyl ester

A mixture of compound 4d) (1.06 g, 2.93 mmol), 0.67 ml (3.81 mmol) of DIPEA and DCM was treated at -78°C dropwise with 0.3 ml (3.32 mmol) of monomethyl oxalylchloride, allowed to warm to 25°C, diluted with TBME, washed with 1N HCl and brine, dried over MgSO₄ x H₂O and purified by chromatography on silica gel (hexane /EtOAc 3:1) to yield the title compound in the form of a colourless solid {HPLC: Rt_{H3} = 3.445 min; ESIMS: 469, 471 [(M+Na)⁺, 1Br]; ¹H-NMR (360 MHz, CDCl₃; major rotamer): 9.90 (br s, 1H), 7.45 - 7.36 (m, 2H), 6.95 (dd, 1H), 3.92 (s, 3H), 3.65 (br s, 2H), 2.65 (s, 3H), 1.93 (s, 3H), 1.57 (s, 9H)}.

### 4f) 5-(5-Bromo-2-fluoro-phenyl)-1,5-dimethyl-piperazine-2,3-dione

A mixture of compound 4e) (1.16 g) and 13 ml of 4N HCl in dioxane was slightly warmed to 25°C, stirred for 1 h and evaporated. The residue was taken up in saturated aq NaHCO₃ solution and extracted with DCM. The organic phase was dried over MgSO₄ x H₂O, evaporated and diluted with TBME /hexane to yield the title compound in the form of colourless crystals {HPLC: Rt_{H2} = 2.566 min; ESIMS: 315, 317 [(M+H)⁺, 1Br]; ¹H-NMR (360 MHz, DMSO-d₆): 9.38 (br s, 1 H), 7.63 (ddd, 1 H), 6.30 (dd, 1 H), 4.00 (d, 1H), 3.88 (d, 1 H), 2.81 (s, 3H), 1.55 (s, 3H)}.

### 4g) 5-(5-Bromo-2-fluoro-phenyl)-1,5-dimethyl-3-thioxo-piperazin-2-one

To a solution of compound 4f) (674 mg, 2.139 mmol) in pyridine were added 475 mg (2.139 mmol) of phosphorous pentasulfide. The mixture was stirred for 3 h at 80°C, cooled down, diluted with EtOAc, washed with 1N HCl, 5 % aq NaHCO₃ solution and brine, dried over MgSO₄ x H₂O and purified by chromatography on silica gel (hexane /35 to 50 % EtOAc) to yield the title compound in the form of a yellow foam {HPLC: Rt_{H2}= 2.783 min; ESIMS: 331, 333 [(M+H)⁺, 1 Br]; ¹H-NMR (360 MHz, DMSO-d₆): 11.66 (s, 1 H), 7.64 (ddd, 1 H), 7.28 - 6.36 (m, 2H), 4.02 (d, 1H), 3.94 (d, 1 H), 2.85 (s, 3H), 1.61 (s, 3H)}.

### 4h) 3-Amino-5-(5-bromo-2-fluoro-phenyl)-1,5-dimethyl-5,6-dihydro-1H-pyrazin-2-one

A mixture of compound 4g) (545 g, 1.646 mmol) in 7 ml of a 7M methanolic solution of NH₃ was stirred for 18 h at 25°C, evaporated and purified by chromatography on silica gel (DCM / 0.5 to 5 % EtOH) to yield the title compound in the form of a colourless solid {HPLC: Rt_{H2} = 2.402 min; ESIMS: 314, 316 [(M+H)⁺, 1Br]; ¹H-NMR (360 MHz, DMSO-d₆): 7.84 (dd, 1 H), 7.53 (ddd, 1 H), 7.21 (dd, 1 H), 6.49 (br s, 2H), 3.78 (d, 1H), 3.68 (d, 1H), 2.92 (s, 3H), 1.45 (s, 3H)}.

### 4i) 5-Bromo-pyridine-2-carboxylic acid [3-(6-amino-2,4-dimethyl-5-oxo-2,3,4,5-tetra-hydro-pyrazin-2-yl)-4-fluoro-phenyl]-amide hydrochloride

The title compound was prepared by procedures analogous to those used in example 1, starting from compound 4h) {HPLC: Rt_{H2} = 2.787 min; ESIMS: 434, 436 [(M+H)⁺, 1Br]; ¹H-NMR (600 MHz, DMSO-d₆): 11.22 (s, 1 H), 10.97 (s, 1 H), 9.78 (br s, 1H), 9.48 (br s, 1 H), 8.87 (s, 1 H), 8.34 (dd, 1 H), 8.08 (d, 1 H), 7.99 - 7.93 (m, 2H), 7.32 (dd, 1 H), 4.10 (d, 1 H), 3.98 (d, 1H), 2.97 (s, 3H), 1.68 (s, 3H)}.

### Example 5: 5-Chloro-pyridine-2-carboxylic acid [3-(6-amino-2,4-dimethyl-5-oxo-2,3,4,5-tetrahydro-pyrazin-2-yl)-4-fluoro-phenyl]-amide hydrochloride

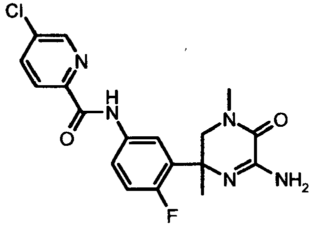

The title compound was prepared by procedures analogous to those used in the examples hereinbefore {HPLC: Rt_{H2} = 2.723 min; ESIMS: 390 [(M+H)⁺]; ¹H-NMR (600 MHz, DMSO-d₆): 11.28 (s, 1 H), 10.97 (s, 1 H), 9.79 (br s, 1 H), 9.48 (br, 1 H), 8.79 (d, 1 H), 8.21 (dd, 1 H), 8.15 (d, 1 H), 7.99 - 7.93 (m, 2H), 7.32 (dd, 1 H), 4.11 (d, 1H), 3.98 (d, 1 H), 2.95 (s, 3H), 1.68 (s, 3H)}.

### Example 6: 5-Bromo-pyridine-2-carboxylic acid [3-(6-amino-4-ethyl-2-methyl-5-oxo-2,3,4,5-tetrahydro-pyrazin-2-yl)-4-fluoro-phenyl]-amide hydrochloride

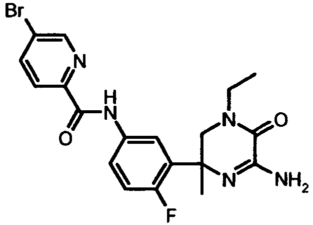

The title compound was prepared by procedures analogous to those used in the examples hereinbefore {HPLC: Rt_{H2} = 2.913 min; ESIMS: 448, 450 [(M+H)⁺, 1Br]; ¹H-NMR (600 MHz, DMSO-d₆): 11.23 (br s, 1H), 10.98 (s, 1H), 9.81 (br s, 1 H), 9.58 (br s, 1H), 8.87 (d, 1 H), 8.34 (dd, 1 H), 8.07 (d, 1 H), 8.02 - 7.98 (m, 1 H), 7.92 (dd, 1 H), 7.33 (dd, 1 H), 4.11 (d, 1 H), 3.97 (d, 1H), 3.48 - 3.44 (m, 1 H), 3.30 - 3.26 (m, 1 H), 1.70 (s, 3H), 0.80 (t, 3H)}.

### Example 7: 3,5-Dichloro-pyridine-2-carboxylic acid [3-(6-amino-2,4-dimethyl-5-oxo-2,3,4,5-tetrahydro-pyrazin-2-yl)-4-fluoro-phenyl]-amide hydrochloride

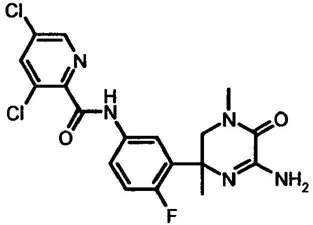

The title compound was prepared by procedures analogous to those used in the examples hereinbefore {HPLC: Rt_{H2} = 2.732 min; ESIMS: 424 [(M+H)⁺]; ¹H-NMR (600 MHz, DMSO-d₆): 11.27 (br s, 1H), 10.97 (s, 1 H), 9.80 (br s, 1 H), 9.52 (br s, 1 H), 8.74 (d, 1H), 8.48 (d, 1 H), 7.79 (dd, 1H), 7.73 (dd, 1 H), 7.35 (dd, 1 H), 4.11 (d, 1H), 3.98 (d, 1H), 2.95 (s, 3H), 1.69 (s, 3H)}.

### Example 8: 3,5-dichloro-pyridine-2-carboxylic acid [3-(6-amino-4-ethyl-2-methyl-5-oxo-2,3,4,5-tetrahydro-pyrazin-2-yl)-4-fluoro-phenyl]-amide hydrochloride

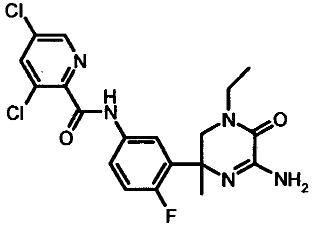

The title compound was prepared by procedures analogous to those used in the examples hereinbefore {HPLC: Rt_{H2} = 2.849 min; ESIMS: 438 [(M+H)⁺]; ¹H-NMR (600 MHz, DMSO-d₆): 11.27 (br s, 1 H), 10.97 (s, 1 H), 9.85 (br s, 1 H), 9.63 (br s, 1 H), 8.74 (d, 1 H), 8.47 (d, 1 H), 7.85 - 7.81 (m, 1 H), 7.67 (dd, 1 H), 7.34 (dd, 1H), 4.10 (d, 1 H), 3.93 (d, 1 H), 3.48 - 3.39 (m, 1 H), 3.31 - 3.22 (m, 1H), 1.71 (s, 3H), 0.80 (t, 3H)}.

### Example 9: 5-Bromo-pyridine-2-carboxylic acid [3-(6-amino-4-isopropyl-2-methyl-5-oxo-2,3,4,5-tetrahydro-pyrazin-2-yl)-4-fluoro-phenyl]-amide hydrochloride

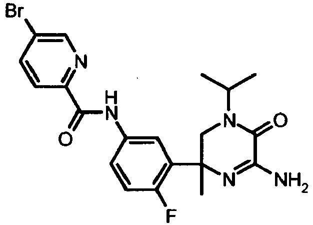

The title compound was prepared by procedures analogous to those used in the examples hereinbefore {HPLC: Rt_{H2} = 3.006 min; ESIMS: 462, 464 [(M+H)⁺, 1Br]; ¹H-NMR (600 MHz, DMSO-d₆): 11.19 (br s, 1H), 10.98 (s, 1 H), 9.84 (br s, 1 H), 9.62 (br s, 1 H), 8.87 (d, 1H), 8.33 (dd, 1 H), 8.03 - 7.98 (m, 1 H), 7.90 (dd, 1 H), 7.32 (dd, 1 H), 4.41 (heptett, 1 H), 3.88 (s, 2H), 1.75 (s, 3H), 1.07 (d, 3H), 0.66 (d, 3H)}.

### Example 10: 3,5-Dichloro-pyridine-2-carboxylic acid {3-[6-amino-4-(2-methoxy-ethyl)-2-methyl-5-oxo-2,3,4,5-tetrahydro-pyrazin-2-yl]-4-fluoro-phenyl}-amide hydrochloride

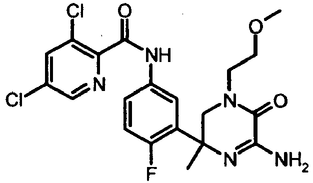

The title compound was prepared by procedures analogous to those used in the examples hereinbefore {HPLC: Rt_{H2} = 2.783 min; ESIMS: 468 [(M+H)⁺]; ¹H-NMR (600 MHz, DMSO-d₆): 11.22 (br s, 1 H), 10.93 (s, 1 H), 9.87 (br s, 1H), 9.60 (br s, 1H), 8.74 (d, 1H), 8.47 (d, 1 H), 7.86 - 7.82 (m, 1H), 7.65 (dd, 1 H), 7.34 (dd, 1 H), 4.11 (d, 1H), 4.04 (d, 1 H), 3.63 - 3.58 (m, 1H), 3.42 - 3.37 (m, 1 H), 3.30 - 3.25 (m, 1 H), 3.20 - 3.15 (m, 1 H), 3.00 (s, 3H), 1.69 (s, 3H)}.

### Example 11: 5-Bromo-pyridine-2-carboxylic acid {3-[6-amino-4-(2-methoxy-ethyl)-2-methyl-5-oxo-2,3,4,5-tetrahydro-pyrazin-2-yl]-4-fluoro-phenyl}-amide hydrochloride

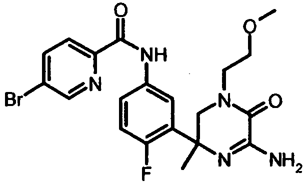

The title compound was prepared by procedures analogous to those used in the examples hereinbefore {HPLC: Rt_{H2} = 2.802 min; ESIMS: 478, 480 [(M+H)⁺, 1 Br]; ¹H-NMR (600 MHz, DMSO-d₆): 11.20 (br s, 1 H), 10.96 (s, 1 H), 9.81 (br s, 1 H), 9.57 (br s, 1 H), 8.87 (d, 1 H), 8.33 (dd, 1 H), 8.07 (d, 1H), 8.03 - 7.98 (m, 1 H), 7.91 (dd, 1 H), 7.32 (dd, 1 H), 4.09 (d, 1 H), 4.04 (d, 1 H), 3.63 - 3.58 (m, 1 H), 3.45 - 3.40 (m, 1 H), 3.30 - 3.25 (m, 1 H), 3.20 - 3.15 (m, 1H), 3.00 (s, 3H), 1.70 (s, 3H)}.

### Example 12: 5-Bromo-pyridine-2-carboxylic acid {3-[6-amino-2-methyl-4-(1-methyl-1H-pyrazol-4-yl)-5-oxo-2,3,4,5-tetrahydro-pyrazin-2-yl]-4-fluoro-phenyl}-amide hydrochloride

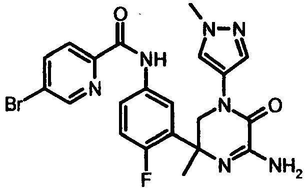

The title compound was prepared by procedures analogous to those used in the examples hereinbefore {HPLC: Rt_{H2} = 2.777 min; ESIMS: 500, 502 [(M+H)⁺, 1Br]; ¹H-NMR (600 MHz, DMSO-d₆): 11.40 (br s, 1H), 10.95 (s, 1 H), 9.98 (br d, 1 H), 9.68 (br d, 1 H), 8.86 (d, 1 H), 8.34 (dd, 1 H), 8.08 (s, 1 H), 8.06 (d, 1 H), 7.98 - 7.92 (m, 2H), 7.67 (s, 1 H), 7.29 (dd, 1 H), 4.47 (s, 2H), 3.79 (s, 3H), 1.78 (s, 3H)}.

### Example 13: 5-Bromo-pyridine-2-carboxylic acid [3-(6-amino-2-methyl-5-oxo-4-pyridin-3-yl-2,3,4,5-tetrahydro-pyrazin-2-yl)-4-fluoro-phenyl]-amide hydrochloride

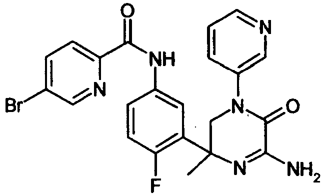

The title compound was prepared by procedures analogous to those used in the examples hereinbefore {HPLC: Rt_{H2} = 2.667 min; ESIMS: 497, 499 [(M+H)⁺, 1Br]; ¹H-NMR (600 MHz, DMSO-d₆): 11.56 (br s, 1 H), 10.99 (s, 1H), 10.08 - 10.04 (m, 1 H), 9.82 - 9.78 (m, 1 H), 8.87 (d, 1 H), 8.54 (d, 1 H), 8.38 (s, 1 H), 8.34 (dd, 1 H), 8.07 (s, 1 H), 8.05 - 7.98 (m, 2H), 7.63 (d, 1 H), 7.54 (dd, 1 H), 7.34 (dd, 1H), 4.60 (d, 1 H), 4.31 (d, 1 H), 1.80 (s, 3H)}.

### Example 14: 5-Cyano-pyridine-2-carboxylic acid [3-(6-amino-4-ethyl-2-methyl-5-oxo-2,3,4,5-tetrahydro-pyrazin-2-yl)-4-fluoro-phenyl]-amide hydrochloride

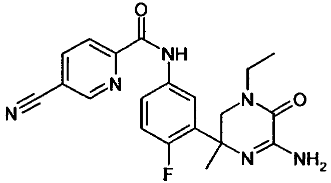

The title compound was prepared by procedures analogous to those used in the examples hereinbefore {HPLC: Rt_{H2} = 2.635 min; ESIMS: 395 [(M+H)⁺]; ¹H-NMR (600 MHz, DMSO-d₆): 11.22 (br s, 1 H), 11.14 (s, 1 H), 9.81 (br s, 1 H), 9.51 (br s, 1 H), 9.19 (s, 1H), 8.59 (dd, 1 H), 8.27 (d, 1H), 8.03 - 7.99 (m, 1 H), 7.95 - 7.91 (m, 1 H), 7.34 (dd, 1 H), 4.10 (d, 1 H), 3.95 (d, 1 H), 3.47 - 3.38 (m, 1 H), 3.30 - 3.21 (m, 1 H), 1.70 (s, 3H), 0.79 (t, 3H)}.

### Example 15: 5-Bromo-pyridine-2-carboxylic acid [3-(6-amino-5-ethyl-2,4-dimethyl-3-oxo-2,3,4,5-tetrahydro-pyrazin-2-yl)-phenyl]-amide (9 : 1 mixture of two diastereomers)

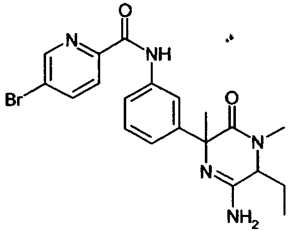

The title compound was prepared by procedures analogous to those used in the examples hereinbefore {HPLC: Rt_{H2} = 2.866 min (major diastereomer); ESIMS: 444, 446 [(M+H)⁺, 1 Br]; ¹H-NMR (600 MHz, DMSO-d₆; major diastereomer): 10.53 (s, 1 H), 8.85 (s, 1 H), 8.32 (dd, 1H), 8.07 (dd, 1H), 7.97 (s, 1 H), 7.68 (d, 1 H), 7.26 - 7.20 (m, 2H), 5.98 (s, 2H), 3.79 (t, 1 H), 2.87 (s, 3H), 1.42 - 1.29 (m, 2H), 0.67 (t, 3H)}.

### Example 16: 5-Bromo-pyridine-2-carboxylic acid [3-(6-amino-2-difluoromethyl-2,3,4,5-tetrahydro-pyrazin-2-yl)-4-fluoro-phenyl]-amide

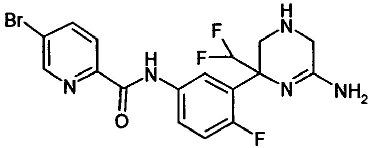

### 16a) 1-(5-Bromo-2-fluoro-phenyl)-ethanone

A solution of 711 ml (5.03 mol) diisopropyl amine in 8 L THF was cooled to -80 °C. A 2.5 M solution of BuLi in hexanes (2.01 L, 5.03 mol) was added over a period of 15 minutes. After 30 minutes a solution of 500 ml of 4-bromo-1-fluoro benzene (4.574 mol) was added while keeping the temperature below -65 °C. After stirring for 2.5 h at -65 °C the mixture was cooled to -80 °C and 681 g ethyl difluoro acetate (5.488 mol) were added, while keeping the temperature below -65 °C. The mixture was warmed to -40 °C and then quenched by pouring the mixture onto 15 L ice-cold 1 M HCl and 15 L TBME. The phases were separated and the organic phase was washed with 10% aq NaHCO₃ and brine. The extract was dried with sodium sulfate, filtered, concentrated and purified by distillation at 0.1 mbar. The fraction boiling at 67-72 °C was collected. Yield 856 g (74%) of a pale yellow liquid. ¹H-NMR (CDCl₃, 360 MHz): 8.09 (dd, 1H), 7.82-7.77 (m, 1 H), 7.17 (t, 1 H), 6.45 (t, 1H, CHF₂).

### 16b) [1-(5-Bromo-2-fluoro-phenyl)-2,2-difluoro-eth-(Z)-ylidene]-carbamic acid tert-butyl ester

A mixture of 675 g (2.668 mol) 1-(5-bromo-2-fluoro-phenyl)-ethanone (compound 16a) and 1007 g (2.668 mol) N-tert-butyloxycarbonyl-triphenyliminophosphorane were suspended in 505 ml toluene and heated at 105 °C for 4 h. After cooling down to 80 °C 3 L heptane were added and the mixture was stirred at 25 °C overnight. Crystallized triphenylphosphine oxide was removed by filtration and the filtrate was purified via chromatography on silica gel (heptane/5% EtOAc)
¹H-NMR (CDCl₃, 360 MHz): 7.90-7.84 (m, 1H), 7.75-7.67 (m, 1H), 7.47 (t, 1 H), 6.88 (t, 1H, CHF₂), 1.30 (br s, 9H).

### 16c) [1-(5-Bromo-2-fluoro-phenyl)-2,2-difluoro-1-nitromethyl-ethyl]-carbamic acid tert-butyl ester

At 25 °C a solution of 7.5 g (21.3 mmol) compound 16b in 30 ml of nitromethane was treated with 0.2 ml DBU (1.3 mmol). After 2 h the mixture was diluted with 50 ml TBME, washed with 1 N HCl and water. The organic phase was evaporated and the residue was crystallized from TBME/hexane to give the title compound as white crystals. HPLC: Rt_{H3} = 3.418 min; ESIMS: 435, 437 [(M+Na)⁺, 1Br]; ¹H-NMR (CDCl₃, 400 MHz): 7.56-7.48 (m, 2H), 7.01 (dd, 1 H), 6.56 (t, 1 H, CHF₂), 5.53 (br d, 1H), 5.38 (br d, 1H), 1.45 (br s, 9H).

### 16d) [1-Aminomethyl-1-(5-bromo-2-fluoro-phenyl)-2,2-difluoro-ethyl]-carbamic acid tert-butyl ester

Zinc dust (2.74 g, 41.8 mmol) was suspended in 20 ml acetic acid and a solution of 2.47 g (5.98 mmol) compound 16c in 20 ml acetic acid was added dropwise keeping the temperature below 40 °C. After 2h stirring at rt the mixture was filtered over celite. The filter cake was washed with MeOH, the filtrated was basified with 10% aq Na₂CO₃ and extracted with EtOAc. The organic phase was washed with brine, dried with sodium sulfate and evaporated. The product was obtained as white crystals (from hexane).
HPLC: Rt_{H5} = 2.177 min; ESIMS: 383, 385 [(M+H)⁺, 1 Br];
¹H-NMR (CDCl₃, 360 MHz): 7.55 (dd, 1 H), 7.46 (ddd, 1 H), 7.00 (dd, 1H), 6.42 (t, 1H, CHF₂), 5.78 (br s, 2H), 3.49 (br d, 1 H), 3.87 (br d, 1 H), 1.65-1.25 (br, 9H).

### 16e) [2-(5-Bromo-2-fluoro-phenyl)-2-tert-butoxycarbonylamino-3,3-difluoro-propylamino]-acetic acid tert-butyl ester

A mixture of 887 mg (2.315 mmol) compound 16d, 451 mg (2.315 mmol) tert-butyl bromoacetate and 1.21 ml (6.94 mmol) DIPEA in 8 ml ACN was stirred at 80 °C for 1.5 h. The mixture was diluted with EtOAc, washed with 1 N HCl, 5% aq NaHCO₃ and water. The organic phase was dried with sodium sulfate and the product was purified via chromatography on silica gel (heptane/ 15% EtOAc) to give the title compound as a colorless resin. TLC: Rf 0.21 (EtOAc/ heptane 1:6; HPLC: Rt_{H3} = 2.785 min; ESIMS: 497, 499 [(M+H)⁺, 1Br]; ¹H-NMR (CDCl₃, 360 MHz): 7.51 (dd, 1H), 7.33 (ddd, 1H), 6.87 (dd, 1H), 6.44 (t, 1H, CHF₂), 6.04 (br s, 1H), 3.28-3.02 (m, 4H), 1.39 (s, 9H), 1.32 (br s, 9H).

### 16f) 6-(5-Bromo-2-fluoro-phenyl)-6-difluoromethyl-piperazin-2-one

A solution of 1.0 g (2.011 mmol) compound 16e in 8 ml DCM was treated with 5 ml 4N HCl in dioxane. After 4 h the mixture was evaporated, dissolved in 10 ml MeOH and left standing overnight. The MeOH was partially removed and crystallization initiated by careful addition of TBME. The hydrochloride salt of the title compound was isolated as white crystals. TLC (free base): Rf 0.39 (EtOAc); HPLC: Rt_{H1} = 2.543 min; ESIMS: 323, 325 [(M+H)⁺, 1 Br]; ¹H-NMR (HCl salt, dmso-d6, 360 MHz): 10.2-9.7 (br, 2H), 7.78-7.73 (m, 2H), 7.35 (dd, 1 H), 6.65 (t, 1H, CHF₂), 3.90-3.68 (m, 4H).

### 16g) 3-(5-Bromo-2-fluoro-phenyl)-3-difluoromethyl-5-oxo-piperazine-1-carboxylic acid tert-butyl ester

A suspension of 300 mg (0.834 mmol) compound 16f and 273 mg (1.25 mmol) Boc2O in 4 ml ACN was treated with 0.4 ml (2.25 mmol) DIPEA. The mixture was stirred overnight, diluted with EtOAc, washed with 1 N HCl, brine and 10% aq NaHCO₃, and dried with MgSO₄.H₂O. The crude product was purified via chromatography on silica gel (heptane/ 0-50% EtOAc) to give the title compound as a white solid. HPLC: Rt_{H3} = 2.776 min; ESIMS: 445, 447 [(M+Na)⁺, 1Br]; ¹H-NMR (CDCl₃, 360 MHz, broad signals due to rotamers): 7.60-7.52 (m, 2H), 7.09 (dd, 1 H), 6.6-6.1 (m, 3H), 4.6-3.63 (m, 4H), 1.35 and 1.29 (br s, 9H).

### 16h) 3-(5-Bromo-2-fluoro-phenyl)-3-difluoromethyl-5-thioxo-piperazine-1-carboxylic acid tert-butyl ester

A mixture of 329 mg (0.777 mmol) compound 16g and 283 mg (0.7 mmol) Lawesson's reagent in 4 ml THF was stirred overnight. The mixture was concentrated and purified via chromatography on silica gel (heptane/ 0-15% EtOAc) to give the title compound as a white solid. HPLC: Rt_{H3}= 3.317 min; ESIMS: 461, 463 [(M+Na)⁺, 1Br]; ¹H-NMR (CDCl₃, 360 MHz, broad signals due to rotamers): 8.45-8.32 (br, 1 H), 7.62-7.54 (br, 1 H), 7.44 (dd, 1 H), 7.11 (dd, 1 H), 6.6-6.2 (br, 1H), 5.1-4.3 (m, 3H), 3.75-3.65 (m, 1 H), 1.35 and 1.29 (br s, 9H).

### 16i) 5-Amino-3-(5-bromo-2-fluoro-phenyl)-3-difluoromethyl-3,6-dihydro-2H-pyrazine-1-carboxylic acid tert-butyl ester

A solution of 310 mg (0.706 mmol) of compound 16h in 4 ml 7M NH3/MeOH was stirred at rt for 15h. The mixture was evaporated, dissolved in EtOAc, washed with aq NaHCO₃ and brine. The org phase was dried with Na₂SO₄ and evaporated to give the title compound as a white solid, pure enough for further synthesis. HPLC: Rt_{H5}= 2.270 min; ESIMS [M+H]⁺ =422/424(1Br); ¹H-NMR (CDCl₃, 360 MHz, broadened signals, rotamers): 7.7-7.58 (m, 1 H), 7.38-7.30 (m, 1H), 6.88 (dd, 1 H), 6.03 (br t, 1 H, CHF2, major rotamer), 4.7 (br, 2H), 4.10-3.56 (m, 4H), 1.26 (br s, 9H, major rotamer). TLC (Hexane, EtOAc 1:1) Rf 0.42

### 16j) 3-(5-Bromo-2-fluoro-phenyl)-5-tert-butoxycarbonylamino-3-difluoromethyl-3,6-dihydro-2H-pyrazine-1-carboxylic acid tert-butyl ester

To an ice-cold solution of 290 mg (0.687 mmol) compound 16i in 4 ml ACN were added 225 mg (1.02 mmol) Boc₂O and 0.205 ml (1.17 mmol) DIPEA. The mixture was stirred for 4h at rt. Then the mixture was diluted with TBME and washed with 5% aq NaHCO₃. The organic phase was dried with MgSO₄.H₂O, filtered and concentrated. Purification by chromatography on silica gel (hexane/ 0-25% EtOAc) gave the desired product as a colorless foam.
TLC: Rf (Hexane / EtOAc 6:1) = 0.27. HPLC: Rt_{H6}= 2.724 min; ESIMS [M+H]⁺ =522/524(1Br); ¹H-NMR (CDCl₃, 360 MHz): Spectrum uninterpretable due to complex rotamer mixture.

### 16k) 3-(5-Amino-2-fluoro-phenyl)-5-tert-butoxycarbonylamino-3-difluoromethyl-3,6-dihydro-2H-pyrazine-1-carboxylic acid tert-butyl ester

Compound 16j (350 mg, 0.671 mmol) and rac-trans-N,N-dimethylcyclohexane-1,2-diamine (28.6 mg, 0.201 mmol) were dissolved in 2.5 ml of EtOH. The mixture was treated with an aq solution of 174 mg (2.68 mmol) of NaN₃ and 26.5 mg (0.134 mmol) of L-(+)-ascorbic acid sodium salt, degassed, brought under a nitrogen atmosphere, treated with 25.5 mg (0.134 mmol) of CuI, stirred for 30 min at 70 °C, diluted with TBME, washed with water, dried over MgSO₄.H₂O and evaporated. The residue was taken up in EtOH and stirred under a hydrogen atmosphere in the presence of 5 mg of Pd on carbon (10 %), until all of the azide had been hydrogenated. The mixture was filtered over celite, the filtrate was evaporated, and the residue was purified by chromatography on silica gel (hexane / 15 to 70 % EtOAc) to yield the title compound in the form of a colourless foam. HPLC: Rt_{H3} = 2.411 min; ESIMS: 459 [(M+H)⁺]; ¹H-NMR (360 MHz, CDCl₃; very broad signals due to rotamers): 7.30 - 5.90 (br m), 4.80 - 3.40 (br), 1.50-1.10 (br m).

### 161) 3-{5-[(5-Bromo-pyridine-2-carbonyl)-amino]-2-fluoro-phenyl}-5-tert-butoxycarbonylamino-3-difluoromethyl-3,6-dihydro-2H-pyrazine-1-carboxylic acid tert-butyl ester

To an ice-cold solution of compound 16k (70 mg, 0.153 mmol), 34 mg (0.168 mmol) of 5-bromo-pyridine-2-carboxylic acid, 27 mg (0.198 mmol) of HOAt and 44 mg (0.23 mmol) of EDC x HCl in DCM were added 0.053 ml (0.382 mmol) of Et₃N. The mixture was stirred overnight, diluted with EtOAc, washed with 5 % aq NaHCO₃ solution and brine, dried over Na₂SO₄ and evaporated. The residue was purified by chromatography on silica gel (heptane / EtOAc 0 to 40 % EtOAc) to yield the title compound in the form of a colourless foam {HPLC: Rt_{H6} = 2.713 min; ESIMS: 642, 644 [(M+H)⁺, 1Br]; ¹H-NMR (360 MHz, CDCl₃; very broad signals due to rotamers): 9.75 (s, 1 H), 8.61 (s, 1 H), 8.12-7.95 (m), 7.40 - 7.0 (m), 4.50-3.50 (m), 1.52-1.17 (br).

### 16m) 5-Bromo-pyridine-2-carboxylic acid [3-(6-amino-2-difluoromethyl-2,3,4,5-tetrahydro-pyrazin-2-yl)-4-fluoro-phenyl]-amide

A mixture of compound 161 (33 mg, 0.051 mmol) and 0.5 ml of 3N HCl in MeOH was stirred overnight. The mixture was evaporated, redissolved in methanol and triturated with TBME to give the hydrochloride salt of the title compound as white crystals. {TLC (DCM: MeOH: NH₃ (25 %, aq)/ 90: 10: 0.5) Rf 0.27; HPLC: Rt_{H2} = 2.677 min; ESIMS: 442, 444 [(M+H)⁺, 1Br]; 1 H-NMR (600 MHz, DMSO-d₆): 10.90 (s, 1 H), 10.73 (br s, 1 H), 9.79 (br s, 1 H), 8.88 (s, 1 H), 8.66 (br s, 1H), 8.35 (d, 1 H), 8.14-8.00 (m, 3H), 7.41-7.33 (m, 1 H), 6.85-6.61 (m, 1H), 3.95 (d, 1 H), 3.87 (d, 1H), 3.56 (d, 1H), 3.48 (d, 1 H)}.

### Example 17: 5-Cyano-pyridine-2-carboxylic acid [3-(6-amino-2-difluoromethyl-2,3,4,5-tetrahydro-pyrazin-2-yl)-4-fluoro-phenyl]-amide

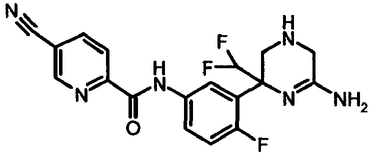

The title compound was prepared by procedures analogous to those used in Example 16 hereinbefore. {TLC (DCM: MeOH: NH3 (25 %, aq)/ 90: 10: 0.5) Rf 0.22; HPLC: Rt_{H1} = 2.783 min; ESIMS: 389 [(M+H)⁺]; 1H-NMR (360 MHz, DMSO-d₆): 10.78 (br s, 1H), 9.22 (s, 1 H), 8.60 (dd, 1H), 8.30 (d, 1H), 8.10-8.05 (m, 1H), 7.88 (br s, 1H), 7.21 (br s, 1H), 6.15 (t, 1H, *J*= 56 Hz), 5.92 (s, 1 H), 3.31-2.98 (br m, 4H)}.

### Example 18: 5-Cyano-pyridine-2-carboxylic acid [3-(6-amino-2-difluoromethyl-5-oxo-2,3,4,5-tetrahydro-pyrazin-2-yl)-4-fluoro-phenyl]-amide

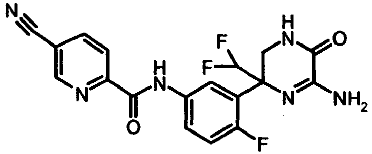

The title compound was isolated as a side product from the preparation of Example 17. {TLC (DCM: MeOH: NH3 (25 %, aq)/ 90: 10: 0.5) Rf 0.32; HPLC: Rt_{H1} = 2.780 min; ESIMS: 403 [(M+H)⁺]; 1 H-NMR (360 MHz, DMSO-d₆): 10.83 (s, 1H), 9.14 (d, 1 H), 8.52 (dd, 1H), 8.40 (s, 1 H), 8.21 (d, 1 H), 8.07 (dd, 1 H), 7.84-7.77 (m, 1H), 7.18 (dd, 1 H), 6.66 (br s, 1 H), 6.14 (t, 1H, *J*= 56 *Hz),* 3.81 (d, 1H), 3.70 (d, 1H}.

### Example 19: 5-Amino-3-{5-[(5-bromo-pyridine-2-carbonyl)-amino]-2-fluoro-phenyl}-3-difluoromethyl-3,6-dihydro-2H-pyrazine-1-carboxylic acid methyl ester

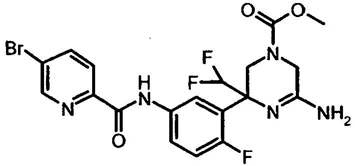

The title compound was prepared by procedures analogous to Example 16, except that in step 16g methyl chloroformate was used instead of Boc2O. {TLC (DCM: MeOH: NH3 (25 %, aq)/ 90: 10: 0.5) Rf 0.35; HPLC: Rt_{H1} = 3.120 min; ESIMS: 500, 502 [(M+H)⁺, 1Br]; 1H-NMR (600 MHz, DMSO-d₆ 1:1.5 mixture of rotamers): 11.01 (d, 1H), 10.95 (d, 1H), 9.89 (br s, 1H), 8.88 (s, 1 H), 8.79 (d, 1 H), 8.35 (d, 1 H), 8.09 (d, 1H), 7.94 (br s, 1 H), 7.39 (br s, 1 H), 6.81 (t, 1H, *J=* 54 *Hz),* 4.67-4.50 (m, 2H), 4.33-4.22 (m, 1 H), 3.94 (d, 1H), 3.56/ 3.40 (2 s, 3H)}.

### Example 20: 5-Amino-3-{5-[(5-cyano-pyridine-2-carbonyl)-amino]-2-fluoro-phenyl}-3-difluoromethyl-3,6-dihydro-2H-pyrazine-1-carboxylic acid methyl ester

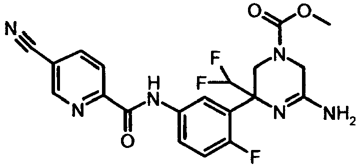

The title compound was prepared by procedures analogous to those used in the examples hereinbefore {TLC (DCM: MeOH: NH3 (25 %, aq)/ 90: 10: 0.5) Rf 0.60; HPLC: Rt_{H1} = 2.946 min; ESIMS: 447 [(M+H)⁺]; 1 H-NMR (400 MHz, DMSO-d₆: 10.81 (s, 1 H), 9.18 (s, 1H), 8.56 (dd, 1H), 8.25 (d, 1H), 8.00 (br s, 1H), 7.82 (br s, 1H), 7.19 (dd, 1 H), 6.29 (br s, 1 H), 6.15 (t, 1 H, J= 54 Hz), 3.97-3.67 (m, 4H), 3.53/ 3.46 (2 s, 3 H, rotameres, relation 1:1)}.

### Example 21: 5-Bromo-3-methyl-pyridine-2-carboxylic acid [3-(4-acetyl-6-amino-2-difluoromethyl-2,3,4,5-tetrahydro-pyrazin-2-yl)-4-fluoro-phenyl]-amide

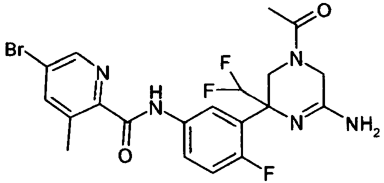

### 21 a) [1-Aminomethyl-2,2-difluoro-1-(2-fluoro-phenyl)-ethyl]-carbamic acid tert-butyl ester.

Compound 16d (4.0 g, 10.44 mmol) and 1.713 g (20.88 mmol) NaOAc were suspended in 50 ml EtOH and stirred under a hydrogen atmosphere in the presence of 200 mg of Pd on carbon (5 %), until all of the bromide was hydrogenated. The mixture was treated with 10% aq Na₂CO₃, filtered over celite, extracted with EtOAc, dried with Na₂SO₄ and evaporated. The residual oil was stirred with hexane and, after filtration, the title compound was isolated as a white solid. {HPLC: Rt_{H1} = 2.908 min; ESIMS: 305 [(M+H)⁺]; 1H-NMR (360 MHz, CDCl3): 7.43 (t, 1H), 7.36 (q, 1H), 7.21 (t, 1H), 7.11 (dd, 1H), 6.48 (t, CHF2), 5.78 (br s, 1H), 3.52 (br d, 1 H), 3.42 (br d, 1 H), 1.43 (br s, 9H}.

### 21b) [1-[(Cyanomethyl-amino)-methyl]-2,2-difluoro-1-(2-fluoro-phenyl)-ethyl]-carbamic acid tert-butyl ester

A mixture of 14.0 g (46.0 mmol) compound 21a, 9.22 g (55.2 mmol) iodoacetonitrile and 17.84 g (138 mmol) DIPEA in 90 ml ACN was stirred at 80 °C for 3 h. The mixture was diluted with EtOAc, washed with 1 N HCl, 5% aq NaHCO₃ and water. The organic phase was dried with sodium sulfate and the product was purified via chromatography on silica gel (heptane/ 30% EtOAc) to give the title compound as a yellowish oil. TLC: Rf 0.20 (EtOAc/ heptane 1:3; HPLC: Rt_{H3} = 2.682 min; ESIMS: 344 [(M+H)⁺]; ¹H-NMR (CDCl₃, 360 MHz): 7.44-7.34 (m, 2H), 7.22 (t, 1H), 7.13 (d d, 1H), 6.47 (t, 1H, CHF₂), 5.62 (br s, 1H),3.72-3.37 (m, 4H), 1.95 (br s, 1H), 1.33 (s, 9H).

### 21c) [2-tert-Butoxycarbonylamino-3,3-difluoro-2-(2-fluoro-phenyl)-propyl]-cyanomethyl-carbamic acid 2,2,2-trichloro-ethyl ester

To a vigorously stirred suspension of 16.0 g (46.6 mmol) compound 21 b in 80 ml DCM and 150 ml 10% aq NaHCO₃ were added dropwise 24.7 g (117 mmol) trichloroethyl chloroformate over a period of 10 minutes. The reaction temperature was kept below 26 °C with the aid of an ice-bath. Stirring was continued for 3.5 h at 25 °C. The phases were separated and the organic phase was dried with MgSO₄.H₂O, filtered, evaporated and purified via chromatography on silica gel (heptane/ 15% EtOAc) to give the title compound as a colorless foam. TLC: Rf 0.50 (EtOAc/ heptane 1:3; HPLC: Rt_{H6} = 2.758 min; ESIMS: 518 [(M+H)⁺, 3Cl]; ¹H-NMR (CDCl₃, 360 MHz, broad signals, 2:1 mixture of rotamers): 7.45-7.35 (m, 2H), 7.30-7.22 (m, 1H), 7.15 (dd, 1H); 6.88-6.50 (m, 1 H, CHF₂), 6.18 (br s, NH, major rotamer), 5.70 (br s, NH, minor rotamer), 4.92-4.22 (m, 6H), 1.48 (br s, 9H).

### 21d) 5-Amino-3-difluoromethyl-3-(2-fluoro-phenyl)-3,6-dihydro-2H-pyrazine-1-carboxylic acid 2,2,2-trichloro-ethyl ester

Compound 21c (24.17 g, 46.6 mmol) was dissolved in 93 ml DCM and treated with 87 ml 4N HCl in dioxane. After stirring for 4 h at room temperature the mixture was evaporated to yield the title compound as a colorless foam, pure enough for further syntheses.
TLC (DCM: MeOH: NH3 (25 %, aq.)/ 90: 10: 0.5) Rf 0.42. Rt_{H1} = 3.203 min; ESIMS: 418 [(M+H)⁺, 3Cl]; ¹H-NMR (DMSO-d6, 360 MHz, broad signals): 11.26 (s, 1 H), 10.0-9.9 (br, 1 H), 8.98 (br s, 1H), 7.65-7.50 (m, 2H), 7.48-7.35 (m, 2H), 6.83 (br t, CHF₂), 4.92-4.68 (m, 4H), 4.45-4.16 (m, 2H).

### 21e) 5-Amino-3-difluoromethyl-3-(2-fluoro-5-nitro-phenyl)-3,6-dihydro-2H-pyrazine-1-carboxylic acid 2,2,2-trichloro-ethyl ester

To a stirred solution of compound 21d (21.16 g, 46.5 mmol) in 60 ml 95% H₂SO₄ were added portion wise 6.11 g (60.5 mmol) KNO₃ while keeping the reaction temperature below 30 °C with the help of a water-bath. After 30 min the mixture was poured onto 200 g crushed ice and water. The mixture was neutralized with 4N NaOH and solid Na₂CO₃ (caution, foaming). The mixture was extracted with EtOAc twice, dried with Na₂SO₄ and evaporated the crude product was purified by crystallization from TBME/ hexanes to give the title compound as a white solid. TLC: Rf 0.50 (EtOAc/ heptane 1:3), Rt_{H1} = 3.211 min; ESIMS: 463 [(M+H)⁺, 3Cl]; ¹H-NMR (DMSO-d6, 360 MHz, broad signals): 8.64-8.56 (m, 1 H), 8.34-8.27 (m, 1 H), 7.55 (t, 2H), 6.63 (br d, 2H), 6.22 (t, CHF₂), 4.90-4.72 (m, 2H), 4.23-3.85 (m, 4H).

### 21f) 5-tert-Butoxycarbonylamino-3-difluoromethyl-3-(2-fluoro-5-nitro-phenyl)-3,6-dihydro-2H-pyrazine-1-carboxylic acid 2,2,2-trichloro-ethyl ester

The title compound was prepared from compound 21 e by a procedure similar to that used to obtain compound 16j.
TLC: Rf 0.36 (EtOAc/ heptane 1:3), Rt_{H6} = 3.010 min; ESIMS: 585 [(M+Na)⁺, 3Cl]; ¹H-NMR (DMSO-d6, 360 MHz, broad signals): 10.34 (br s, 1 H), 8.70-8.64 (m, 1H), 8.37-8.30 (m, 1 H), 7.59 (dd, 2H), 6.33 (br t, CHF₂), 4.93-4.66 (m, 3H), 4.53-4.28 (m, 2H), 3.84-3.75 (m, 1 H).

### 21g) 3-(5-Amino-2-fluoro-phenyl)-5-tert-butoxycarbonylamino-3-difluoromethyl-3,6-dihydro-2H-pyrazine-1-carboxylic acid 2,2,2-trichloro-ethyl ester

A mixture of compound 21f (3 g, 5.32 mmol). 2.97 g (53.2 mmol) Fe and 3.42 g (63.9 mmol) NH₄Cl in 55 ml MeOH was refluxed for 3 h. The mixture was filtered over celite and washed with EtOAc. The organic phase was washed with 5% NaHCO₃, brine and was dried with Na₂SO₄ and purified via chromatography on silica gel (heptane/EtOAc 0-50% EtOAc) to give the title compound as a colorless foam.
TLC: Rf 0.32 (EtOAc/ heptane 1:2), Rt_{H3} = 2.842 min; ESIMS: 533 [(M+H)⁺, 3Cl]; ¹H-NMR (CDCl₃, 360 MHz, broad signals): 7.39 (br s, 1 H), 6.98-6.84 (m, 2H), 6.75-6.55 (m, 3H), 6.28 (t, CHF₂), 4.90-3.55 (m, 6H), 1.55 and 1.52 (br s, 9H)

### 21h) 3-{5-[(5-Bromo-3-methyl-pyridine-2-carbonyl)-amino]-2-fluoro-phenyl}-5-tert-butoxycarbonylamino-3-difluoromethyl-3,6-dihydro-2H-pyrazine-1-carboxylic acid 2,2,2-trichloro-ethyl ester

The title compound was prepared from compound 21 g by a procedure similar to that used to obtain compound 16g.
TLC: Rf 0.25 (EtOAc/ heptane 1:3), {HPLC: Rt_{H6} = 3.535 min; ESIMS: 730, 732 [(M+H)⁺, 1 Br, 3Cl]; ¹H-NMR (360 MHz, CDCl₃): 10.18-9.98 (m, 1H), 8.54 (s, 1H), 8.11-7.98 (m, 1H), 7.85 (s, 1 H), 7.60 - 7.45 (m, 2H), 7.13 (t, 1 H), 6.78 (t, CHF2), 4.92-4.42 (m, 4H), 4.30-3.95 (m, 2H), 2.81 (s, 3H),1.55 (s, 9H).

### 21i) (6-{5-[(5-Bromo-3-methyl-pyridine-2-carbonyl)-amino]-2-fluoro-phenyl}-6-difluoromethyl-3,4,5,6-tetrahydro-pyrazin-2-yl)-carbamic acid tert-butyl ester

A mixture of compound 21 h (620 mg, 0.805 mmol), 526 mg (8.05 mmol) Zn powder and 43 mg (0.805 mmol) NH₄Cl in 4 ml MeOH was stirred 30 min. The mixture was made basic with a small amount 25% aq NH₄OH, filtered over celite and washed with MeOH and EtOAc. The filtrate was washed with brine, the aq phase extracted 3 times with EtOAc and the combined organic layers dried with Na₂SO₄. Purification via chromatography on silica gel (heptane/ 0-70% EtOAc/0.005% 25% aq NH4OH) gave the title compound as a colorless foam. TLC: Rf 0.31 (EtOAc/heptane 1:1), {HPLC: Rt_{H3} = 2.864 min; ESIMS: 556, 558 [(M+H)⁺, 1 Br]; ¹H-NMR (360 MHz, CDCl₃, broad signals due to rotamers): 10.15-10.0 (m, 1 H), 8.54 (br s, 1H), 8.11-8.02 (m, 1 H), 7.84 (s, 1 H), 7.63 - 7.60 (m, 1 H), 7.22-7.10 (m, 1 H), 6.6-6.0 (br, CHF2), 4.10-3.2 (m, 4H), 2.81 (s, 3H),1.56 and 1.51 (s, 9H).

### 21j) (4-Acetyl-6-{5-[(5-bromo,3-methyl-pyridine-2-carbonyl)-amino]-2-fluoro-phenyl}-6-difluoromethyl-3,4,5,6-tetrahydro-pyrazin-2-yl)-carbamic acid tert-butyl ester

A mixture of compound 21 i (150 mg, 0.270 mmol), 55 mg (0.539 mmol) acetic anhydride and 45 mg (0.566 mmol) pyridine 1 ml DCM was stirred for 1h. The mixture was quenched with 10% aq Na₂CO₃ and extracted with DCM. The org phase was dried with Na₂SO₄ and evaporated. Purification via chromatography on silica gel (heptane/EtOAc 0-50% EtOAc) gave the title compound as a colorless solid. TLC: Rf 0.19 (EtOAc/ heptane 1:2), {HPLC: Rt_{H3} = 3.242 min; ESI MS: 598, 600 [(M+H)⁺, 1Br]; ¹H-NMR (360 MHz, CDCl₃, ca 1:1 mixture of rotamers): 9.93 (br d, 1 H), 8.44 (br s, 1H), 8.03-7.0 (m, 5H), 6.4-5.85 (m, 1H), 4.75-3.65 (m, 4H), 2.70 (s, 3H),2.03 and 1.91 (s, 3H), 1.49 and 1.44 (s, 9H).

### 21k) 5-Bromo-3-methyl-pyridine-2-carboxylic acid [3-(4-acetyl-6-amino-2-difluoromethyl-2,3,4,5-tetrahydro-pyrazin-2-yl)-4-fluoro-phenyl]-amide

Compound 21j (140 mg, 0.234 mmol) was taken up in 0.5 ml DCM and 1 ml 4N HCl in dioxane was stirred 2 h. The mixture was evaporated, taken up in 10% Na₂CO₃ and EtOAc. The aq phase was extracted twice with EtOAc, the combined org layers were dried with Na₂SO₄. Purification via chromatography on silica gel (DCM/MeOH/25% aq NH40H 90:10:0.5) gave the title compound as a colorless solid. {TLC (DCM: MeOH: NH3 (25 %, aq)/ 90: 10: 0.5) Rf 0.34; HPLC: Rt_{H1} = 3.071 min; ESIMS: 498, 500 [(M+H)⁺, 1Br]; 1H-NMR (360 MHz, DMSO-d₆ (rotameric mixture, relation 2:1): 10.65-10.51 (m, 1 H), 8.66 (s, 1 H), 8.17 (s, 1 H), 7.91-7.75 (m, 2H), 7.30-7.11 (m, 1 H), 6.44-6.01 (m, 2H), 4.09-3.66 (m, 4H), 2.56-2.54 (m, 3H), 1.93-1.86 (m, 3H)}.

### Example 22: 5-Amino-3-{5-[(5-bromo-3-methyl-pyridine-2-carbonyl)-amino]-2-fluorophenyl}-3-difluoromethyl-3,6-dihydro-2H-pyrazine-1-carboxylic acid 2,2-dichloro-ethyl ester

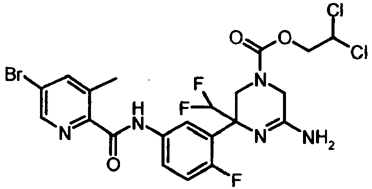

The title compound was prepared from a side product isolated in step 21 i. {TLC (DCM: MeOH: NH3 (25 %, aq)/ 90: 10: 0.5) Rf 0.44; HPLC: Rt_{H3} = 2.776 min; ESIMS: 598 [(M+H)⁺]; 1 H-NMR (400 MHz, DMSO-d₆, broad signals due to rotamers: 10.59-10.55 (m, 1 H), 8.62 (d, 1 H), 7.87-7.82 (m, 1 H), 7.82-7.75 (m, 1 H), 7.16 (br s, 1 H), 6.37-6.28 (m, 3H), 4.41-4.34 (m, 2H), 3.97-3.71 (m, 4H), 2.52 (s, 3H)}.

### Example 23: 5-Bromo-3-methyl-pyridine-2-carboxylic acid {3-[6-amino-2-difluoromethyl-4-(2-methoxy-acetyl)-2,3,4,5-tetrahydro-pyrazin-2-yl]-4-fluoro-phenyl}-amide

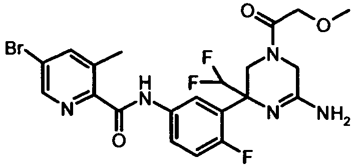

The title compound was prepared from compound 21 i using methoxy-acetyl chloride instead of acetic anhydride and by procedures analogous to those used in Example 21. {TLC (DCM: MeOH: NH3 (25 %, aq)/ 90: 10: 0.5) Rf 0.38; HPLC: Rt_{H1} = 3.083 min; ESIMS: 528, 530 [(M+H)⁺, 1Br]; 1H-NMR (360 MHz, DMSO-d₆(1:1 mixture of diastereomers): 10.64-10.52 (m, 1 H), 8.66 (s, 1 H), 8.16 (s, 1 H), 7.90-7.76 (m, 2H), 7.29-7.13 (m, 1 H), 6.47-6.04 (m, 2H), 6.41-6.28 (m, 2H), 6.22 (t, 1H, J= 55 *Hz),* 4.08-3.74 (m, 6H), 3.22-3.15 (m, 3H), 2.55 (s, **3H)}.**

### Example 24: 5-Bromo-3-methyl-pyridine-2-carboxylic acid [3-(6-amino-4-cyclopropanecarbonyl-2-difluoromethyl-2,3,4,5-tetrahydro-pyrazin-2-yl)-4-fluorophenyl]-amide

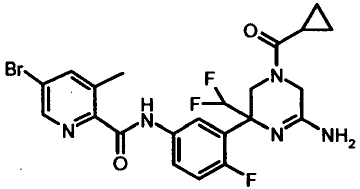

The title compound was prepared from compound 21 i using cyclopropanecarbonyl chloride instead of acetic anhydride and by procedures analogous to those used in Example 21. { HPLC: Rt_{H1} = 3.187 min; ESIMS: 524, 526 [(M+H)⁺, 1Br]; 1 H-NMR (360 MHz, DMSO-d₆ (2:1 mixture of rotamers): 10.63-10.53 (m, 1H), 8.66 (s, 1 H), 8.16 (s, 1 H), 7.92-7.74 (m, 2H), 7.27-7.12 (m, 1H), 6.37-6.29 (m, 2H), 6.25 (t, 1H, *J=55 Hz),* 4.48-3.75 (m, 4H), 2.55 (s, 3H), 1.82-1.65 (m, 1 H), 0.77-0.29 (m, 1H)}.

### Example 25: 5-Bromo-3-methyl-pyridine-2-carboxylic acid [3-(6-amino-2-difluoromethyl-2,3,4,5-tetrahydro-pyrazin-2-yl)-4-fluoro-phenyl]-amide

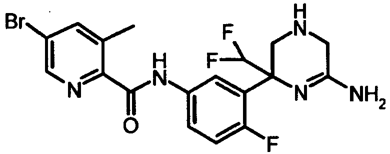

The title compound was prepared from compound 21 i via direct Boc-deprotection as in Example 21. {TLC (DCM: MeOH: NH3 (25 %, aq)/ 90: 10: 0.5) Rf 0.20; HPLC: Rt_{H1} = 3.050 min; ESIMS: 456, 458 [(M+H)⁺, 1 Br]; 1 H-NMR (360 MHz, DMSO-d₆): 10.53 (s, 1 H), 8.65 (s, 1H), 8.16 (s, 1H), 7.93-7.88 (m, 1H), 7.87-7.80 (m, 1H), 7.16(dd, 1H), 6.13(t, 1H, *J=* 57 *Hz),* 5.90 (br s, 1 H), 3.18 (t, 2H), 3.07 (t, 2H), 2.56 (s, 3H)}.

### Example 26: 5-Difluoromethoxy-3-methyl-pyridine-2-carboxylic acid [3-(4-acetyl-6-amino-2-difluoromethyl-2,3,4,5-tetrahydro-pyrazin-2-yl)-4-fluoro-phenyl]-amide

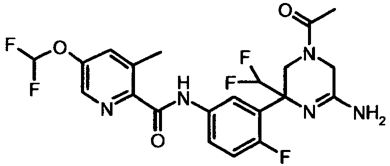

The title compound was prepared from compound 21g by procedures analogous to those used in Example 21 and instead using Acid 1 as a coupling partner in the amide coupling. { HPLC: Rt_{H2} = 2.795 min; ESIMS: 486 [(M+H)⁺]; 1 H-NMR (400 MHz, DMSO-d₆ (2 :1 mixture of rotamers): 10.56-10.46 (m, 1 H), 8.40 (s, 1 H), 7.87-7.74 (m, 1 H), 7.69 (s, 1 H), 7.25-7.09 (m, 2H), 6.37-5.99 (m, 3H), 4.04-3.94 (m, 1H), 3.84 (s, 1 H), 2.58-2.54 (m, 3H), 1.90-1.82 (m, 3H)}.

### Example 27: 5-Difluoromethoxy-3-methyl-pyridine-2-carboxylic acid [3-((R)-6-amino-2-difiuoromethyl-2,3,4,5-tetrahydro-pyrazin-2-yl)-4-fluoro-phenyl]-amide

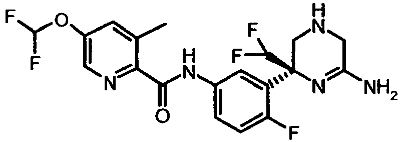

The title compound was prepared from compound 21g by procedures analogous to those used in Example 21 and instead using Acid 1 as a coupling partner in the amide coupling. The enantiomers were separated on Chiralpak® OD-H, 30 x 250 mm column using CO₂/(MeOH + 1 %IPAm)/ 60:40 (isocratic) as an eluent. The title compound is the faster moving enantiomer. { HPLC: Rt_{H1} = 3.047 min; ESIMS: 444 [(M+H)⁺]; 1 H-NMR (400 MHz, DMSO-d₆): 10.46 (s, 1 H), 8.40 (d, 1 H), 7.91-7.86 (m, 1H), 7.84-7.78 (m, 1H), 7.42 (t, 1 H, *J*= *73 Hz),* 7.12 (dd, 1 H), 6.71 (t, 1H, *J*=56 *Hz),* 5.88 (br s, 2H), 3.20-2.98 (m, 4H), 2.57 (s, 3H)}.

### Example 28: 3-Amino-5-methoxy-pyrazine-2-carboxylic acid [3-((R)-6-amino-2-difluoromethyl-2,3,4,5-tetrahydro-pyrazin-2-yl)-4-fluoro-phenyl]-amide

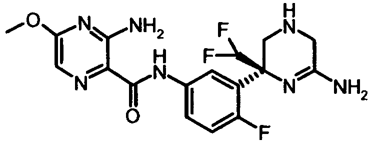

The title compound was prepared from compound 21 g by procedures analogous to those used in Example 21 and instead using Acid 2 as a coupling partner in the amide coupling. The enantiomers were separated on a Chiralpak® AD 20um (5x50cm) column using MeOH/EtOH/+0.01%DEA as an eluent. The title compound is the slower moving enantiomer. {HPLC: Rt_{H1} = 2.889 min; ESIMS: 410 [(M+H)⁺],; 1H-NMR (400 MHz, DMSO-d₆): 10.04 (s, 1H), 7.88 (dd, 1 H), 8.16 (s, 1H), 7.77-7.71 (m, 1 H), 7.50 (s, 1 H), 7.09 (dd, 1H), 6.09 (t, 1 H, *J=55 Hz),* 5.88 (br s, 1H), 3.88 (s, 3H), 3.21-2.94 (m, 5H)}.

### Example 29: 3-Amino-5-oxo-4,5-dihydro-pyrazine-2-carboxylic acid [3-((R)-6-amino-2-difluoromethyl-2,3,4,5-tetrahydro-pyrazin-2-yl)-4-fluoro-phenyl]-amide

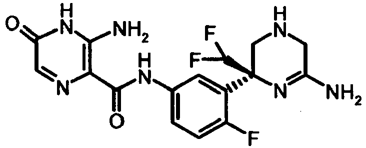

The title compound was isolated during the purification of Example 28 as a side product. {HPLC: Rt_{H1} = 2.427/ 2.547 min; ESIMS: 396 [(M+H)⁺]; 1H-NMR (600 MHz, DMSO-d₆): 10.00 (s, 1 H), 9.90 (s, 1 H), 8.90 (s, 1 H), 8.02-7.97 (m, 1 H), 7.84-7.80 (m, 1 H), 7.30 (dd, 1 H), 7.17 (s, 1 H), 6.71 (t, 1 H, *J=54 Hz),* 4.23 (d, 1 H), 4.12 (d, 1H), 3.80 (s, 2H)}.

### Preparation of Acid Intermediates

The substituted acid building blocks were either commercially available or can be prepared as described in the literature or in an analogous manner, e.g. WO 2005063738, WO 2009091016, WO 2010047372, Bioorg. Med. Chem. 2001, 9, 2061-2071, or can be prepared as described hereafter or in an analogous manner.

### Acid-1: 5-Difluoromethoxy-3-methyl-pyridine-2-carboxylic acid

### a) 5-Difluoromethoxy-3-methyl-pyridine-2-carbonitrile

A solution of 5-hydroxy-3-methyl-pyridine-2-carbonitrile (CAS registry 228867-86-5) (228 mg, 1.70 mmol), sodium chlorodifluoroacetate (CAS registry 1895-39-2) (518 mg, 3.40 mmol) and K₂CO₃ (705 mg, 5.10 mmol) in DMF (7 ml) was stirred for 0.5 h at 100 °C. The reaction mixture was diluted with EtOAc and washed with sat. aq. NH₄Cl soln. and brine. The aq. layers were reextracted with EtOAc, the combined organic layers dried over Na₂SO₄, filtrated and the filtrate was concentrated. The title compound was obtained as a colourless oil after flash chromatography on silica gel (cyclohexane / EtOAc gradient 0-3 min 95:5, 3-35 min 95:5 to 60:40).
UPLC Rt_{H7} = 0.87 min; ESIMS: 185 [(M+H)⁺];
¹H NMR (400 MHz, CDCl₃): 8.40 (d, 1 H), 7.45 (d, 1H), 6.64 (t, 1 H), 2.61 (s, 3H).

### b) 5-Difluoromethoxy-3-methyl-pyridine-2-carboxylic acid

To a solution of 5-difluoromethoxy-3-methyl-pyridine-2-carbonitrile (145 mg, 0.787 mmol) in EtOH (5 ml) was added 1 M aq. NaOH soln. (2.5 ml). The reaction mixture was stirred for 7h at 70 °C, then for 9h at room temperature. It was diluted with Et₂O and twice extracted with water. The combined aq. layers were reextracted with Et₂O, acidified to pH 2 with 1 M aq. HCl and twice extracted with TBME. The combined organic layers were dried over Na₂SO₄, filtrated and the filtrate was concentrated to yield the title compound as a white solid which was used for the next step without further purification.
UPLC Rt_{H7}= 0.61 min; ESIMS: 204 [(M+H)⁺];
¹H NMR (400 MHz, MeOD): 8.32 (d, 1 H), 7.61 (d, 1 H), 7.06 (t, 1H), 2.64 (s, 3H).

### Acid-2: 3-Amino-5-methoxy-pyrazine-2-carboxylic acid

### a) 3-Amino-5-methoxy-pyrazine-2-carboxylic acid methyl ester

At 0 °C 75 mg (1.866 mmol) 60% sodium hydride in oil was added in portions to 5 ml MeOH and the mixture was stirred at room temperature for 30 min. After re-cooling to 0 °C 350 mg (1.866 mmol) 3-amino-5-chloro-pyrazine-2-carboxylic acid methyl ester (GB 1248146) was added and the mixture was allowed to warm to room temperature and stirred over night. Saturated aq. NH₄Cl was added and the mixture was extracted with DCM and EtOAc, the combined organic layers were washed with saturated aq. sodium chloride, dried with Na₂SO₄ and evaporated. The residue was purified by chromatography on silica gel (cyclohexane to EtOAc) to provide the title compound as colorless solid.
UPLC: Rt_{H7} = 0.61 min; ESIMS [M+H]⁺= 184.2;
¹H-NMR (360 MHz, DMSO-*d*₆): 7.52 (s, 1 H), 7.49 (br s, 2 H), 3.91 (s, 3 H), 3.81 (s, 3 H).

### b) 3-Amino-5-methoxy-pyrazine-2-carboxylic acid

To a solution of 200 mg (1.092 mmol) 3-amino-5-methoxy-pyrazine-2-carboxylic acid methyl ester in 4 ml THF was added 1.20 ml (1.20 mmol) 1 N sodium hydroxide and the mixture was stirred at room temperature for 29 h. To the mixture were added 1.09 ml (1.09 mmol) 1 N HCl after stirring for 5 min toluene was added and the solvents were evaporated to provide the title compound together with sodium chloride as colorless solid. The mixture was used for coupling reactions without further purification.
HPLC: Rt_{H8}= 0.52 min; ESIMS [M+H]⁺ = 170.0;
¹H NMR (600 MHz, DMSO-*d*₆): 12.48 (br s, 1 H), 7.57 (br s, 2 H), 7.48 (s, 1 H), 3.88 (s, 3 H).

### Example 30: Biological activity of compounds of the formula I

The compounds of the Examples hereinbefore show the following IC₅₀ values in Test 1 described hereinbefore:

**Table 1**

| **Example** | **Bace IC₅₀ [µM]** | **Example** | **Bace IC₅₀ [µM]** |
|---|---|---|---|
| 1 | 4.1 | 2 | 0.18 |
| 3 | 0.4 | 4 | 0.095 |
| 5 | 0.12 | 6 | 0.051 |
| 7 | 0.068 | 8 | 0.021 |
| 9 | 0.033 | 10 | 0.034 |
| 11 | 0.02 | 12 | 0.02 |
| 13 | 0.007 | 14 | 0.063 |
| 15 | 1.3 | 16 | 0.03 |
| 17 | 0.028 | 18 | 0.021 |
| 19 | 0.022 | 20 | 0.052 |
| 21 | 0.009 | 22 | 0.005 |
| 23 | 0.007 | 24 | 0.011 |
| 25 | 0.028 | 26 | 0.023 |
| 27 | 0.022 | 28 | 0.02 |
| 29 | 0.22 | | |

Compounds of the Examples hereinbefore show the following IC₅₀ values in Test 4 described hereinbefore:

**Table 2**

| **Example** | **Bace IC₅₀ [µM]** | **Example** | **Bace IC₅₀ [µM]** |
|---|---|---|---|
| 1 | 3.2 | 2 | 0.12 |
| 3 | 0.26 | 4 | 0.091 |
| 5 | 0.19 | 6 | 0.059 |
| 7 | 0.16 | 8 | 0.074 |
| 9 | 0.037 | 10 | 0.074 |
| 11 | 0.04 | 12 | 0.084 |
| 13 | 0.28 | 14 | 0.13 |
| 15 | 0.98 | 16 | 0.0067 |
| 17 | 0.074 | 18 | 0.061 |
| 19 | 0.015 | 20 | 0.057 |
| 21 | 0.031 | 22 | 0.021 |
| 23 | 0.034 | 24 | 0.028 |
| 25 | 0.01 | 26 | 0.044 |
| 27 | 0.012 | 28 | 0.0057 |
| 29 | 0.86 | | |

The following are further embodiments on the invention:
Embodiment 1: A compound of formula (I), or a Pharmaceutical acceptable salt thereof, 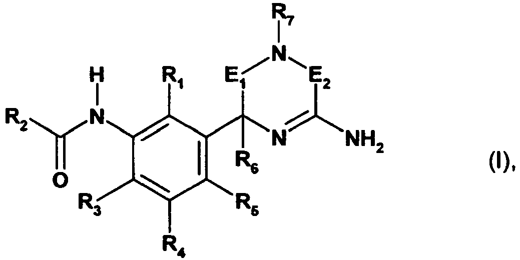 in which
   R₁ is hydrogen, cyano, halogen, (C₁-₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl;
   R₂ is an aryl, heteroaryl or non-aromatic heterocyclyl group G₁, which group G₁ is optionally substituted by 1 to 4 substituents independently selected from the group, consisting of cyano, amino, aminocarbonyl, halogen, (C₁₋₈)alkyl, halogen-(C₁₋₈)alkyl, hydroxy, oxo, (C₁₋₈)alkoxy, halogen-(C₁₋₈)alkoxy, (C₁₋₈)alkylthio, halogen-(C₁₋₈)alkylthio, (C₁₋₈)alkoxy-(C₁-₈)alkyl, (C₁₋₈)alkoxy-(C₁₋₈)alkoxy, (C₁₋₈)alkoxy-(C₁₋₈)alkyltlhio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl, (C₂₋₈)alkenoxy, (C₂₋₈)alkynoxy and a (C₃₋₈)cycloalkyl, aryl, heteroaryl or non-aromatic heterocyclyl group G₂, which group G₂ is optionally substituted by 1 to 4 substituents independently selected from the group, consisting of cyano, aminocarbonyl, halogen, (C₁-₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkyltlhio-(C₁₋₈)alkylthio, (C₂₋₈)alkenyl and (C₂₋₈)alkynyl;
   R₃ is hydrogen, cyano, halogen, (C₁₋₈)alkyl, halogen-(C₁₋₈)alkyl, (C₁₋₈)alkoxy; halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl;
   either
   R₄ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C_{1- 8})alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl; and
   R₅ is hydrogen, cyano, halogen, (C₁-₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, or (C₂₋₈)alkynyl;
   or
   R₄ and R₅, taken together, are -C(H)=C(H)-C(H)=C(H)- or a (C₁₋₈)alkylene group, in which (C₁₋₈)alkylene group 1 or 2 -CH₂- ring members are optionally replaced with hetero ring members independently selected from the group, consisting of -N(H)-, -N[(C₁₋₈)alkyl]-, -O-, -S-, -S(=O)- or -S(=O)₂-;
   R₆ is hydrogen, (C₁₋₈)alkyl, halogen-(C₁₋₈)alkyl, hydroxy-(C₁₋₈)alkyl, (C₁₋₈)alkoxy-(C₁₋₈)alkyl, mercapto-(C₁₋₈)alkyl, (C₁₋₈)alkytthio-(C₁₋₈)alkyl, amino-(C₁₋₈)alkyl, N-(C₁₋₈)alkylamino-(C₁₋₈)alkyl, N,N-di-[(C₁₋₈)alkyl]amino-(C₁₋₈)alkyl with two identical or different (C₁₋₈)alkyl moieties in the N,N-di-[(C₁₋₈)alkyl]amino moiety, (C₂₋₈)alkenyl, or (C₂₋₈)alkynyl;
   R₇ is hydrogen, (C₁₋₈)alkyl, (C₁₋₈)alkyl substituted by halogen, (C₃₋₈)cycloalkyl-(C₁₋₈)alkyl, (C₃₋₈)cycloalkoxy-(C₁₋₈)alkyl, aryloxy-(C₁₋₈)alkyl, (C₁₋₈)alkoxy-(C₁₋₈)alkyl, (C₁₋₈)alkylthio-(C₁₋₈)alkyl, (C₁₋₈)alkylsulfinyl, (C₁₋₈)alkylsulfinyl-(C₁₋₈)alkyl, (C₁₋₈)alkylsulfonyl, (C₁₋₈)alkylsulfonyl-(C₁₋₈)alkyl, amino-(C₁₋₈)alkyl, (C₁₋₈)alkylamino-(C₁₋₈)alkyl, di(C₁₋₈)alkylamino-(C₁₋₈)alkyl with two identical or different (C₁₋₈)alkyl moieties in the di(C₁₋₈)alkylamino moiety, aminosulfonyl, (C₁₋₈)alkylaminosulfonyl, di(C₁₋₈)alkylaminosulfonyl with two identical or different (C₁₋₈)alkyl moieties, formyl, (C₁₋₈)alkylcarbonyl, formyl-(C₁₋₈)alkyl, (C₁₋₈)alkylcarbonyl-(C₁₋₈)alkyl, (C₁₋₈)alkoxycarbonyl, halogen-(C₁₋₈)alkoxycarbonyl, (C₁₋₈)alkoxycarbonyl-(C₁₋₈)alkyl, (C₁₋₈)alkoxy-(C₁₋₈)alkylcarbonyl, or a (C₃₋₈)cycloalkylcarbonyl, arylcarbonyl, aryl-(C₁₋₈)alkylcarbonyl, heteroarylcarbonyl, heteroaryl-(C₁₋₈)alkylcarbonyl, non-aromatic heterocyclyl-carbonyl, (C₃₋₈)cycloalkylsulfonyl, arylsulfonyl, aryl-(C₁₋₈)alkylsulfonyl, heteroarylsulfonyl, heteroaryl-(C₁₋₈)alkylsulfonyl, non-aromatic heterocyclylsulfonyl, (C₃₋₈)cycloalkyl, aryl, aryl-(C₁₋₈)alkyl, heteroaryl, heteroaryl-(C₁₋₈)alkyl or non-aromatic heterocyclyl group G₃, which group G₃ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl, (C₂₋₈)alkynyl and a (C₃₋₈)cycloalkyl, aryl, heteroaryl or non-aromatic heterocyclyl group G₄, which group G₄ is optionally substituted by 1 to 4 substituents independently selected from the group, consisting of cyano, aminocarbonyl, halogen, (C₁-₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl and (C₂₋₈)alkynyl;
   E₁ is -C(R₈)(R₉)-, or -C(R₈)(R₉)-C(R₁₀)(R₁₁)-;
   E₂ is -C(R₁₂)(R₁₃)-, or -C(R₁₂)(R₁₃)-C(R₁₄)(R₁₅)-;
   either
   each of R₈ and R₉ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
   or
   R₈ and R₉, taken together, are oxo or -CH₂-CH₂-;
   either
   each of R₁₀ and R₁₁ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
   or
   R₁₀ and R₁₁, taken together, are oxo or -CH₂-CH₂-;
   either
   each of R₁₂ and R₁₃ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
   or
   R₁₂ and R₁₃, taken together, are oxo or -CR₁₆R₁₇CR₁₈R₁₉-
   wherein R₁₆, R₁₇, R₁₈ and R₁₉ are independently selected from hydrogen and fluoro; and
   either
   each of R₁₄ and R₁₅ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₋₈)alkyl;
   or
   R₁₄ and R₁₅, taken together, are oxo or -CH₂-CH₂-.
Embodiment 2: A compound according to Embodiment 1, or a pharmaceutically acceptable salt thereof, wherein R₁ is hydrogen.
Embodiment 3: A compound according to Embodiment 1 or Embodiment 2, or a pharmaceutically acceptable salt thereof, wherein R₂ is phenyl or a 5- or 6-membered heteroaryl group G₁ in which structure 1, 2, 3, or 4 ring members are hetero ring members independently selected from the group consisiting of a nitrogen ring member, an oxygen ring member and a sulfur ring member, which group G₁ is optionally substituted by 1, 2, 3 or 4 substituents independently selected from the group, consisting of cyano, amino, aminocarbonyl, halogen, (C₁₋₄)alkyl, halogen-(C₁₄)alkyl, hydroxy, oxo, (C₁₄)alkoxy, halogen-(C₁₄)alkoxy, (C₁₄)alkylthio, halogen-(C₁₋₄)alkylthio, (C₁₋₄)alkoxy-(C₁₋₄)alkyl, (C₁₋₄)alkoxy-(C₁₄)alkoxy, (C₁₋₄)alkoxy-(C₁₋₄)alkylthio, (C₁₋₄)alkylthio-(C₁₋₄)alkyl, (C₁₋₄)alkylthio-(C₁₋₄)alkoxy, (C₁₄)alkylthio-(C₁₋₄)alkylthio, (C₂₋₄)alkenyl, (C₂₋₄)alkynyl, (C₂₋₄)alkenoxy, and (C₂₋₄)alkynoxy.
Embodiment 4: A compound according to any one of Embodiments 1 to 3, or a pharmaceutically acceptable salt thereof, wherein R₃ is hydrogen.
Embodiment 5: A compound according to any one of Embodiments 1 to 4, or a pharmaceutically acceptable salt thereof, wherein R₄ is hydrogen, or halogen; and R₅ is hydrogen, or halogen.
Embodiment 6: A compound according to any one of Embodiments 1 to 5, or a pharmaceutically acceptable salt thereof, wherein R₆ is (C₁₋₃)alkyl, or halogen-(C₁-₃)alkyl.
Embodiment 7: A compound according to any one of Embodiments 1 to 6, or a pharmaceutically acceptable salt thereof, wherein R₇ is hydrogen, (C₁₋₆)alkyl, halogen-(C₁₆)alkyl, (C₁₋₄)alkoxy-(C₁₋₄)alkyl, (C₁₋₆)alkylcarbonyl, (C₁₋₆)alkoxycarbonyl, halogen-(C₁₆)alkoxycarbonyl, (C₁₋₆)alkoxy-(C₁₋₆)alkylcarbonyl, (C₃₋₈)cycloalkyl, (C₃₋₆)cycloalkyl-carbonyl, or a heteroaryl group optionally substituted by 1, 2, or 3 substituents independently selected from the group consisting of cyano, halogen, hydroxyl, (C₁₋₄)alkyl, halogen-(C₁₋₄)alkyl, (C₁₄)alkoxy, halogen-(C₁₋₄)alkoxy, (C₁₋₃)alkoxy-(C₁₋₃)alkyl and (C₁₋₃)alkoxy-(C₁₋₃)alkoxy.
Embodiment 8: A compound according to any one of Embodiments 1 to 7, or a pharmaceutically acceptable salt thereof, wherein E₁ is -C(R₈)(R₉)- and
   either
   each of R₈ and R₉ is independently selected from the group, consisting of hydrogen, cyano, halogen, (C₁₋₃)alkyl and halogen-(C₁₋₃)alkyl;
   or
   R₈ and R₉, taken together, are oxo or -CH₂-CH₂-.
Embodiment 9. A compound according to any one of Embodiments 1 to 8, or a pharmaceutically acceptable salt thereof, wherein E₂ is -C(R₁₂)(R₁₃)- and
   either
   each of R₁₂ and R₁₃ is independently selected from the group, consisting of hydrogen, cyano, halogen, (C₁₋₃)alkyl and halogen-(C₁₋₃)alkyl;
   or
   R₁₂ and R₁₃, taken together, are oxo or -CH₂-CH₂-.
Embodiment 10: A compound according to any one of Embodiments 1 to 9, or a pharmaceutically acceptable salt thereof, which is selected from the group consisting of:
   5-Bromo-pyridine-2-carboxylic acid [3-(6-amino-2,4-dimethyl-3-oxo-2,3,4,5-tetrahydro-pyrazin-2-yl)-4-fluoro-phenyl]-amide;
   5-Bromo-pyridine-2-carboxylic acid [3-(6-amino-2,4-dimethyl-2,3,4,5-tetrahydro-pyrazin-2-yl)-4-fluoro-phenyl]-amide;
   5-Bromo-pyridine-2-carboxylic acid [3-(6-amino-2,4-dimethyl-3-oxo-2,3,4,5-tetrahydro-pyrazin-2-yl)-phenyl]-amide;
   5-Bromo-pyridine-2-carboxylic acid [3-(6-amino-2,4-dimethyl-5-oxo-2,3,4,5-tetrahydro-pyrazin-2-yl)-4-fluoro-phenyl]-amide;
   5-Chloro-pyridine-2-carboxylic acid [3-(6-amino-2,4-dimethyl-5-oxo-2,3,4,5-tetrahydro-pyrazin-2-yl)-4-fluoro-phenyl]-amide;
   5-Bromo-pyridine-2-carboxylic acid [3-(6-amino-4-ethyl-2-methyl-5-oxo-2,3,4,5-tetrahydro-pyrazin-2-yl)-4-fluoro-phenyl]-amide;
   3,5-Dichloro-pyridine-2-carboxylic acid [3-(6-amino-2,4-dimethyl-5-oxo-2,3,4,5-tetrahydro-pyrazin-2-yl)-4-fluoro-phenyl]-amide;
   3,5-Dichloro-pyridine-2-carboxylic acid [3-(6-amino-4-ethyl-2-methyl-5-oxo-2,3,4,5-tetrahydro-pyrazin-2-yl)-4-fluoro-phenyl]-amide;
   5-Bromo-pyridine-2-carboxylic acid [3-(6-amino-4-isopropyl-2-methyl-5-oxo-2,3,4,5-tetrahydro-pyrazin-2-yl)-4-fluoro-phenyl]-amide;
   3,5-Dichloro-pyridine-2-carboxylic acid {3-[6-amino-4-(2-methoxy-ethyl)-2-methyl-5-oxo-2,3,4,5-tetrahydro-pyrazin-2-yl]-4-fluoro-phenyl}-amide;
   5-Bromo-pyridine-2-carboxylic acid {3-[6-amino-4-(2-methoxy-ethyl)-2-methyl-5-oxo-2,3,4,5-tetrahydro-pyrazin-2-yl]-4-fluoro-phenyl}-amide;
   5-Bromo-pyridine-2-carboxylic acid {3-[6-amino-2-methyl-4-(1-methyl-1H-pyrazol-4-yl)-5-oxo-2,3,4, 5-tetrahydro-pyrazin-2-yl]-4-fluoro-phenyl}-amide;
   5-Bromo-pyridine-2-carboxylic acid [3-(6-amino-2-methyl-5-oxo-4-pyridin-3-yl-2,3,4,5-tetrahydro-pyrazin-2-yl)-4-fluoro-phenyl]-amide;
   5-Cyano-pyridine-2-carboxylic acid [3-(6-amino-4-ethyl-2-methyl-5-oxo-2,3,4,5-tetrahydro-pyrazin-2-yl)-4-fluoro-phenyl]-amide;
   5-Bromo-pyridine-2-carboxylic acid [3-(6-amino-5-ethyl-2,4-dimethyl-3-oxo-2,3,4,5-tetrahydro-pyrazin-2-yl)-phenyl]-amide;
   5-Bromo-pyridine-2-carboxylic acid [3-(6-amino-2-difluoromethyl-2,3,4,5-tetrahydro-pyrazin-2-yl)-4-fluoro-phenyl]-amide;
   5-Cyano-pyridine-2-carboxylic acid [3-(6-amino-2-difluoromethyl-2,3,4,5-tetrahydro-pyrazin-2-yl)-4-fluoro-phenyl]-amide;
   5-Cyano-pyridine-2-carboxylic acid [3-(6-amino-2-difluoromethyl-5-oxo-2,3,4,5-tetrahydro-pyrazin-2-yl)-4-fluoro-phenyl]-amide;
   5-Amino-3-{5-[(5-bromo-pyridine-2-carbonyl)-amino]-2-fluoro-phenyl}-3-difluoromethyl-3,6-dihydro-2H-pyrazine-1-carboxylic acid methyl ester;
   5-Amino-3-{5-[(5-cyano-pyridine-2-carbonyl)-amino]-2-fluoro-phenyl}-3-difluoromethyl-3,6-dihydro-2H-pyrazine-1-carboxylic acid methyl ester;
   5-Bromo-3-methyl-pyridine-2-carboxylic acid [3-(4-acetyl-6-amino-2-difluoromethyl-2,3,4,5-tetrahydro-pyrazin-2-yl)-4-fluoro-phenyl]-amide;
   5-Amino-3-{5-[(5-bromo-3-methyl-pyridine-2-carbonyl)-amino]-2-fluoro-phenyl}-3-difluoromethyl-3,6-dihydro-2H-pyrazine-1-carboxylic acid 2,2-dichloro-ethyl ester;
   5-Bromo-3-methyl-pyridine-2-carboxylic acid {3-[6-amino-2-difluoromethyl-4-(2-methoxy-acetyl)-2,3,4,5-tetrahydro-pyrazin-2-yl]-4-fluoro-phenyl}-amide;
   5-Bromo-3-methyl-pyridine-2-carboxylic acid [3-(6-amino-4-cyclopropanecarbonyl-2-difluoromethyl-2,3,4,5-tetrahydro-pyrazin-2-yl)-4-fluoro-phenyl]-amide;
   5-Bromo-3-methyl-pyridine-2-carboxylic acid [3-(6-amino-2-difluoromethyl-2,3,4,5-tetrahydro-pyrazin-2-yl)-4-fluoro-phenyl]-amide;
   5-Difluoromethoxy-3-methyl-pyridine-2-carboxylic acid [3-(4-acetyl-6-amino-2-difluoromethyl-2,3,4,5-tetrahydro-pyrazin-2-yl)-4-fluoro-phenyl]-amide;
   5-Difluoromethoxy-3-methyl-pyridine-2-carboxylic acid [3-(6-amino-2-difluoromethyl-2,3,4,5-tetrahydro-pyrazin-2-yl)-4-fluoro-phenyl]-amide;
   3-Amino-5-methoxy-pyrazine-2-carboxylic acid [3-(6-amino-2-difluoromethyl-2,3,4,5-tetrahydro-pyrazin-2-yl)-4-fluoro-phenyl]-amide; and
   3-Amino-5-oxo-4,5-dihydro-pyrazine-2-carboxylic acid [3-(6-amino-2-difluoromethyl-2,3,4,5-tetrahydro-pyrazin-2-yl)-4-fluoro-phenyl]-amide.
Embodiment 11: A compound according to any one of Embodiments 1 to 10, or a pharmaceutically acceptable salt thereof, for use as a medicament.
Embodiment 12: A compound according to any one of Embodiments 1 to 10, or a pharmaceutically acceptable salt thereof, for use in the treatment or prevention of Alzheimer's disease or mild cognitive impairment.
Embodiment 13: A pharmaceutical composition comprising a compound according to any one of Embodiments 1 to 10, or a pharmaceutically acceptable salt thereof, as active ingredient and a pharmaceutical carrier or diluent.
Embodiment 14: The use of a compound according to any one of Embodiments 1 to 10, or a pharmaceutically acceptable salt thereof, for the manufacture of a medicament for the treatment or prevention of Alzheimer's disease or mild cognitive impairment.
Embodiment 15: A combination comprising a therapeutically effective amount of a compound according to any one of Embodiments 1 to 10, or a pharmaceutically acceptable salt thereof, and a second drug substance, for simultaneous or sequential administration.

## Claims

1. A compound of formula (I), or a pharmaceutically acceptable salt thereof, 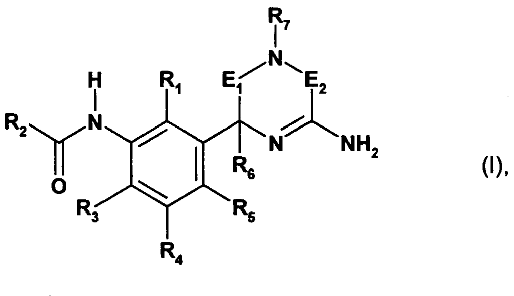 in which
R₁ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₈)alkylthio-(C₁₋₈)alkylthio, (C₂₋₈)alkenyl, or (C₂₋₈)alkynyl;
R₂ is an aryl, heteroaryl or non-aromatic heterocyclyl group G₁, which group G₁ is optionally substituted by 1 to 4 substituents independently selected from the group, consisting of cyano, amino, aminocarbonyl, halogen, (C₁₋₈)alkyl, halogen-(C₁₋₈)alkyl, hydroxy, oxo, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₈)alkylthio-(C₁₋₈)alkoxy, (C₁₋₈)alkylthio-(C₁₋₈)alkylthio, (C₂₋₈)alkenyl, (C₂₋₈)alkynyl, (C₂₈)alkenoxy, (C₁₋₈)alkynoxy and a (C₃₋₈)cycloalkyl, aryl, heteroaryl or non-aromatic heterocyclyl group G₂, which group G₂ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alk-ylthio, (C₂₋₈)alkenyl and (C₂₈)alkynyl;
R₃ is hydrogen, cyano, halogen, (C₁₋₈)alkyl, halogen-(C₁₋₈)alkyl, (C₁₋₈)alkoxy; halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₈)alkylthio-(C₁₋₈)alkylthio, (C₂₋₈)alkenyl, or (C₂₋₈)alkynyl;
either
R₄ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₈)alkylthio-(C₁₋₈)alkylthio, (C₂₋₈)alkenyl, or (C₂₋₈)alkynyl; and
R₅ is hydrogen, cyano, halogen, (C₁₋₈)alkyl, halogen-(C₁₋₈)alkyl, (C₁₋₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₈)alkylthio-(C₁₋₈)alkylthio, (C₁₋₈)alkenyl, or (C₁₋₈)alkynyl;
or
R₄ and R₅, taken together, are -C(H)=C(H)-C(H)=C(H)- or a (C₁₋₈)alkylene group, in which (C₁₋₈)alkylene group 1 or 2 -CH₂- ring members are optionally replaced with hetero ring members independently selected from the group, consisting of -N(H)-, -N[(C₁₋₈)alkyl]-, -O-, -S-, -S(=O)- or -S(=O)₂-;
R₆ is hydrogen, (C₁₋₈)alkyl, halogen-(C₁₋₈)alkyl, hydroxy-(C₁₋₈)alkyl, (C₁₋₈)alkoxy-(C₁₈)alkyl, mercapto-(C₁₋₈)alkyl, (C₁₋₈)alkylthio-(C₁₋₈)alkyl, amino-(C₁₋₈)alkyl, N-(C₁₋₈)alkylamino-(C₁₋₈)alkyl, N,N-di-[(C₁₋₈)alkyl]amino-(C₁₋₈)alkyl with two identical or different (C₁₋₈)alkyl moieties in the N,N-di-[(C₁₋₈)alkyl]amino moiety, (C₂₋₈)alkenyl, or (C₂₋₈)alkynyl;
R₇ is hydrogen, (C₁₋₈)alkyl, (C₁₋₈)alkyl substituted by halogen, (C₃₋₈)cycloalkyl-(C₁₈)alkyl, (C₃₋₈)cycloalkoxy-(C₁₋₈)alkyl, aryloxy-(C₁₋₈)alkyl, (C₁₋₈)alkoxy-(C₁₋₈)alkyl, (C₁₋₈)alkylthio-(C₁₋₈)alkyl, (C₁₋₈)alkylsulfinyl, (C₁₋₈)alkylsulfinyl-(C₁₋₈)alkyl, (C₁₋₈)alkylsulfonyl, (C₁₈)alkylsulfonyl-(C₁₋₈)alkyl, amino-(C₁₋₈)alkyl, (C₁₋₈)alkylamino-(C₁₋₈)alkyl, di(C₁₋₈)alkylamino-(C₁₈)alkyl with two identical or different (C₁₋₈)alkyl moieties in the di(C₁₋₈)alkylamino moiety, aminosulfonyl, (C₁₋₈)alkylaminosulfonyl, di(C₁₋₈)alkylaminosulfonyl with two identical or different (C₁₋₈)alkyl moieties, formyl, (C₁₋₈)alkylcarbonyl, formyl-(C₁₋₈)alkyl, (C₁₋₈)alkylcarbonyl-(C₁₋₈)alkyl, (C₁₋₈)alkoxycarbonyl, halogen-(C₁₋₈)alkoxycarbonyl, (C₁₋₈)alkoxycarbonyl-(C₁₈)alkyl, (C₁₋₈)alkoxy-(C₁₋₈)alkylcarbonyl, or a (C₃₋₈)cycloalkylcarbonyl, arylcarbonyl, aryl-(C₁₈)alkylcarbonyl, heteroarylcarbonyl, heteroaryl-(C₁₋₈)alkylcarbonyl, non-aromatic heterocyclyl-carbonyl, (C₃₋₈)cycloalkylsulfonyl, arylsulfonyl, aryl-(C₁₋₈)alkylsulfonyl, heteroarylsulfonyl, heteroaryl-(C₁₋₈)alkylsulfonyl, non-aromatic heterocyclylsulfonyl, (C₃₋₈)cycloalkyl, aryl, aryl-(C₁₋₈)alkyl, heteroaryl, heteroaryl-(C₁₋₈)alkyl or non-aromatic heterocyclyl group G₃, which group G₃ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₋₈)alkoxy, halogen-(C₁₋₈)alkoxy, (C₁₋₈)alkylthio, halogen-(C₁₋₈)alkylthio, (C₁₋₈)alkoxy-(C₁₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₈)alkylthio-(C₁₋₈)alkoxy, (C₁₋₈)alkylthio-(C₁₋₈)alkylthio, (C₂₋₈)alkenyl, (C₂₋₈)alkynyl and a (C₃₋₈)cycloalkyl, aryl, heteroaryl or non-aromatic heterocyclyl group G₄, which group G₄ is optionally substituted by 1 to 4 substituents independently selected from the group, consisting of cyano, aminocarbonyl, halogen, (C₁₋₈)alkyl, halogen-(C₁₋₈)alkyl, hydroxy, (C₁₈)alkoxy, halogen-(C₁₋₈)alkoxy, (C₁₋₈)alkylthio, halogen-(C₁₋₈)alkylthio, (C₁₋₈)alkoxy-(C₁₋₈)alkyl, (C₁₋₈)alkoxy-(C₁₋₈)alkoxy, (C₁₋₈)alkoxy-(C₁₋₈)alkylthio, (C₁₋₈)alkylthio-(C₁₋₈)alkyl, (C₁₋₈)alkylthio-(C₁₋₈)alkoxy, (C₁₋₈)alkylthio-(C₁₋₈)alkylthio, (C₂₋₈)alkenyl and (C₂₋₈)alkynyl;
E₁ is -C(R₈)(R₉)-, or -C(R₈)(R₉)-C(R₁₀)(R₁₁)-;
E₂ is -C(R₁₂)(R₁₃)-, or -C(R₁₂)(R₁₃)-C(R₁₄)(R₁₅)-;
either
each of R₈ and R₉ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₈)alkyl;
or
R₈ and R₉, taken together, are oxo or -CH₂-CH₂-;
either
each of R₁₀ and R₁₁ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₈)alkyl;
or
R₁₀ and R₁₁, taken together, are oxo or -CH₂-CH₂-;
either
each of R₁₂ and R₁₃ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₈)alkyl;
or
R₁₂ and R₁₃, taken together, are oxo or -CR₁₆R₁₇-CR₁₈R₁₉-
wherein R₁₆, R₁₇, R₁₈ and R₁₉ are independently selected from hydrogen and fluoro; and
either
each of R₁₄ and R₁₅ is independently selected from the group, consisting of hydrogen, cyano, halogen, (C₁₋₈)alkyl, halogen-(C₁₋₈)alkyl, (C₁₋₈)alkoxy-(C₁₋₈)alkyl and (C₁₋₈)alkylthio-(C₁₈)alkyl;
or
R₁₄ and R₁₅, taken together, are oxo or -CH₂-CH₂-.

2. A compound according to Claim 1, or a pharmaceutically acceptable salt thereof, wherein R₁ is hydrogen.

3. A compound according to Claim 1 or Claim 2, or a pharmaceutically acceptable salt thereof, wherein R₂ is phenyl or a 5- or 6-membered heteroaryl group G₁ in which structure 1, 2, 3, or 4 ring members are hetero ring members independently selected from the group consisting of a nitrogen ring member, an oxygen ring member and a sulfur ring member, which group G₁ is optionally substituted by 1, 2, 3 or 4 substituents independently selected from the group, consisting of cyano, amino, aminocarbonyl, halogen, (C₁₋₄)alkyl, halogen-(C₁₄)alkyl, hydroxy, oxo, (C₁₋₄)alkoxy, halogen-(C₁₋₄)alkoxy, (C₁₋₄)alkylthio, halogen-(C₁₄)alkylthio, (C₁₋₄)alkoxy-(C₁₋₄)alkyl, (C₁₋₄)alkoxy-(C₁₋₄)alkoxy, (C₁₋₄)alkoxy-(C₁₋₄)alkylthio, (C₁₄)alkylthio-(C₁₋₄)alkyl, (C₁₋₄)alkylthio-(C₁₋₄)alkoxy, (C₁₋₄)alkylthio-(C₁₋₄)alkylthio, (C₂₋₄)alkenyl, (C₂₋₄)alkynyl, (C₂₋₄)alkenoxy, and (C₂₋₄)alkynoxy.

4. A compound according to any one of Claims 1 to 3, or a pharmaceutically acceptable salt thereof, wherein R₃ is hydrogen.

5. A compound according to any one of Claims 1 to 4, or a pharmaceutically acceptable salt thereof, wherein R₄ is hydrogen, or halogen; and R₅ is hydrogen, or halogen.

6. A compound according to any one of Claims 1 to 5, or a pharmaceutically acceptable salt thereof, wherein R₆ is (C₁₋₃)alkyl, or halogen-(C₁₋₃)alkyl.

7. A compound according to any one of Claims 1 to 6, or a pharmaceutically acceptable salt thereof, wherein R₇ is hydrogen, (C₁₋₆)alkyl, halogen-(C₁₋₆)alkyl, (C₁₋₄)alkoxy-(C₁₋₄)alkyl, (C₁₆)alkylcarbonyl, (C₁₋₆)alkoxycarbonyl, halogen-(C₁₋₆)alkoxycarbonyl, (C₁₋₆)alkoxy-(C₁₆)alkylcarbonyl, (C₃₋₆)cycloalkyl, (C₃₋₆)cycloalkyl-carbonyl, or a heteroaryl group optionally substituted by 1, 2, or 3 substituents independently selected from the group consisting of cyano, halogen, hydroxyl, (C₁₋₄)alkyl, halogen-(C₁₋₄)alkyl, (C₁₋₄)alkoxy, halogen-(C₁₋₄)alkoxy, (C₁₋₃)alkoxy-(C₁₋₃)alkyl and (C₁₋₃)alkoxy-(C₁₋₃)alkoxy.

8. A compound according to any one of Claims 1 to 7, or a pharmaceutically acceptable salt thereof, wherein E₁ is -C(R₈)(R₉)- and
either
each of R₈ and R₉ is independently selected from the group, consisting of hydrogen, cyano, halogen, (C₁₋₃)alkyl and halogen-(C₁₋₃)alkyl;
or
R₈ and R₉, taken together, are oxo or -CH₂-CH₂-.

9. A compound according to any one of Claims 1 to 8, or a pharmaceutically acceptable salt thereof, wherein E₂ is -C(R₁₂)(R₁₃)- and
either
each of R₁₂ and R₁₃ is independently selected from the group, consisting of hydrogen, cyano, halogen, (C₁₋₃)alkyl and halogen-(C₁₋₃)alkyl;
or
R₁₂ and R₁₃, taken together, are oxo or -CH₂-CH₂-.

10. A compound according to any one of Claims 1 to 9, or a pharmaceutically acceptable salt thereof, which is selected from the group consisting of:
5-Bromo-pyridine-2-carboxylic acid [3-(6-amino-2,4-dimethyl-3-oxo-2,3,4,5-tetrahydro-pyrazin-2-yl)-4-fluoro-phenyl]-amide;
5-Bromo-pyridine-2-carboxylic acid [3-(6-amino-2,4-dimethyl-2,3,4,5-tetrahydro-pyrazin-2-yl)-4-fluoro-phenyl]-amide;
5-Bromo-pyridine-2-carboxylic acid [3-(6-amino-2,4-dimethyl-3-oxo-2,3,4,5-tetrahydro-pyrazin-2-yl)-phenyl]-amide;
5-Bromo-pyridine-2-carboxylic acid [3-(6-amino-2,4-dimethyl-5-oxo-2,3,4,5-tetrahydro-pyrazin-2-yl)-4-fluoro-phenyl]-amide;
5-Chloro-pyridine-2-carboxylic acid [3-(6-amino-2,4-dimethyl-5-oxo-2,3,4,5-tetrahydro-pyrazin-2-yl)-4-fluoro-phenyl]-amide;
5-Bromo-pyridine-2-carboxylic acid [3-(6-amino-4-ethyl-2-methyl-5-oxo-2,3,4,5-tetrahydro-pyrazin-2-yl)-4-fluoro-phenyl]-amide;
3,5-Dichloro-pyridine-2-carboxylic acid [3-(6-amino-2,4-dimethyl-5-oxo-2,3,4,5-tetrahydro-pyrazin-2-yl)-4-fluoro-phenyl]-amide;
3,5-Dichloro-pyridine-2-carboxylic acid [3-(6-amino-4-ethyl-2-methyl-5-oxo-2,3,4,5-tetrahydro-pyrazin-2-yl)-4-fluoro-phenyl]-amide;
5-Bromo-pyridine-2-carboxylic acid [3-(6-amino-4-isopropyl-2-methyl-5-oxo-2,3,4,5-tetrahydro-pyrazin-2-yl)-4-fluoro-phenyl]-amide;
3,5-Dichloro-pyridine-2-carboxylic acid {3-[6-amino-4-(2-methoxy-ethyl)-2-methyl-5-oxo-2,3,4,5-tetrahydro-pyrazin-2-yl]-4-fluoro-phenyl}-amide;
5-Bromo-pyridine-2-carboxylic acid {3-[6-amino-4-(2-methoxy-ethyl)-2-methyl-5-oxo-2,3,4,5-tetrahydro-pyrazin-2-yl]-4-fluoro-phenyl}-amide;
5-Bromo-pyridine-2-carboxylic add {3-[6-amino-2-methyl-4-(1-methyl-1 H-pyrazol-4-yl)-5-oxo-2,3,4,5-tetrahydro-pyrazin-2-yl]-4-fluoro-phenyl}-amide;
5-Bromo-pyridine-2-carboxylic acid [3-(6-amino-2-methyl-5-oxo-4-pyridin-3-yl-2,3,4,5-tetrahydro-pyrazin-2-yl)-4-fluoro-phenyl]-amide;
5-Cyano-pyridine-2-carboxylic acid [3-(6-amino-4-ethyl-2-methyl-5-oxo-2,3,4,5-tetrahydro-pyrazin-2-yl)-4-fluoro-phenyl]-amide;
5-Bromo-pyridine-2-carboxylic acid [3-(6-amino-5-ethyl-2,4-dimethyl-3-oxo-2,3,4,5-tetrahydro-pyrazin-2-yl)-phenyl]-amide;
5-Bromo-pyridine-2-carboxylic acid [3-(6-amino-2-difluoromethyl-2,3,4,5-tetrahydro-pyrazin-2-yl)-4-fluoro-phenyl]-amide;
5-Cyano-pyridine-2-carboxylic acid [3-(6-amino-2-difluoromethyl-2,3,4,5-tetrahydro-pyrazin-2-yl)-4-fluoro-phenyl]-amide;
5-Cyano-pyridine-2-carboxylic acid [3-(6-amino-2-difluoromethyl-5-oxo-2,3,4,5-tetrahydro-pyrazin-2-yl)-4-fluoro-phenyl]-amide;
5-Amino-3-{5-[(5-bromo-pyridine-2-carbonyl)-amino]-2-fluoro-phenyl}-3-difluoromethyl-3,6-dihydro-2H-pyrazine-1-carboxylic acid methyl ester;
5-Amino-3-{5-[(5-cyano-pyridine-2-carbonyl)-amino]-2-fluoro-phenyl}-3-difluoromethyl-3,6-dihydro-2H-pyrazine-1-carboxylic add methyl ester;
5-Bromo-3-methyl-pyridine-2-carboxylic acid [3-(4-acetyl-6-amino-2-difluoromethyl-2,3,4,5-tetrahydro-pyrazin-2-yl)-4-fluoro-phenyl]-amide;
5-Amino-3-{5-[(5-bromo-3-methyl-pyridine-2-carbonyl)-amino]-2-fluoro-phenyl}-3-difluoromethyl-3,6-dihydro-2H-pyrazine-1-carboxylic acid 2,2-dichloro-ethyl ester;
5-Bromo-3-methyl-pyridine-2-carboxylic acid {3-[6-amino-2-difluoromethyl-4-(2-methoxy-acetyl)-2,3,4,5-tetrahydro-pyrazin-2-yl]-4-fluoro-phenyl}-amide;
5-Bromo-3-methyl-pyridine-2-carboxylic add [3-(6-amino-4-cydopropanecarbonyl-2-difluoromethyl-2,3,4,5-tetrahydro-pyrazin-2-yl)-4-fluoro-phenyl]-amide;
5-Bromo-3-methyl-pyridine-2-carboxylic acid [3-(6-amino-2-difluoromethyl-2,3,4,5-tetrahydro-pyrazin-2-yl)-4-fluoro-phenyl]-amide;
5-Difluoromethoxy-3-methyl-pyridine-2-carboxylic acid [3-(4-acetyl-6-amino-2-difluoromethyl-2,3,4,5-tetrahydro-pyrazin-2-yl)-4-fluoro-phenyl]-amide;
5-Difluoromethoxy-3-methyl-pyridine-2-carboxylic acid [3-(6-amino-2-difluoromethyl-2,3,4,5-tetrahydro-pyrazin-2-yl)-4-fluoro-phenyl]-amide;
3-Amino-5-methoxy-pyrazine-2-carboxylic acid [3-(6-amino-2-difluoromethyl-2,3,4,5-tetrahydro-pyrazin-2-yl)-4-fluoro-phenyl]-amide; and
3-Amino-5-oxo-4,5-dihydro-pyrazine-2-carboxylic acid [3-(6-amino-2-difluoromethyl-2,3,4,5-tetrahydro-pyrazin-2-yl)-4-fluoro-phenyl]-amide.

11. A compound according to any one of Claims 1 to 10, or a pharmaceutically acceptable salt thereof, for use as a medicament.

12. A compound according to any one of Claims 1 to 10, or a pharmaceutically acceptable salt thereof, for use in the treatment or prevention of Alzheimer's disease or mild cognitive impairment.

13. A pharmaceutical composition comprising a compound according to any one of Claims 1 to 10, or a pharmaceutically acceptable salt thereof, as active ingredient and a pharmaceutical carrier or diluent.

14. The use of a compound according to any one of Claims 1 to 10, or a pharmaceutically acceptable salt thereof, for the manufacture of a medicament for the treatment or prevention of Alzheimer's disease or mild cognitive impairment.

15. A combination comprising a therapeutically effective amount of a compound according to any one of Claims 1 to 10, or a pharmaceutically acceptable salt thereof, and a second drug substance, for simultaneous or sequential administration.

## Patentansprüche

1. Eine Verbindung der Formel (I), oder ein pharmazeutisch verträgliches Salz davon, 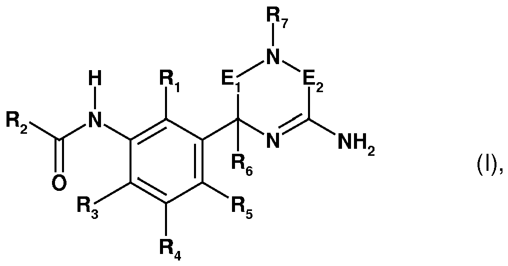 wobei gilt:
R₁ ist Wasserstoff, Cyano, Halogen, (C₁₋₈)Alkyl, Halogen-(C₁₋₈)alkyl, (C₁₋₈)Alkoxy, Halogen-(C₁₋₈)alkoxy, (C₁₋₈)Alkylthio, Halogen-(C₁₋₈)alkylthio, (C₁₋₈)Alkoxy-(C₁₋₈)alkyl, (C₁₋₈)Alkoxy-(C₁₋₈)alkoxy, (C₁₋₈)Alkoxy-(C₁₋₈)alkylthio, (C₁₋₈)Alkylthio-(C₁₋₈)alkyl, (C₁₋₈)Alkylthio-(C₁₋₈)alkoxy, (C₁₋₈)Alkylthio-(C₁-₈)alkylthio, (C₂₋₈)Alkenyl oder (C₂₋₈)Alkinyl;
R₂ ist eine Aryl-, Heteroaryl- oder nicht-aromatische Heterocyclyl-Gruppe G₁, wobei die Gruppe G₁ wahlweise durch 1 bis 4 Substituenten substituiert ist, unabhängig ausgewählt aus der Gruppe bestehend aus Cyano, Amino, Aminocarbonyl, Halogen, (C₁₋₈)Alkyl, Halogen-(C₁₋₈)alkyl, Hydroxy, Oxo, (C₁₋₈)Alkoxy, Halogen-(C₁₋₈)alkoxy, (C₁₋₈)Alkylthio, Halogen-(C₁₋₈)alkylthio, (C₁₋₈)Alkoxy-(C₁₋₈)alkyl, (C₁₋₈)Alkoxy-(C₁₋₈)alkoxy, (C₁₈)Alkoxy-(C₁₋₈)alkylthio, (C₁₋₈)Alkylthio-(C₁₋₈)alkyl, (C₁₋₈)Alkylthio-(C₁₋₈)alkoxy, (C₁₋₈)Alkylthio-(C₁₋₈)alkylthio, (C₂₋₈)Alkenyl, (C₂₋₈)Alkinyl, (C₂₋₈)Alkenoxy, (C₂₋₈)Alkinoxy und einer (C₃₋₈)Cycloalkyl-, Aryl-, Heteroaryl- oder nicht-aromatischen Heterocyclyl-Gruppe G₂, wobei die Gruppe G₂ wahlweise durch 1 bis 4 Substituenten substituiert ist, unabhängig ausgewählt aus der Gruppe bestehend aus Cyano, Aminocarbonyl, Halogen, (C₁₋₈)Alkyl, Halogen-(C₁₈)alkyl, Hydroxy, (C₁₋₈)Alkoxy, Halogen-(C₁₋₈)alkoxy, (C₁₋₈)Alkylthio, Halogen-(C₁₋₈)alkylthio, (C₁₋₈)Alkoxy-(C₁₋₈)alkyl, (C₁₋₈)Alkoxy-(C₁₋₈)alkoxy, (C₁₋₈)Alkoxy-(C₁₋₈)alkylthio, (C₁₋₈)Alkylthio-(C₁₋₈)alkyl, (C₁₋₈)Alkylthio-(C₁₋₈)alkoxy, (C₁₋₈)Alkylthio-(C₁₋₈)alkylthio, (C₂₋₈)Alkenyl und (C₂₋₈)Alkinyl;
R₃ ist Wasserstoff, Cyano, Halogen, (C₁₋₈)Alkyl, Halogen-(C₁₋₈)alkyl, (C₁₋₈)Alkoxy, Halogen-(C₁₋₈)alkoxy, (C₁₋₈)Alkylthio, Halogen-(C₁₋₈)alkylthio, (C₁₋₈)Alkoxy-(C₁₋₈)alkyl, (C₁₋₈)Alkoxy-(C₁₋₈)alkoxy, (C₁₋₈)Alkoxy-(C₁₋₈)alkylthio, (C₁₋₈)Alkylthio-(C₁₋₈)alkyl, (C₁₋₈)Alkylthio-(C₁₋₈)alkoxy, (C₁₋₈)Alkylthio-(C₁-₈)alkylthio, (C₂₋₈)Alkenyl oder (C₂₋₈)Alkinyl;
entweder
R₄ ist Wasserstoff, Cyano, Halogen, (C₁₋₈)Alkyl, Halogen-(C₁₋₈)alkyl, (C₁₋₈)Alkoxy, Halogen-(C₁₋₈)alkoxy, (C₁₋₈)Alkylthio, Halogen-(C₁₋₈)alkylthio, (C₁₋₈)Alkoxy-(C₁₋₈)alkyl, (C₁₋₈)Alkoxy-(C₁₋₈)alkoxy, (C₁₋₈)Alkoxy-(C₁₋₈)alkylthio, (C₁₋₈)Alkylthio-(C₁₋₈)alkyl, (C₁₋₈)Alkylthio-(C₁₋₈)alkoxy, (C₁₋₈)Alkylthio-(C₁₋₈)alkylthio, (C₂₋₈)Alkenyl oder (C₂₋₈)Alkinyl; und
R₅ ist Wasserstoff, Cyano, Halogen, (C₁₋₈)Alkyl, Halogen-(C₁₋₈)alkyl, (C₁₋₈)Alkoxy, Halogen-(C₁₋₈)alkoxy, (C₁₋₈)Alkylthio, Halogen-(C₁₋₈)alkylthio, (C₁₋₈)Alkoxy-(C₁₋₈)alkyl, (C₁₋₈)Alkoxy-(C₁₋₈)alkoxy, (C₁₋₈)Alkoxy-(C₁₋₈)alkylthio, (C₁₋₈)Alkylthio-(C₁₋₈)alkyl, (C₁₋₈)Alkylthio-(C₁₋₈)alkoxy, (C₁₋₈)Alkylthio-(C₁-₈)alkylthio, (C₂₋₈)Alkenyl oder (C₂₋₈)Alkinyl;
oder
R₄ und R₅ sind zusammen genommen -C(H)=C(H)-C(H)=C(H)- oder eine (C₁₋₈)Alkylen-Gruppe, wobei in der (C₁₋₈)Alkylen-Gruppe 1 oder 2 -CH₂-Ringelemente wahlweise ersetzt sind mit Hetero-Ringelementen, unabhängig ausgewählt aus der Gruppe bestehend aus -N(H)-, -N[(C₁₋₈)Alkyl]-, -O-, -S-, -S(=O)- oder -S(=O)₂-;
R₆ ist Wasserstoff, (C₁₋₈)Alkyl, Halogen-(C₁₋₈)alkyl, Hydroxy-(C₁₋₈)alkyl, (C₁₋₈)Alkoxy-(C₁₋₈)alkyl, Mercapto-(C₁₋₈)alkyl, (C₁₋₈)Alkylthio-(C₁₋₈)alkyl, Amino-(C₁₋₈)alkyl, N-(C₁₋₈)Alkylamino-(C₁₋₈)alkyl, N,N-Di-[(C₁₋₈)alkyl]amino-(C₁₋₈)alkyl mit zwei identischen oder verschiedenen (C₁₋₈)Alkyl-Resten im N,N-Di-[(C₁₋₈)alkyl]amino-Rest, (C₂₋₈)Alkenyl oder (C₂₋₈)Alkinyl;
R₇ ist Wasserstoff, (C₁₋₈)Alkyl, (C₁₋₈)Alkyl substituiert durch Halogen, (C₃₋₈)Cycloalkyl-(C₁₋₈)alkyl, (C₃₋₈)Cycloalkoxy-(C₁₋₈)alkyl, Aryloxy-(C₁₋₈)alkyl, (C₁₋₈)Alkoxy-(C₁₋₈)alkyl, (C₁₋₈)Alkylthio-(C₁₋₈)alkyl, (C₁₋₈)Alkylsulfinyl, (C₁₋₈)Alkylsulfinyl-(C₁₋₈)alkyl, (C₁₋₈)Alkylsulfonyl, (C₁₋₈)Alkylsulfonyl-(C₁₋₈)alkyl, Amino-(C₁₋₈)alkyl, (C₁₋₈)Alkylamino-(C₁₋₈)alkyl, Di(C₁₋₈)alkylamino-(C₁₋₈)alkyl mit zwei identischen oder verschiedenen (C₁₋₈)Alkyl-Resten im Di(C₁₋₈)alkylamino-Rest, Aminosulfonyl, (C₁₋₈)Alkylaminosulfonyl, Di(C₁₋₈)alkylaminosulfonyl mit zwei identischen oder verschiedenen (C₁₋₈)Alkyl-Resten, Formyl, (C₁₋₈)Alkylcarbonyl, Formyl-(C₁₋₈)alkyl, (C₁₋₈)Alkylcarbonyl-(C₁₋₈)alkyl, (C₁₋₈)Alkoxycarbonyl, Halogen-(C₁₋₈)alkoxycarbonyl, (C₁₋₈)Alkoxycarbonyl-(C₁₋₈)alkyl, (C₁₋₈)Alkoxy-(C₁₋₈)alkylcarbonyl oder eine (C₃₋₈)Cycloalkylcarbonyl-, Arylcarbonyl-, Aryl-(C₁₋₈)alkylcarbonyl-, Heteroarylcarbonyl-, Heteroaryl-(C₁₋₈)alkylcarbonyl-, nicht-aromatische Heterocyclyl-carbonyl-, (C₃₋₈)Cycloalkylsulfonyl-, Arylsulfonyl-, Aryl-(C₁₋₈)alkylsulfonyl-, Heteroarylsulfonyl-, Heteroaryl-(C₁₋₈)alkylsulfonyl-, nicht-aromatische Heterocyclylsulfonyl-, (C₃₋₈)Cycloalkyl-, Aryl-, Aryl-(C₁₋₈)alkyl-, Heteroaryl-, Heteroaryl-(C₁₋₈)alkyl- oder nicht-aromatische Heterocyclyl-Gruppe G₃, wobei die Gruppe G₃ wahlweise durch 1 bis 4 Substituenten substituiert ist, unabhängig ausgewählt aus der Gruppe bestehend aus Cyano, Aminocarbonyl, Halogen, (C₁₋₈)Alkyl, Halogen-(C₁₈)alkyl, Hydroxy, (C₁₋₈)Alkoxy, Halogen-(C₁₋₈)alkoxy, (C₁₋₈)Alkylthio, Halogen-(C₁₋₈)alkylthio, (C₁₋₈)Alkoxy-(C₁₋₈)alkyl, (C₁₋₈)Alkoxy-(C₁₋₈)alkoxy, (C₁₋₈)Alkoxy-(C₁₋₈)alkylthio, (C₁₋₈)Alkylthio-(C₁₋₈)alkyl, (C₁₋₈)Alkylthio-(C₁₋₈)alkoxy, (C₁₋₈)Alkylthio-(C₁₋₈)alkylthio, (C₂₋₈)Alkenyl, (C₂₋₈)Alkinyl und einer (C₃₋₈)Cycloalkyl-, Aryl-, Heteroaryl- oder nicht-aromatischen Heterocyclyl-Gruppe G₄, wobei die Gruppe G₄ wahlweise durch 1 bis 4 Substituenten substituiert ist, unabhängig ausgewählt aus der Gruppe bestehend aus Cyano, Aminocarbonyl, Halogen, (C₁₋₈)Alkyl, Halogen-(C₁₈)alkyl, Hydroxy, (C₁₋₈)Alkoxy, Halogen-(C₁₋₈)alkoxy, (C₁₋₈)Alkylthio, Halogen-(C₁-₈)alkylthio, (C₁₋₈)Alkoxy-(C₁₋₈)alkyl, (C₁₋₈)Alkoxy-(C₁₋₈)alkoxy, (C₁₈)Alkoxy-(C₁₋₈)alkylthio, (C₁₋₈)Alkylthio-(C₁₋₈)alkyl, (C₁₋₈)Alkylthio-(C₁₋₈)alkoxy, (C₁₋₈)Alkylthio-(C₁₋₈)alkylthio, (C₂₋₈)Alkenyl und (C₂₋₈)Alkinyl;
E₁ ist -C(R₈)(R₉)- oder -C(R₈)(R₉)-C(R₁₀)(R₁₁)-;
E₂ ist -C(R₁₂)(R₁₃)- oder -C(R₁₂)(R₁₃)-C(R₁₄)(R₁₅)-;
entweder
R₈ und R₉ sind jeweils unabhängig ausgewählt aus der Gruppe bestehend aus Wasserstoff, Cyano, Halogen, (C₁₋₈)Alkyl, Halogen-(C₁₋₈)alkyl, (C₁₋₈)Alkoxy-(C₁₋₈)alkyl und (C₁₋₈)Alkylthio-(C₁₋₈)alkyl;
oder
R₈ und R₉ sind zusammen genommen Oxo oder -CH₂-CH₂-; entweder
R₁₀ und R₁₁ sind jeweils unabhängig ausgewählt aus der Gruppe bestehend aus Wasserstoff, Cyano, Halogen, (C₁₋₈)Alkyl, Halogen-(C₁₋₈)alkyl, (C₁₋₈)Alkoxy-(C₁₋₈)alkyl und (C₁₋₈)Alkylthio-(C₁₋₈)alkyl;
oder
R₁₀ und R₁₁ sind zusammen genommen Oxo oder -CH₂-CH₂-; entweder
R₁₂ und R₁₃ sind jeweils unabhängig ausgewählt aus der Gruppe bestehend aus Wasserstoff, Cyano, Halogen, (C₁₋₈)Alkyl, Halogen-(C₁₋₈)alkyl, (C₁₋₈)Alkoxy-(C₁₋₈)alkyl und (C₁₋₈)Alkylthio-(C₁₋₈)alkyl;
oder
R₁₂ und R₁₃ sind zusammen genommen Oxo oder -CR₁₆R₁₇-CR₁₈R₁₉- , wobei R₁₆, R₁₇, R₁₈ und R₁₉ unabhängig ausgewählt sind aus Wasserstoff und Fluor; und
entweder
R₁₄ und R₁₅ sind jeweils unabhängig ausgewählt aus der Gruppe bestehend aus Wasserstoff, Cyano, Halogen, (C₁₋₈)Alkyl, Halogen-(C₁₋₈)alkyl, (C₁₋₈)Alkoxy-(C₁₋₈)alkyl und (C₁₋₈)Alkylthio-(C₁₋₈)alkyl;
oder
R₁₄ und R₁₅ sind zusammen genommen Oxo oder -CH₂-CH₂-.

2. Eine Verbindung nach Anspruch 1, oder ein pharmazeutisch verträgliches Salz davon, wobei R₁ Wasserstoff ist.

3. Eine Verbindung nach Anspruch 1 oder Anspruch 2, oder ein pharmazeutisch verträgliches Salz davon, wobei R₂ Phenyl oder eine 5- oder 6-gliedrige Heteroaryl-Gruppe G₁ ist, in deren Struktur 1, 2, 3 oder 4 Ringelemente Heteroringelemente sind, unabhängig ausgewählt aus der Gruppe bestehend aus einem Stickstoff-Ringelement, einem Sauerstoff-Ringelement und einem Schwefel-Ringelement, wobei die Gruppe G₁ wahlweise substituiert ist mit 1, 2, 3 oder 4 Substituenten, unabhängig ausgewählt aus der Gruppe bestehend aus Cyano, Amino, Aminocarbonyl, Halogen, (C₁₋₄)Alkyl, Halogen-(C₁₋₄)alkyl, Hydroxy, Oxo, (C₁₋₄)Alkoxy, Halogen-(C₁₋₄)alkoxy, (C₁₋₄)Alkylthio, Halogen-(C₁₋₄)alkylthio, (C₁₋₄)Alkoxy-(C₁₋₄)alkyl, (C₁₋₄)Alkoxy-(C₁₋₄)alkoxy, (C₁₋₄)Alkoxy-(C₁₋₄)alkylthio, (C₁₋₄)Alkylthio-(C₁₋₄)alkyl, (C₁₋₄)Alkylthio-(C₁₋₄)alkoxy, (C₁₋₄)Alkylthio-(C₁₋₄)alkylthio, (C₂₋₄)Alkenyl, (C₂₋₄)Alkinyl, (C₂₋₄)Alkenoxy und (C₂₋₄)Alkinoxy.

4. Eine Verbindung nach einem der Ansprüche 1 bis 3, oder ein pharmazeutisch verträgliches Salz davon, wobei R₃ Wasserstoff ist.

5. Eine Verbindung nach einem der Ansprüche 1 bis 4, oder ein pharmazeutisch verträgliches Salz davon, wobei R₄ Wasserstoff oder Halogen ist; und R₅ Wasserstoff oder Halogen ist.

6. Eine Verbindung nach einem der Ansprüche 1 bis 5, oder ein pharmazeutisch verträgliches Salz davon, wobei R₆ (C₁₋₃)Alkyl oder Halogen-(C₁₋₃)alkyl ist.

7. Eine Verbindung nach einem der Ansprüche 1 bis 6, oder ein pharmazeutisch verträgliches Salz davon, wobei R₇ Wasserstoff, (C₁₋₆)Alkyl, Halogen-(C₁₋₆)alkyl, (C₁₋₄)Alkoxy-(C₁₋₄)alkyl, (C₁₋₆)Alkylcarbonyl, (C₁₋₆)Alkoxycarbonyl, Halogen-(C₁₋₆)alkoxycarbonyl, (C₁₋₆)Alkoxy-(C₁₋₆)alkylcarbonyl, (C₃₋₆)Cycloalkyl, (C₃₋₆)Cycloalkylcarbonyl oder eine Heteroaryl-Gruppe ist, wahlweise durch 1, 2 oder 3 Substituenten substituiert, unabhängig ausgewählt aus der Gruppe bestehend aus Cyano, Halogen, Hydroxyl, (C₁₋₄)Alkyl, Halogen-(C₁₋₄)alkyl, (C₁₋₄)Alkoxy, Halogen-(C₁₋₄)alkoxy, (C₁₋₃)Alkoxy-(C₁₋₃)alkyl und (C₁₋₃)Alkoxy-(C₁₋₃)alkoxy.

8. Eine Verbindung nach einem der Ansprüche 1 bis 7, oder ein pharmazeutisch verträgliches Salz davon, wobei E₁ -C(R₈)(R₉)- ist und entweder
R₈ und R₉ jeweils unabhängig ausgewählt sind aus der Gruppe bestehend aus Wasserstoff, Cyano, Halogen, (C₁₋₃)Alkyl und Halogen-(C₁₋₃)alkyl;
oder
R₈ und R₉ zusammen genommen Oxo oder -CH₂-CH₂- sind.

9. Eine Verbindung nach einem der Ansprüche 1 bis 8, oder ein pharmazeutisch verträgliches Salz davon, wobei E₂ -C(R₁₂)(R₁₃)- ist und entweder
R₁₂ und R₁₃ jeweils unabhängig ausgewählt sind aus der Gruppe bestehend aus Wasserstoff, Cyano, Halogen, (C₁₋₃)Alkyl und Halogen-(C₁₋₃)alkyl;
oder
R₁₂ und R₁₃ zusammen genommen Oxo oder -CH₂-CH₂- sind.

10. Eine Verbindung nach einem der Ansprüche 1 bis 9, oder ein pharmazeutisch verträgliches Salz davon, die ausgewählt ist aus der Gruppe bestehend aus:
5-Brom-pyridin-2-carbonsäure-[3-(6-amino-2,4-dimethyl-3-oxo-2,3,4,5-tetrahydro-pyrazin-2-yl)-4-fluor-phenyl]-amid;
5-Brom-pyridin-2-carbonsäure-[3-(6-amino-2,4-dimethyl-2,3,4,5-tetrahydro-pyrazin-2-yl)-4-fluor-phenyl]-amid;
5-Brom-pyridin-2-carbonsäure-[3-(6-amino-2,4-dimethyl-3-oxo-2,3,4,5-tetrahydro-pyrazin-2-yl)-phenyl]-amid;
5-Brom-pyridin-2-carbonsäure-[3-(6-amino-2,4-dimethyl-5-oxo-2,3,4,5-tetrahydro-pyrazin-2-yl)-4-fluor-phenyl]-amid;
5-Chlor-pyridin-2-carbonsäure-[3-(6-amino-2,4-dimethyl-5-oxo-2,3,4,5-tetrahydro-pyrazin-2-yl)-4-fluor-phenyl]-amid;
5-Brom-pyridin-2-carbonsäure-[3-(6-amino-4-ethyl-2-methyl-5-oxo-2,3,4,5-tetrahydro-pyrazin-2-yl)-4-fluor-phenyl]-amid;
3,5-Dichlor-pyridin-2-carbonsäure-[3-(6-amino-2,4-dimethyl-5-oxo-2,3,4,5-tetrahydro-pyrazin-2-yl)-4-fluor-phenyl]-amid;
3,5-Dichlor-pyridin-2-carbonsäure-[3-(6-amino-4-ethyl-2-methyl-5-oxo-2,3,4,5-tetrahydro-pyrazin-2-yl)-4-fluor-phenyl]-amid;
5-Brom-pyridin-2-carbonsäure-[3-(6-amino-4-isopropyl-2-methyl-5-oxo-2,3,4,5-tetrahydro-pyrazin-2-yl)-4-fluor-phenyl]-amid;
3,5-Dichlor-pyridin-2-carbonsäure-{3-[6-amino-4-(2-methoxy-ethyl)-2-methyl-5-oxo-2,3,4,5-tetrahydro-pyrazin-2-yl]-4-fluor-phenyl}-amid;
5-Brom-pyridin-2-carbonsäure-{3-[6-amino-4-(2-methoxy-ethyl)-2-methyl-5-oxo-2,3,4,5-tetrahydro-pyrazin-2-yl]-4-fluor-phenyl}-amid;
5-Brom-pyridin-2-carbonsäure-{3-[6-amino-2-methyl-4-(1-methyl-1H-pyrazol-4-yl)-5-oxo-2,3,4,5-tetrahydro-pyrazin-2-yl]-4-fluor-phenyl}-amid;
5-Brom-pyridin-2-carbonsäure-[3-(6-amino-2-methyl-5-oxo-4-pyridin-3-yl-2,3,4,5-tetrahydro-pyrazin-2-yl)-4-fluor-phenyl]-amid;
5-Cyano-pyridin-2-carbonsäure-[3-(6-amino-4-ethyl-2-methyl-5-oxo-2,3,4,5-tetrahydro-pyrazin-2-yl)-4-fluor-phenyl]-amid;
5-Brom-pyridin-2-carbonsäure-[3-(6-amino-5-ethyl-2,4-dimethyl-3-oxo-2,3,4,5-tetrahydro-pyrazin-2-yl)-phenyl]-amid;
5-Brom-pyridin-2-carbonsäure-[3-(6-amino-2-difluormethyl-2,3,4,5-tetrahydro-pyrazin-2-yl)-4-fluor-phenyl]-amid;
5-Cyano-pyridin-2-carbonsäure-[3-(6-amino-2-difluormethyl-2,3,4,5-tetrahydro-pyrazin-2-yl)-4-fluor-phenyl]-amid;
5-Cyano-pyridin-2-carbonsäure-[3-(6-amino-2-difluormethyl-5-oxo-2,3,4,5-tetrahydro-pyrazin-2-yl)-4-fluor-phenyl]-amid;
5-Amino-3-{5-[(5-brom-pyridin-2-carbonyl)-amino]-2-fluor-phenyl}-3-difluormethyl-3,6-dihydro-2H-pyrazin-1-carbonsäure-methylester;
5-Amino-3-{5-[(5-cyano-pyridin-2-carbonyl)-amino]-2-fluor-phenyl}-3-difluormethyl-3,6-dihydro-2H-pyrazin-1-carbonsäure-methylester;
5-Brom-3-methyl-pyridin-2-carbonsäure-[3-(4-acetyl-6-amino-2-difluormethyl-2,3,4,5-tetrahydro-pyrazin-2-yl)-4-fluor-phenyl]-amid;
5-Amino-3-{5-[(5-brom-3-methyl-pyridin-2-carbonyl)-amino]-2-fluor-phenyl}-3-difluormethyl-3,6-dihydro-2H-pyrazin-1-carbonsäure-2,2-dichlor-ethylester;
5-Brom-3-methyl-pyridin-2-carbonsäure-{3-[6-amino-2-difluormethyl-4-(2-methoxy-acetyl)-2,3,4,5-tetrahydro-pyrazin-2-yl]-4-fluor-phenyl}-amid;
5-Brom-3-methyl-pyridin-2-carbonsäure-[3-(6-amino-4-cyclopropancarbonyl-2-difluormethyl-2,3,4,5-tetrahydro-pyrazin-2-yl)-4-fluor-phenyl]-amid;
5-Brom-3-methyl-pyridin-2-carbonsäure-[3-(6-amino-2-difluormethyl-2,3,4,5-tetrahydro-pyrazin-2-yl)-4-fluor-phenyl]-amid;
5-Difluormethoxy-3-methyl-pyridin-2-carbonsäure-[3-(4-acetyl-6-amino-2-difluormethyl-2,3,4,5-tetrahydro-pyrazin-2-yl)-4-fluor-phenyl]-amid;
5-Difluormethoxy-3-methyl-pyridin-2-carbonsäure-[3-(6-amino-2-difluormethyl-2,3,4,5-tetrahydro-pyrazin-2-yl)-4-fluor-phenyl]-amid;
3-Amino-5-methoxy-pyrazin-2-carbonsäure-[3-(6-amino-2-difluormethyl-2,3,4,5-tetrahydro-pyrazin-2-yl)-4-fluor-phenyl]-amid; und
3-Amino-5-oxo-4,5-dihydro-pyrazin-2-carbonsäure-[3-(6-amino-2-difluormethyl-2,3,4,5-tetrahydro-pyrazin-2-yl)-4-fluor-phenyl]-amid.

11. Eine Verbindung nach einem der Ansprüche 1 bis 10, oder ein pharmazeutisch verträgliches Salz davon, zur Verwendung als Medikament.

12. Eine Verbindung nach einem der Ansprüche 1 bis 10, oder ein pharmazeutisch verträgliches Salz davon, zur Verwendung bei der Behandlung oder Prävention von Alzheimer-Krankheit oder leichter kognitiver Beeinträchtigung.

13. Eine pharmazeutische Zusammensetzung, umfassend eine Verbindung nach einem der Ansprüche 1 bis 10, oder ein pharmazeutisch verträgliches Salz davon, als Wirkstoff und einen pharmazeutischen Träger oder Verdünner.

14. Die Verwendung einer Verbindung nach einem der Ansprüche 1 bis 10, oder eines pharmazeutisch verträglichen Salzes davon, zur Herstellung eines Medikaments zur Behandlung oder Prävention von Alzheimer-Krankheit oder leichter kognitiver Beeinträchtigung.

15. Eine Kombination, umfassend eine therapeutisch wirksame Menge einer Verbindung nach einem der Ansprüche 1 bis 10, oder eines pharmazeutisch verträglichen Salzes davon, und einen zweiten Wirkstoff zur gleichzeitigen oder sequenziellen Verabreichung.

## Revendications

1. Composé de formule (I), ou sel pharmaceutiquement acceptable de celui-ci : 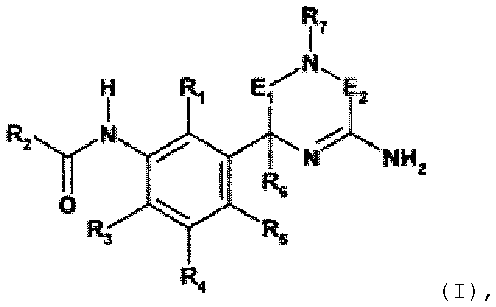 formule dans laquelle
- R₁ représente hydrogène, cyano, halogène, alkyle en C₁-C₈, halogéno-alkyle en C₁-C₈, alcoxy en C₁-C₈, halogéno-alcoxy en C₁-C₈, alkylthio en C₁-C₈, halogéno-alkylthio en C₁-C₈, alcoxy en C₁-C₈-alkyle en C₁-C₈, alcoxy en C₁-C₈-alcoxy en C₁-C₈, alcoxy en C₁-C₈-alkylthio en C₁-C₈, alkylthio en C₁-C₈-alkyle en C₁-C₈, alkylthio en C₁-C₈-alcoxy en C₁-C₈, alkylthio en C₁-C₈-alkylthio en C₁-C₈, alcényle en C₂-C₈ ou alcynyle en C₂-C₈;
- R₂ représente un groupe aryle, hétéroaryle ou hétérocyclyle non aromatique G₁, lequel groupe G₁ est facultativement substitué par 1 à 4 substituants indépendamment choisis dans le groupe consistant en cyano, amino, aminocarbonyle, halogène, alkyle en C₁-C₈, halogéno-alkyle en C₁-C₈, hydroxy, oxo, alcoxy en C₁-C₈, halogéno-alcoxy en C₁-C₈, alkylthio en C₁-C₈, halogéno-alkylthio en C₁-C₈, alcoxy en C₁-C₈-alkyle en C₁-C₈, alcoxy en C₁-C₈-alcoxy en C₁-C₈, alcoxy en C₁-C₈-alkylthio en C₁-C₈, alkylthio en C₁-C₈-alkyle en C₁-C₈, alkylthio en C₁-C₈-alcoxy en C₁-C₈, alkylthio en C₁-C₈-alkylthio en C₁-C₈, alcényle en C₂-C₈, alcynyle en C₂-C₈, alcénoxy en C₂-C₈, alcynoxy en C₂-C₈ et un groupe cycloalkyle en C₃-C₈, aryle, hétéroaryle ou hétérocyclyle non aromatique G₂, lequel groupe G₂ est facultativement substitué par 1 à 4 substituants indépendamment choisis dans le groupe consistant en cyano, aminocarbonyle, halogène, alkyle en C₁-C₈, halogéno-alkyle en C₁-C₈, hydroxy, alcoxy en C₁-C₈, halogéno-alcoxy en C₁-C₈, alkylthio en C₁-C₈, halogéno-alkylthio en C₁-C₈, alcoxy en C₁-C₈-alkyle en C₁-C₈, alcoxy en C₁-C₈-alcoxy en C₁-C₈, alcoxy en C₁-C₈-alkylthio en C₁-C₈, alkylthio en C₁-C₈-alkyle en C₁-C₈, alkylthio en C₁-C₈-alcoxy en C₁-C₈, alkylthio en C₁-C₈-alkylthio en C₁-C₈, alcényle en C₂-C₈ et alcynyle en C₂-C₈;
- R₃ représente hydrogène, cyano, halogène, alkyle en C₁-C₈, halogéno-alkyle en C₁-C₈, alcoxy en C₁-C₈, halogéno-alcoxy en C₁-C₈, alkylthio en C₁-C₈, halogéno-alkylthio en C₁-C₈, alcoxy en C₁-C₈-alkyle en C₁-C₈, alcoxy en C₁-C₈-alcoxy en C₁-C₈, alcoxy en C₁-C₈-alkylthio en C₁-C₈, alkylthio en C₁-C₈-alkyle en C₁-C₈, alkylthio en C₁-C₈-alcoxy en C₁-C₈, alkylthio en C₁-C₈-alkylthio en C₁-C₈, alcényle en C₂-C₈ ou alcynyle en C₂-C₈;
ou
- R₄ représente hydrogène, cyano, halogène, alkyle en C₁-C₈, halogéno-alkyle en C₁-C₈, alcoxy en C₁-C₈, halogéno-alcoxy en C₁-C₈, alkylthio en C₁-C₈, halogéno-alkylthio en C₁-C₈, alcoxy en C₁-C₈-alkyle en C₁-C₈, alcoxy en C₁-C₈-alcoxy en C₁-C₈, alcoxy en C₁-C₈-alkylthio en C₁-C₈, alkylthio en C₁-C₈-alkyle en C₁-C₈, alkylthio en C₁-C₈-alcoxy en C₁-C₈, alkylthio en C₁-C₈-alkylthio en C₁-C₈, alcényle en C₂-C₈ ou alcynyle en C₂-C₈; et
- R₅ représente hydrogène, cyano, halogène, alkyle en C₁-C₈, halogéno-alkyle en C₁-C₈, alcoxy en C₁-C₈, halogéno-alcoxy en C₁-C₈, alkylthio en C₁-C₈, halogéno-alkylthio en C₁-C₈, alcoxy en C₁-C₈-alkyle en C₁-C₈, alcoxy en C₁-C₈-alcoxy en C₁-C₈, alcoxy en C₁-C₈-alkylthio en C₁-C₈, alkylthio en C₁-C₈-alkyle en C₁-C₈, alkylthio en C₁-C₈-alcoxy en C₁-C₈, alkylthio en C₁-C₈-alkylthio en C₁-C₈, alcényle en C₂-C₈; ou alcynyle en C₂-C₈;
ou
- R₄ et R₅, pris conjointement, représentent -C(H)=C(H)-C(H)=C(H)- ou un groupe alkylène en C₁-C₈, dans lequel groupe alkylène en C₁-C₈, 1 ou 2 chaînons de cycle -CH₂- sont facultativement remplacés par des chaînons d'hétérocycle indépendamment choisis dans le groupe consistant en -N(H)-, -N[alkyl en C₁-C₈]-, -O-, -S-, -S(=O)- ou -S(=O)₂- ;
- R₆ représente hydrogène, alkyle en C₁-C₈, halogéno-alkyle en C₁-C₈, hydroxy-alkyle en C₁-C₈, alcoxy en C₁-C₈-alkyle en C₁-C₈, mercapto-alkyle en C₁-C₈, alkylthio en C₁-C₈-alkyle en C₁-C₈, amino-alkyle en C₁-C₈, N-alkylamino en C₁-C₈-alkyle en C₁-C₈, N,N-di-[alkyl en C₁-C₈]amino-alkyle en C₁-C₈ avec deux fractions alkyle en C₁-C₈ identiques ou différentes dans la fraction N,N-di-[alkyl en C₁-C₈]amino, alcényle en C₂-C₈ ou alcynyle en C₂-C₈;
- R₇ représente hydrogène, alkyle en C₁-C₈, alkyle en C₁-C₈ substitué par halogène, cycloalkyl en C₃-C₈-alkyle en C₁-C₈, cycloalcoxy en C₃-C₈-alkyle en C₁-C₈, aryloxy-alkyle en C₁-C₈, alcoxy en C₁-C₈-alkyle en C₁-C₈, alkylthio en C₁-C₈-alkyle en C₁-C₈, alkyl en C₁-C₈-sulfinyle, (alkyl en C₁-C₈)-sulfinyl-alkyle en C₁-C₈, alkyl en C₁-C₈-sulfonyle, (alkyl en C₁-C₈)-sulfonyl-alkyle en C₁-C₈, amino-alkyle en C₁-C₈, alkyl en C₁-C₈-amino-alkyle en C₁-C₈, di(alkyl en C₁-C₈)-amino-alkyle en C₁-C₈ ayant deux fractions alkyle en C₁-C₈ identiques ou différentes dans la fraction di(alkyl en C₁-C₈)-amino, aminosulfonyle, alkyl en C₁-C₈-aminosulfonyle, di(alkyl en C₁-C₈-aminosulfonyle) ayant deux fractions alkyle en C₁-C₈ identiques ou différentes, formyle, alkyl en C₁-C₈-carbonyle, formyl-alkyle en C₁-C₈, alkyl en C₁-C₈-carbonyl-alkyle en C₁-C₈, alcoxy en C₁-C₈-carbonyle, halogéno-alcoxy en C₁-C₈-carbonyle, alcoxy en C₁-C₈-carbonyl-alkyle en C₁-C₈, alcoxy en C₁-C₈-alkyl en C₁-C₈-carbonyle ou un groupe cycloalkyl en C₁-C₈-carbonyle, arylcarbonyle, aryl-alkyl en C₁-C₈-carbonyle, hétéroarylcarbonyle, hétéroaryl-alkyl en C₁-C₈-carbonyle, hétérocyclyl non aromatique-carbonyle, cycloalkyl en C₁-C₈-sulfonyle, arylsulfonyle, aryl-alkyl en C₁-C₈-sulfonyle, hétéroarylsulfonyle, hétéroaryl-alkyl en C₁-C₈-sulfonyle, hétérocyclyl non aromatique-sulfonyle, cycloalkyle en C₃-C₈, aryle, aryl-alkyle en C₁-C₈, hétéroaryle, hétéroaryl-alkyle en C₁-C₈ ou hétérocyclyle non aromatique G₃, lequel groupe G₃ est facultativement substitué par 1 à 4 substituants indépendamment choisis dans le groupe consistant en cyano, aminocarbonyle, halogène, alkyle en C₁-C₈, halogéno-alkyle en C₁-C₈, hydroxy, alcoxy en C₁-C₈, halogéno-alcoxy en C₁-C₈, alkylthio en C₁-C₈, halogéno-alkylthio en C₁-C₈, alcoxy en C₁-C₈-alkyle en C₁-C₈, alcoxy en C₁-C₈-alcoxy en C₁-C₈, alcoxy en C₁-C₈-alkylthio en C₁-C₈, alkylthio en C₁-C₈-alkyle en C₁-C₈, alkylthio en C₁-C₈-alcoxy en C₁-C₈, alkylthio en C₁-C₈-alkylthio en C₁-C₈, alcényle en C₂-C₈, alcynyle en C₂-C₈ et un groupe cycloalkyle en C₃-C₈, aryle, hétéroaryle ou hétérocyclyle non aromatique G₄, lequel groupe G₄ est facultativement substitué par 1 à 4 substituants indépendamment choisis dans le groupe consistant en cyano, aminocarbonyle, halogène, alkyle en C₁-C₈, halogéno-alkyle en C₁-C₈, hydroxy, alcoxy en C₁-C₈, halogéno-alcoxy en C₁-C₈, alkylthio en C₁-C₈, halogéno-alkylthio en C₁-C₈, alcoxy en C₁-C₈-alkyle en C₁-C₈, alcoxy en C₁-C₈-alcoxy en C₁-C₈, alcoxy en C₁-C₈-alkylthio en C₁-C₈, alkylthio en C₁-C₈-alkyle en C₁-C₈, alkylthio en C₁-C₈-alcoxy en C₁-C₈, alkylthio en C₁-C₈-alkylthio en C₁-C₈, alcényle en C₂-C₈ et alcynyle en C₂-C₈;
- E₁ représente -C(R₈)(R₉)- ou -C(R₈)(R₉)-C(R₁₀)(R₁₁)- ;
- E₂ représente -C(R₁₂)(R₁₃)- ou -C(R₁₂)(R₁₃)-C(R₁₄)(R₁₅)-;
ou
- chacun parmi R₈ et R₉ est indépendamment choisi dans le groupe consistant en hydrogène, cyano, halogène, alkyle en C₁-C₈, halogéno-alkyle en C₁-C₈, alcoxy en C₁-C₈-alkyle en C₁-C₈ et alkylthio en C₁-C₈-alkyle en C₁-C₈ ;
ou
- R₈ et R₉, pris conjointement, représentent oxo ou -CH₂-CH₂- ;
ou
- chacun parmi R₁₀ et R₁₁ est indépendamment choisi dans le groupe consistant en hydrogène, cyano, halogène, alkyle en C₁-C₈, halogéno-alkyle en C₁-C₈, alcoxy en C₁-C₈-alkyle en C₁-C₈ et alkylthio en C₁-C₈-alkyle en C₁-C₈ ;
ou
- R₁₀ et R₁₁, pris conjointement, représentent oxo ou -CH₂-CH₂- ;
ou
- chacun parmi R₁₂ et R₁₃ est indépendamment choisi dans le groupe consistant en hydrogène, cyano, halogène, alkyle en C₁-C₈, halogéno-alkyle en C₁-C₈, alcoxy en C₁-C₈-alkyle en C₁-C₈ et alkylthio en C₁-C₈-alkyle en C₁-C₈ ;
ou
- R₁₂ et R₁₃, pris conjointement, représentent oxo ou -CR₁₆R₁₇-CR₁₈R₁₉-R₁₆, R₁₇, R₁₈ et R₁₉ étant indépendamment choisis parmi hydrogène et fluoro ; et
ou
- chacun parmi R₁₄ et R₁₅ est indépendamment choisi dans le groupe consistant en hydrogène, cyano, halogène, alkyle en C₁-C₈, halogéno-alkyle en C₁-C₈, alcoxy en C₁-C₈-alkyle en C₁-C₈ et alkylthio en C₁-C₈-alkyle en C₁-C₈ ; ou
- R₁₄ et R₁₅, pris conjointement, représentent oxo ou -CH₂-CH₂-.

2. Composé selon la revendication 1, ou sel pharmaceutiquement acceptable de celui-ci, dans lequel R₁ représente hydrogène.

3. Composé selon la revendication 1 ou la revendication 2, ou sel pharmaceutiquement acceptable de celui-ci, dans lequel R₂ représente phényle ou un groupe hétéroaryle à 5 ou 6 chaînons G₁ dans laquelle structure 1, 2, 3 ou 4 chaînons de cycle sont des chaînons d'hétérocycle indépendamment choisis dans le groupe consistant en un chaînon de cycle azote, un chaînon de cycle oxygène et un chainon de cycle soufre, lequel groupe G₁ est facultativement substitué par 1, 2, 3 ou 4 substituants indépendamment choisis dans le groupe consistant en cyano, amino, aminocarbonyle, halogène, alkyle en C₁-C₄, halogéno-alkyle en C₁-C₄, hydroxy, oxo, alcoxy en C₁-C₄, halogéno-alcoxy en C₁-C₄, alkylthio en C₁-C₄, halogéno-alkylthio en C₁-C₄, alcoxy en C₁-C₄-alkyle en C₁-C₄, alcoxy en C₁-C₄-alcoxy en C₁-C₄, alcoxy en C₁-C₄-alkylthio en C₁-C₄, alkylthio en C₁-C₄-alkyle en C₁-C₄, alkylthio en C₁-C₄-alcoxy en C₁-C₄, alkylthio en C₁-C₄-alkylthio en C₁-C₄, alcényle en C₂-C₄, alcynyle en C₂-C₄, alcénoxy en C₂-C₄ et alcynoxy en C₂-C₄.

4. Composé selon l'une quelconque des revendications 1 à 3, ou sel pharmaceutiquement acceptable de celui-ci, dans lequel R₃ représente hydrogène.

5. Composé selon l'une quelconque des revendications 1 à 4, ou sel pharmaceutiquement acceptable de celui-ci, dans lequel R₄ représente hydrogène ou halogène ; et R₅ représente hydrogène ou halogène.

6. Composé selon l'une quelconque des revendications 1 à 5, ou sel pharmaceutiquement acceptable de celui-ci, dans lequel R₆ représente alkyle en C₁-C₃ ou halogéno-alkyle en C₁-C₃.

7. Composé selon l'une quelconque des revendications 1 à 6, ou sel pharmaceutiquement acceptable de celui-ci, dans lequel R₇ représente hydrogène, alkyle en C₁-C₆, halogéno-alkyle en C₁-C₆, alcoxy en C₁-C₄-alkyle en C₁-C₄, alkyl en C₁-C₆-carbonyle, alcoxy en C₁-C₆-carbonyle, halogéno-alcoxy en C₁-C₆-carbonyle, alcoxy en C₁-C₆-alkyl en C₁-C₆-carbonyle, cycloalkyle en C₃-C₆, cycloalkyl en C₃-C₆-carbonyle, ou un groupe hétéroaryle facultativement substitué par 1, 2 ou 3 substituants indépendamment choisis dans le groupe consistant en cyano, halogène, hydroxyle, alkyle en C₁-C₄, halogéno-alkyle en C₁-C₄, alcoxy en C₁-C₄, halogéno-alcoxy en C₁-C₄, alcoxy en C₁-C₃-alkyle en C₁-C₃ et alcoxy en C₁-C₃-alcoxy en C₁-C₃.

8. Composé selon l'une quelconque des revendications 1 à 7, ou sel pharmaceutiquement acceptable de celui-ci, dans lequel E₁ représente -C(R₈)(R₉)- et
ou
- chacun parmi R₈ et R₉ est indépendamment choisi dans le groupe consistant en hydrogène, cyano, halogène, alkyle en C₁-C₃ et halogéno-alkyle en C₁-C₃ ;
ou
- R₈ et R₉, pris conjointement, représentent oxo ou -CH₂-CH₂-.

9. Composé selon l'une quelconque des revendications 1 à 8, ou sel pharmaceutiquement acceptable de celui-ci, dans lequel E₂ représente -C(R₁₂)(R₁₃₎- et
ou
- chacun parmi R₁₂ et R₁₃ est indépendamment choisi dans le groupe, consistant en hydrogène, cyano, halogène, alkyle en C₁-C₃ et halogéno-alkyle en C₁-C₃ ;
ou
- R₁₂ et R₁₃, pris conjointement, représentent oxo ou -CH₂-CH₂-.

10. Composé selon l'une quelconque des revendications 1 à 9, ou sel pharmaceutiquement acceptable de celui-ci, qui est choisi dans le groupe consistant en :
• le [3-(6-amino-2,4-diméthyl-3-oxo-2,3,4,5-tétrahydro-pyrazin-2-yl)-4-fluoro-phényl]-amide de l'acide 5-bromo-pyridine-2-carboxylique ;
• le [3-(6-amino-2,4-diméthyl-2,3,4,5-tétrahydro-pyrazin-2-yl)-4-fluoro-phényl]-amide de l'acide 5-bromo-pyridine-2-carboxylique ;
• le [3-(6-amino-2,4-diméthyl-3-oxo-2,3,4,5-tétrahydro-pyrazin-2-yl)-phényl]-amide de l'acide 5-bromo-pyridine-2-carboxylique ;
• le [3-(6-amino-2,4-diméthyl-5-oxo-2,3,4,5-tétrahydro-pyrazin-2-yl)-4-fluoro-phényl]-amide de l'acide 5-bromo-pyridine-2-carboxylique ;
• le [3-(6-amino-2,4-diméthyl-5-oxo-2,3,4,5-tétrahydro-pyrazin-2-yl)-4-fluoro-phényl]-amide de l'acide 5-chloro-pyridine-2-carboxylique ;
• le [3-(6-amino-4-éthyl-2-méthyl-5-oxo-2,3,4,5-tétrahydro-pyrazin-2-yl)-4-fluoro-phényl]-amide de l'acide 5-bromo-pyridine-2-carboxylique ;
• le [3-(6-amino-2,4-diméthyl-5-oxo-2,3,4,5-tétrahydro-pyrazin-2-yl)-4-fluoro-phényl]-amide de l'acide 3,5-dichloro-pyridine-2-carboxylique ;
• le [3-(6-amino-4-éthyl-2-méthyl-5-oxo-2,3,4,5-tétrahydro-pyrazin-2-yl)-4-fluoro-phényl]-amide de l'acide 3,5-dichloro-pyridine-2-carboxylique ;
• le [3-(6-amino-4-isopropyl-2-méthyl-5-oxo-2,3,4,5-tétrahydro-pyrazin-2-yl)-4-fluoro-phényl]-amide de l'acide 5-bromo-pyridine-2-carboxylique ;
• le {3-[6-amino-4-(2-méthoxy-éthyl)-2-méthyl-5-oxo-2,3,4,5-tétrahydro-pyrazin-2-yl]-4-fluoro-phényl}-amide de l'acide 3,5-dichloro-pyridine-2-carboxylique ;
• le {3-[6-amino-4-(2-méthoxy-éthyl)-2-méthyl-5-oxo-2,3,4,5-tétrahydro-pyrazin-2-yl]-4-fluoro-phényl}-amide de l'acide 5-bromo-pyridine-2-carboxylique ;
• le {3-[6-amino-2-méthyl-4-(1-méthyl-1H-pyrazol-4-yl)-5-oxo-2,3,4,5-tétrahydro-pyrazin-2-yl]-4-fluoro-phényl}-amide de l'acide 5-bromo-pyridine-2-carboxylique ;
• le [3-(6-amino-2-méthyl-5-oxo-4-pyridin-3-yl-2,3,4,5-tétrahydro-pyrazin-2-yl)-4-fluoro-phényl]-amide de l'acide 5-bromo-pyridine-2-carboxylique ;
• le [3-(6-amino-4-éthyl-2-méthyl-5-oxo-2,3,4,5-tétrahydro-pyrazin-2-yl)-4-fluoro-phényl]-amide de l'acide 5-cyano-pyridine-2-carboxylique ;
• le [3-(6-amino-5-éthyl-2,4-diméthyl-3-oxo-2,3,4,5-tétrahydro-pyrazin-2-yl)-phényl]-amide de l'acide 5-bromo-pyridine-2-carboxylique ;
• le [3-(6-amino-2-difluorométhyl-2,3,4,5-tétrahydro-pyrazin-2-yl)-4-fluoro-phényl]-amide de l'acide 5-bromo-pyridine-2-carboxylique ;
• le [3-(6-amino-2-difluorométhyl-2,3,4,5-tétrahydro-pyrazin-2-yl)-4-fluoro-phényl]-amide de l'acide 5-cyano-pyridine-2-carboxylique ;
• le [3-(6-amino-2-difluorométhyl-5-oxo-2,3,4,5-tétrahydro-pyrazin-2-yl)-4-fluoro-phényl]-amide de l'acide 5-cyano-pyridine-2-carboxylique ;
• l'ester méthylique de l'acide 5-amino-3-{5-[(5-bromo-pyridine-2-carbonyl)-amino]-2-fluoro-phényl}-3-difluorométhyl-3,6-dihydro-2H-pyrazine-1-carboxylique ;
• l'ester méthylique de l'acide 5-amino-3-{5-[(5-cyano-pyridine-2-carbonyl)-amino]-2-fluoro-phényl}-3-difluorométhyl-3,6-dihydro-2H-pyrazine-1-carboxylique ;
• le [3-(4-acétyl-6-amino-2-difluorométhyl-2,3,4,5-tétrahydro-pyrazin-2-yl)-4-fluoro-phényl]-amide de l'acide 5-bromo-3-méthyl-pyridine-2-carboxylique ;
• l'ester 2,2-dichloro-éthylique de l'acide 5-amino-3-{5-[(5-bromo-3-méthyl-pyridine-2-carbonyl)-amino]-2-fluoro-phényl}-3-difluorométhyl-3,6-dihydro-2H-pyrazine-1-carboxylique ;
• le {3-[6-amino-2-difluorométhyl-4-(2-méthoxy-acétyl)-2,3,4,5-tétrahydro-pyrazin-2-yl]-4-fluoro-phényl}-amide de l'acide 5-bromo-3-méthyl-pyridine-2-carboxylique ;
• le [3-(6-amino-4-cyclopropanecarbonyl-2-difluorométhyl-2,3,4,5-tétrahydro-pyrazin-2-yl)-4-fluoro-phényl]-amide de l'acide 5-bromo-3-méthyl-pyridine-2-carboxylique ;
• le [3-(6-amino-2-difluorométhyl-2,3,4,5-tétrahydro-pyrazin-2-yl)-4-fluoro-phényl]-amide de l'acide 5-bromo-3-méthyl-pyridine-2-carboxylique ;
• le [3-(4-acétyl-6-amino-2-difluorométhyl-2,3,4,5-tétrahydro-pyrazin-2-yl)-4-fluoro-phényl]-amide de l'acide 5-difluorométhoxy-3-méthyl-pyridine-2-carboxylique ;
• le [3-(6-amino-2-difluorométhyl-2,3,4,5-tétrahydro-pyrazin-2-yl)-4-fluoro-phényl]-amide de l'acide 5-difluorométhoxy-3-méthyl-pyridine-2-carboxylique ;
• le [3-(6-amino-2-difluorométhyl-2,3,4,5-tétrahydro-pyrazin-2-yl)-4-fluoro-phényl]-amide de l'acide 3-amino-5-méthoxy-pyrazine-2-carboxylique ; et
• le [3-(6-amino-2-difluorométhyl-2,3,4,5-tétrahydro-pyrazin-2-yl)-4-fluoro-phényl]-amide de l'acide 3-amino-5-oxo-4,5-dihydro-pyrazine-2-carboxylique.

11. Composé selon l'une quelconque des revendications 1 à 10, ou sel pharmaceutiquement acceptable de celui-ci, destiné à être utilisé comme médicament.

12. Composé selon l'une quelconque des revendications 1 à 10, ou sel pharmaceutiquement acceptable de celui-ci, destiné à être utilisé dans le traitement ou la prévention de la maladie d'Alzheimer ou du trouble cognitif léger.

13. Composition pharmaceutique comprenant un composé selon l'une quelconque des revendications 1 à 10, ou sel pharmaceutiquement acceptable de celui-ci, comme principe actif, et un support ou diluant pharmaceutique.

14. Utilisation d'un composé selon l'une quelconque des revendications 1 à 10, ou sel pharmaceutiquement acceptable de celui-ci, pour la fabrication d'un médicament pour le traitement ou la prévention de la maladie d'Alzheimer ou du trouble cognitif léger.

15. Combinaison comprenant une quantité thérapeutiquement efficace d'un composé selon l'une quelconque des revendications 1 à 10, ou sel pharmaceutiquement acceptable de celui-ci, et une seconde substance médicamenteuse, pour une administration simultanée ou séquentielle.
